(19) Europäisches Patentamt
European Patent Office
Office européen des brevets

(11) Publication number: **0 094 586**
**A2**

# (12) EUROPEAN PATENT APPLICATION

(21) Application number: 83104545.5

(22) Date of filing: 09.05.83

(51) Int. Cl.$^3$: C 07 C 93/04
C 07 C 103/38, C 07 C 125/065
C 07 C 149/18, C 07 C 154/02
A 61 K 31/13, A 61 K 31/16
A 61 K 31/21

(30) Priority: 13.05.82 JP 79179/82
22.07.82 JP 126664/82
16.12.82 JP 219206/82
21.12.82 JP 224699/82

(43) Date of publication of application:
23.11.83 Bulletin 83/47

(84) Designated Contracting States:
AT BE CH DE FR GB IT LI LU NL SE

(71) Applicant: **ONO PHARMACEUTICAL CO., LTD.**
No. 14, Doshomachi 2-chome Higashi-ku
Osaka-shi Osaka(JP)

(72) Inventor: **Itoh, Hiroyuki**
No. 3-16-20, Yamatedai
Ibaraki-shi Osaka(JP)

(72) Inventor: **Okada, Takanori**
No. 2-14-17, Higashi-Ohta
Ibaraki-shi Osaka(JP)

(72) Inventor: **Miyamoto, Tsumoru**
No. 1-40, Tono-Moriyama
Joyo-shi Kyoto(JP)

(74) Representative: **Hansen, Bernd, Dr.rer.nat. et al,**
Hoffmann, Eitle & Partner Patentanwälte
Arabellastrasse 4
D-8000 München 81(DE)

(54) **Glycerol derivatives, process for preparing same and pharmaceutical composition containing same.**

(57) Glycerol derivatives of the general formula:

$$\begin{array}{ll} 1 & A-R^1 \\ 2 & B-R^2 \\ 3 & D-R^3-R^4 \end{array} \qquad \text{I}$$

in which A, B, D, $R^1$, $R^2$, $R^3$ and $R^4$ have the meaning given in the specification, possess antagonistic effect on PAF, hypotensive effect like PAF, inhibitory effect on phospholipase and inhibitory effect on growth of tumour cell or induction of differentiation, and are, therefore, useful as a platelet aggregation inhibitor, anti-asthmatic agent, anti-allergic agent, anti-inflammatory agent, hypotensive agent and anti-tumour agent for mammals. Disclosed are also methods for preparing the novel compounds.

EP 0 094 586 A2

Croydon Printing Company Ltd

# GLYCEROL DERIVATIVES, PROCESS FOR PREPARING SAME AND PHARMACEUTICAL COMPOSITION, CONTAINING SAME

The present invention is concerned to new glycerol derivatives. More particularly, this invention is concerned to new glycerol derivatives having antagonistic effect on platelet activating factor (abbreviated as PAF hereafter), hypotensive effect like PAF, inhibitory effect on phospholipase and further, inhibitory effect on growth of tumour cell or induction of differentiation, and, furthermore, this invention is concerned to the process for their preparation and pharmaceutical composition comprising them as active ingredients.

Recently, the study on the release reaction of active amine dependent on leukocyte from platelet, has been energetically carried out. As a result, a substance strongly inducing platelet aggregation was discovered and it was named as PAF [cf. J. Immunol., 106, 1244 (1971), J. Exp. Med., 136, 1356 (1972) and Nature, 249, 581 (1974)]. Thereafter, it was confirmed that PAF exists in various animals including human beings. In 1979, its chemical structure was identified [cf. J. Biol. Chem., 254, 9355 (1979) and C. R. Acad. Sci., Série D (Paris), 289, 1017 (1979)] and has turned out to be a mixture of two alkylphospholipids of the following general formula wherein n is 15 and 17 [cf. J. Biol. Chem., 255, 5514 (1980)]:

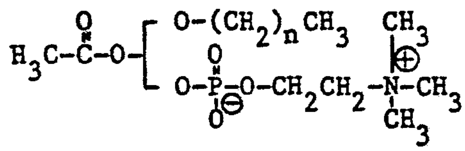

(wherein n represents 15 or 17).

It was found that PAF has not only strong secretory effect on platelet aggregation known at that time, but also powerful hypotensive effect and bronchodilating effect. PAF is considered to be one of platelet aggregating factors in human beings, and further, one of substances inducing allergy and inflammation. Accordingly, there is a great possibility that compounds having antagonistic effect on PAF (inhibitory effect on PAF) may become a platelet aggregation inhibitor, anti-asthmatic agent, anti-allergic agent or anti-inflammatory agent. Furthermore, PAF is considered to become a hypotensive agent without inhibitory effect on platelet aggregation by selecting hypotensive effect out of various effect.

Widespread investigations have been carried out in order to discover compounds possessing antagonistic effect on PAF or hypotensive effect like PAF, we have found that the above purpose can be accomplished by compounds of the general formula (I) hereinafter described, having completed this invention.

Furthermore, it has been found that the compounds of the present invention of the general formula (I) have inhibitory effect on phospholipase $A_2$ and phospholipase C. Phospholipase

$A_2$ and phospholipase C dependent on calcium in a living body are concerned in the mechanism for the release of arachidonic acid from phospholipids. It is known that arachidonic acid is released by physiological stimulus and then becomes incorporated into the metabolic routes of prostaglandins and is finally metabolized to thromboxane $A_2$ having a strong effect on platelet aggregation, to other prostaglandins or to various leukotrienes being a significant bronchostrictor in an asthmatic paroxysm. Accordingly, it is considered that compounds having an inhibitory effect on phospholipase $A_2$ and phospholipase C, which are concerned in the mechanism for the release of arachidonic acid, can become a platelet aggregation inhibitor, anti-asthmatic agent, anti-allergic agent or anti-inflammatory agent, based on the inhibition of phospholipase $A_2$ and phospholipase C, having a different mechanism from PAF antagonist and further, being different from conventional anti-inflammatory agents represented by aspirin based on the inhibition of cyclooxygenase.

Furthermore, the compounds of the present invention of the general formula (I) show inhibitory effect on growth of tumour cell and induction of differentiation and, therefore, are may be useful as anti-tumour agents.

Accordingly, the present invention provides new glycerol derivatives of the general formula:

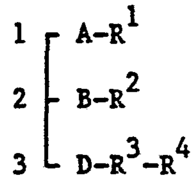

I

[wherein A represents an oxygen atom or a sulfur atom, B represents an oxygen atom or a sulfur atom, D represents an oxygen atom or a carbonyloxy group (i.e. $-O\underset{\underset{O}{\|}}{C}-$ , in which the carbonyl group should be attached to a group $R^3$ and the oxa group should be attached to the carbon atom at the third position of the glycerol skeleton), $R^1$ represents an alkyl group of 6 to 22 carbon atoms, or an alkyl group of 2 to 5 carbon atoms being substituted by a phenyl group, $R^2$ represents, when B is an oxygen atom, a hydrogen atom, or a group of the general formula: $-CH_2-$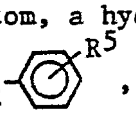 , $-COR^6$ or $-CONHR^7$, in which $R^5$ represents a hydrogen atom, a halogen atom, an alkyl group of 1 to 4 carbon atoms or an alkoxy group of 1 to 4 carbon atoms, $R^6$ represents an alkyl group of 1 to 6 carbon atoms or a phenyl group and $R^7$ represents an alkyl group of 1 to 6 carbon atoms or a phenyl group, and $R^2$ represents, when B is a sulfur atom, a hydrogen atom, an alkyl group of 1 to 6 carbon atoms, or a group of the general formula: $-CH_2-$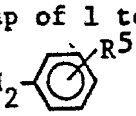 or $-CS-OR^8$, in which $R^8$ represents an alkyl group of 1 to 6 carbon atoms or a phenyl group and $R^5$ is as hereinbefore defined, $R^3$ represents an alkylene group of 1 to 12 carbon atoms and $R^4$ represents an amino group, or a group of the general formula: $-NHCOR^9$,

$-NHR^{10}$, $-N(R^{10})_2$, $-\overset{\oplus}{N}(R^{10})_3 \cdot \overset{\ominus}{Y}$, in which $R^9$ represents an alkyl group of 1 to 6 carbon atoms or a phenyl group, $R^{10}$ represents an alkyl group of 1 to 8 carbon atoms, with the proviso that, when $R^4$ represents a group of the formula $-N(R^{10})_2$ or $-\overset{\oplus}{N}(R^{10})_3 \cdot \overset{\ominus}{Y}$, two or more of $R^{10}$ may be same or different to each other, and $\overset{\ominus}{Y}$ represents an anion of an acid], and non-toxic acid addition salts thereof.

Throughout the specification including claims, the term "alkyl", "alkylene" or "alkoxy" means a straight- or branched-chain alkyl, alkylene or alkoxy group.

As is obvious to those skilled in the art, the compounds of the general formula I have at least one asymmetric center, i.e. the carbon atom at the second position of the glycerol skeleton. When the alkyl or alkylene moiety of various substituents represented by $R^1$, $R^2$, $R^3$ and $R^4$ represents a branched-chain, threre is a possibility that other asymmetric centers are occurred. The existence of an asymmetric center forms isomers as is well known. However the compounds of the general formula I in the present invention are concerned with all isomers or mixtures thereof.

Examples of the alkyl group represented by $R^1$ are hexyl, heptyl, octyl, nonyl, decyl, undecyl, dodecyl, tridecyl, tetradecyl, pentadecyl, hexadecyl, heptadecyl, octadecyl,

nonadecyl, eicosyl, heneicosyl and docosyl group and isomers thereof. Any group is preferred and hexadecyl group is most preferred.

Examples of the alkyl group substituted by a phenyl group, represented by $R^1$ are ethyl, propyl, butyl and pentyl group and isomers thereof replaced an optional hydrogen atom by a phenyl group.

Examples of the substituent $R^5$ in the general formula: 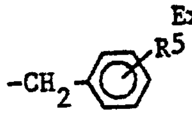 represented by $R^2$ are hydrogen atom, fluorine, chlorine, bromine and iodine atom, methyl, ethyl, propyl and butyl group and isomers thereof and methoxy, ethoxy, propoxy and butoxy group and isomers thereof. Preferably $R^5$ represents hydrogen atom or halogen atom and most preferably $R^5$ is hydrogen atom.

Examples of the alkyl group represented by $R^6$, $R^7$ and $R^8$ in various group represented by $R^2$ are methyl, ethyl, propyl, butyl, pentyl and hexyl group and isomers thereof and any group is preferred. A phenyl group as $R^6$, $R^7$ and $R^8$ is also preferred.

Examples of the alkyl group represented by $R^2$, when B is a sulfur atom, are methyl, ethyl, propyl, butyl, pentyl and hexyl group and isomers thereof and any group is preferred.

A hydrogen atom as $R^2$ is also preferred.

Examples of the alkylene group represented by $R^3$ are methylene, ethylene, trimethylene, tetramethylene, pentamethylene, hexamethylene, heptamethylene, octametylene, nonamethylene, decamethylene, undecamethylene and dodecamethylene group and isomers thereof, and any group is preferred.

Examples of the alkyl group represented by $R^9$ in the general formula $-NHCOR^9$ in $R^4$ are methyl, ethyl, propyl, butyl, pentyl and hexyl group and isomers thereof and any group is preferred. A phenyl group as $R^9$ is also preferred.

Examples of the alkyl group represented by $R^{10}$ in the general formulae $-NHR^{10}$, $-N(R^{10})_2$ and $-\overset{\oplus}{N}(R^{10})_3 \cdot \overset{\ominus}{Y}$ in $R^4$ are methyl, ethyl, propyl, butyl, pentyl, hexyl, heptyl and octyl and isomers thereof and any group is preferred.

The anion of an acid represented by $\overset{\ominus}{Y}$ mean the anion of a pharmaceutically-acceptable non-toxic inorganic or organic acid, and examples of them are the anion of an inorganic acid such as hydrochloric acid, hydrobromic acid, hydroiodic acid, sulfuric acid, phosphoric acid, nitric acid, or the anion of an organic acid such as acetic acid, lactic acid, tartaric acid, benzoic acid, citric acid, methanesulfonic acid, ethanesulfonic acid, benzenesulfonic acid, toluenesulfonic acid, isethionic acid. Any anion is preferred and more preferable anion is a halogen ion, i.e. chlorine, bromine and iodine ions.

According to a feature of the present invention, glycerol derivatives of the general formula I, wherein the group $-B-R^2$ represents a hydroxy group, i.e. compounds of the general formula:

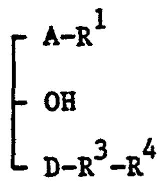

IA

(wherein the various symbols are as hereinbefore defined) may be prepared by reductive elimination of the benzyl group attached to the 2nd position of compounds of the general formula:

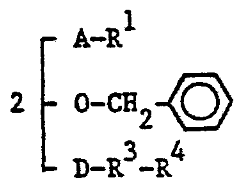

IB'

(wherein the various symbols are as hereinbefore defined). The reaction is well-known and may be carried out, for example, under an atmosphere of hydrogen in the presence of a hydrogenation catalyst such as palladium on carbon, palladium black or platinum dioxide in an inert organic solvent, e.g. a lower alkanol such as methanol or ethanol, or ethyl acetate or acetic acid, or a mixture of two or more of them at a temperature from ambient to the reflux temperature of the reaction mixture.

Glycerol derivatives of the general formula I, wherein the group $-B-R^2$ represents the group of the general formula:

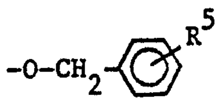 , i.e. compounds of the general formula:

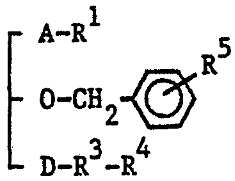

IB

(wherein the various symbols are as hereinbefore defined) may be prepared by the series of reactions depicted schematically below in Scheme A (where D is an oxygen atom) and Scheme B (where D is a carbonyloxy group), wherein X represents a chlorine, bromine or iodine atom, T represents an alkylsulfonyl group or a substituted or unsubstituted arylsulfonyl group, preferably mesyl or tosyl group, Q represents tert-butoxycarbonyl group (abbreviated as boc group hereafter) or benzyloxycarbonyl group (abbreviated as cbz group hereafter), Tr represents a trityl group and $R^{11}$ represents a hydrogen atom or an alkyl group of 1 to 8 carbon atoms and other symbolys are as hereinbefore defined.

Scheme A

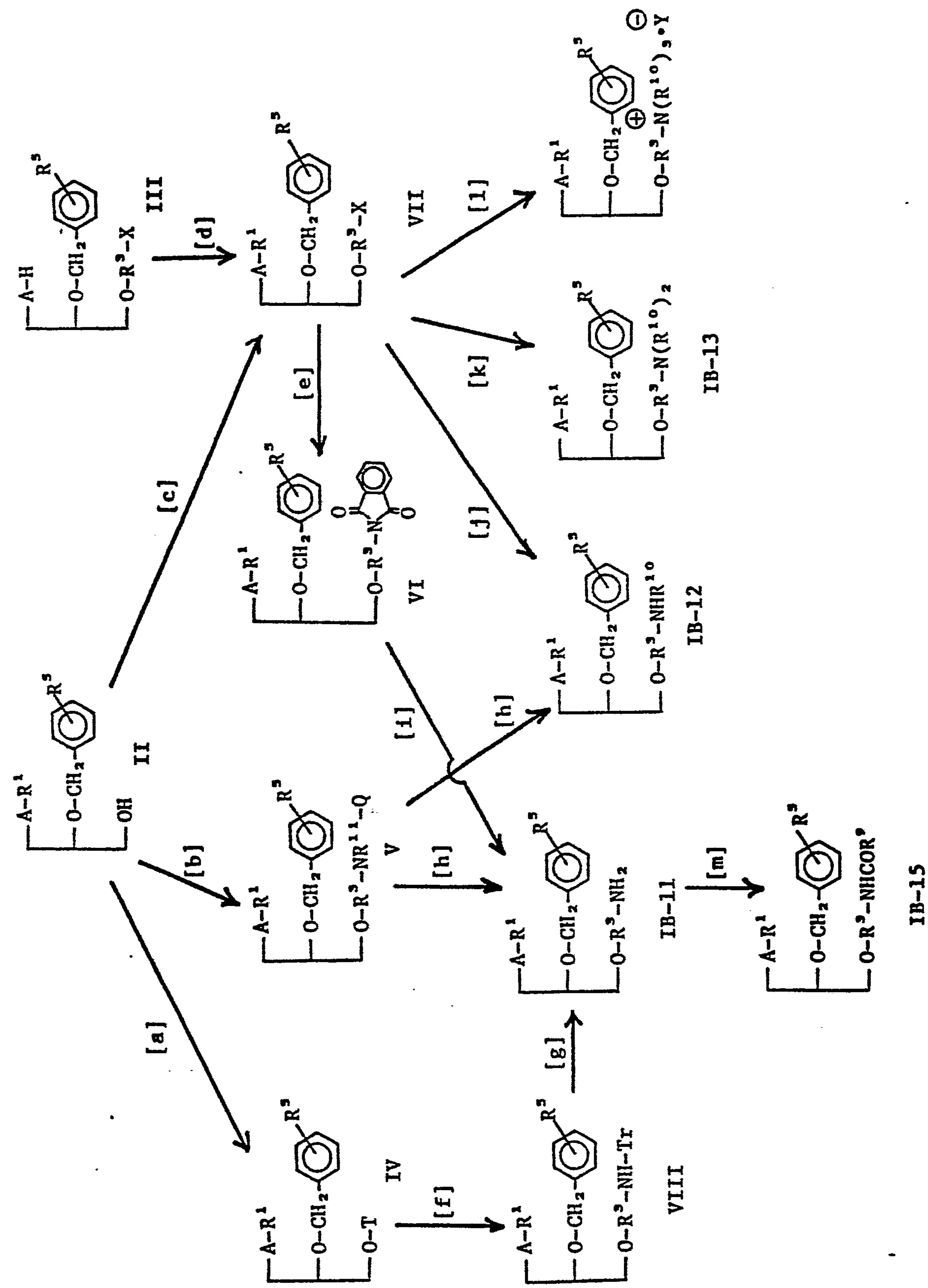


II III IV V VI VII VIII IB-11 IB-12 IB-13 IB-15

Scheme B

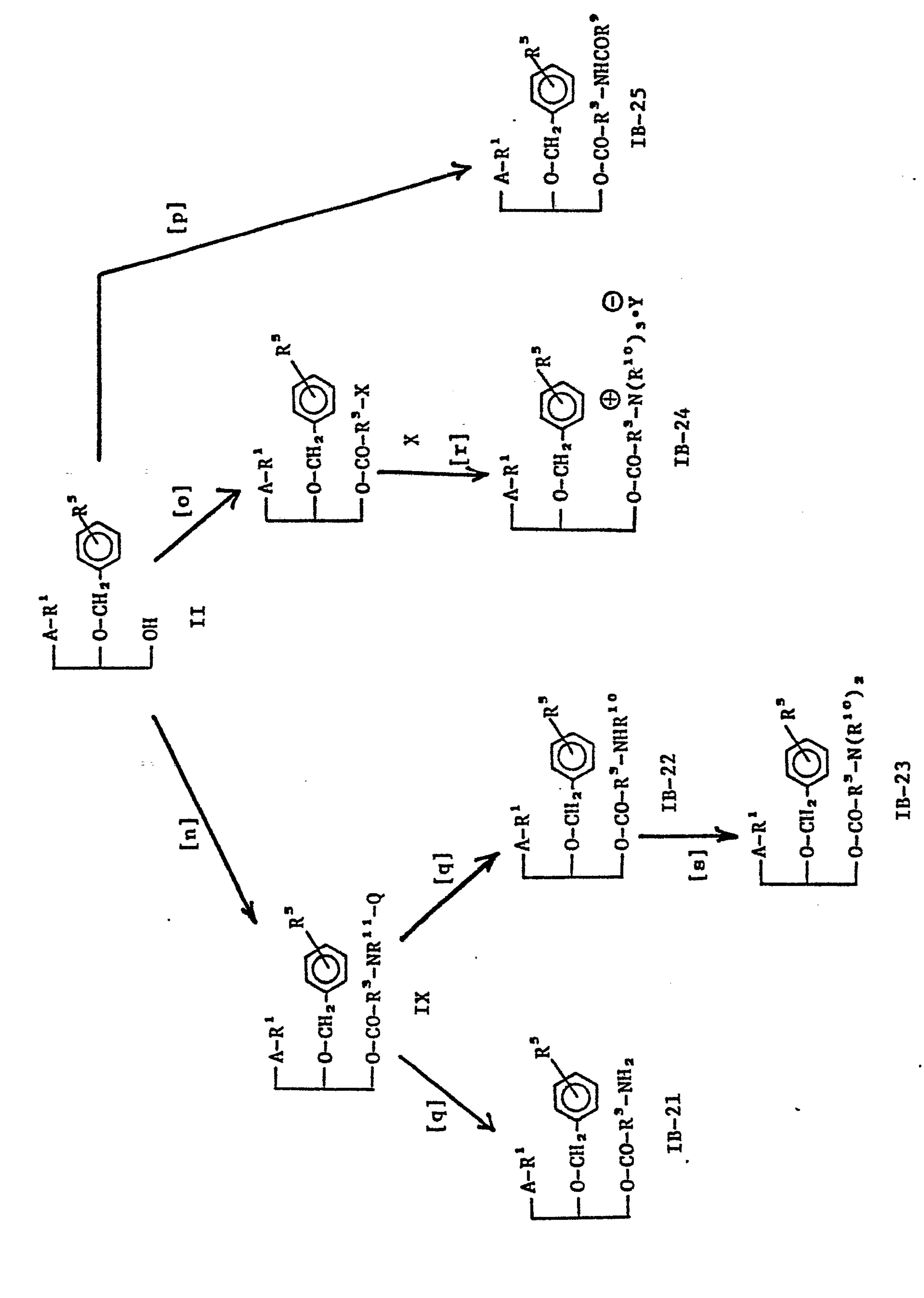


II, IX, X, IB-21, IB-22, IB-23, IB-24, IB-25

In Scheme A and B, each step can be effected using methods known per se. For example, step [a] may be carried out by reacting a compound of the general formula II with an alkylsulfonyl chloride such as mesyl chloride or with an arylsulfonyl chloride such as tosyl chloride at a temperature from -30°C to 50°C (i) in the presence of a tertiary amine such as pyridine or triethylamine, in an inert organic solvent such as methylene chloride or (ii) in pyridine.

Step [b] may be carried out by reacting a compound of the general formula II with a compound of the general formula:

$$T\text{-}O\text{-}R^3\text{-}NR^{11}\text{-}Q \qquad XI$$

(wherein the various symbols are as hereinbefore defined) in the presence of an alkali metal hydride such as sodium hydride in an inert organic solvent such as dimethylformamide (abbreviated as DMF hereafter) or tetrahydrofuran (abbreviated as THF hereafter) at a temperature from ambient to 80°C.

Step [c] may be carried out by reacting a compound of the general formula II with a compound of the general formula:

$$T\text{-}O\text{-}R^3\text{-}X \qquad XII$$

(wherein the various symbols are as hereinbefore defined) in the presence of an alkali metal hydride such as sodium hydride in an inert organic solvent such as DMF or THF at a temperature from ambient to 80°C.

Step [c] may be also carried out by using a

corresponding halide, e.g. 1,6-dibromohexane instead of the sulfonyloxy compound of the general formula XII.

Step [d] may be carried out by reacting a compound of the general formula III with a compound of the general formula:

$$X^1-R^1 \qquad XIII$$

(wherein $X^1$ represents a chlorine, bromine or iodine atom and $R^1$ is as hereinbefore defined) in the presence of an alkali metal hydride such as sodium hydride in anhydrous DMF at a temperature from ambient to 100°C.

Step [e] may be carried out by using phthalimide in an inert organic solvent such as DMF at a temperature from 50°C to a reflux temperature of the reaction mixture.

Step [f] may be carried out by reacting a compound of the general formula IV with a compound of the general formula:

$$HO-R^3-NH-Tr \qquad XIV$$

(wherein the various symbols are as hereinbefore defined) in the presence of an alkali metal hydride such as sodium hydride in an inert organic solvent such as DMF or THF at a temperature from ambient to 80°C.

Step [g] may be carried out by reacting in an aqueous solution of acetic acid at a temperature from 50°C to 100°C, or by using hydrochloric acid in an inert organic solvent such as acetone or a lower alkanol at a temperature from ambient to a reflux temperature of the reaction mixture, or by using

hydrobromic acid in acetic acid at room temperature.

Step [h] may be carried out, (i) when Q is a boc group, by using hydrochloric acid in an inert organic solvent such as ethyl acetate, methylene chloride, chloroform, a lower alkanol at a temperature from ambient to 50°C, or by using trifluoroacetic acid at room temperature, or (ii) when Q is a cbz group, by using trimethylsilyl iodide in acetonitrile at room temperature.

Step [i] may be carried out by using hydrazine in a lower alkanol such as ethanol.

Step [j], [k] and [l] may be carried out by reacting a compound of the general formula VII with compounds of the general formulae:

$H_2N-R^{10}$, $HN(R^{10})_2$ and $N(R^{10})_3$

XV XVI XVII

(wherein $R^{10}$ is as hereinbefore defined), respectively, in an inert organic solvent such as a lower alkanol, e.g. methanol, ethanol, isopropyl alcohol, or DMF or a mixture of two or more of than at a temperature from ambient to a reflux temperature of the reaction mixture. If desired, compounds of the general formula IB-14 wherein $Y^{\ominus}$ is a halogen ion, prepared by the step [l] may be converted into compounds of the general formula IB-14 wherein $Y^{\ominus}$ is an anion of an acid other than halogen ion by using methods for salt-exchange hereinafter described.

Step [m] may be carried out by using a corresponding acyl chloride, e.g. benzoyl chloride or a corresponding acid anhydride, e.g. acetic anhydride in the presence of a tertiary amine such as pyridine or triethylamine in an inert organic solvent such as methylene chloride or in the absence of a solvent at a temperature below 50°C.

In Scheme B, step [n], [o] and [p] may be carried out by esterifying a compound of the general formula II with compounds of the general formulae:

$HOOC-R^3-NR^{11}-Q$ XVIII,

$HOOC-R^3-X$ XIX

and

$HOOC-R^3-NHCOR^9$ XX

(wherein the various symbols are as hereinbefore defined), respectively. The esterification may be carried out by using dicyclohexylcarbodiimide, trifluoroacetic acid anhydride or N,N'-carbonyldiimidazole as a condensing agent in an inert organic solvent such as ethyl acetate, benzene, diethyl ether, chloroform, methylene chloride, THF at a temperature from 0°C to 50°C (preferably at room temperature). When dicyclohexylcarbodiimide is used as condensing agent, 4-dimethylaminopyridine or pyridine etc. is preferably used as a reaction-accelerating agent. Step [o] may be also carried out by using a compound of the general formula:

$$X^2-\underset{\underset{O}{\|}}{C}-R^3-X \qquad \text{XXI}$$

(wherein $X^2$ represents a chlorine or bromine atom and the other symbols are as hereinbefore defined) instead of the compound of the general formula XIX.

Step [q] may be carried out by methods hereinbefore described for step [h] in Scheme A.

Step [r] may be carried out by methods hereinbefore described for step [l] in Scheme A.

Step [s] may be carried out by reacting a compound of the general formula IB-22 with an equimolecular amount of alkyl halide such as methyl iodide in a lower alkanol such as methanol or ethanol at a temperature from ambient to the reflux temperature of the reaction mixture.

Compounds of the general formulae XI to XXI are well known *per se* or may be prepared by methods known *per se*.

Glycerol derivatives of the general formula I, wherein the group $-B-R^2$ represents the group of the general formula: $-O-\underset{\underset{O}{\|}}{C}-R^6$ and $R^4$ represents a group of the general formula: $-NHCOR^9$, $-N(R^{10})_2$ or $-\overset{\oplus}{N}(R^{10})_2 \cdot \overset{\ominus}{Y}$, i.e. compounds of the general formula:

$$\left[\begin{array}{l} A-R^1 \\ O-\underset{\underset{O}{\|}}{C}-R^6 \\ D-R^3-R^{4a} \end{array}\right. \qquad \text{IC-1}$$

(wherein $R^{4a}$ represents a group of the general formula:

$-NHCOR^9$, $-N(R^{10})_2$ or $-\overset{\oplus}{N}(R^{10})_3 \cdot \overset{\ominus}{Y}$, in which the various symbols are as hereinbefore defined and the other symbols are as hereinbefore defined.) may be prepared by acylating a compound of the general formula:

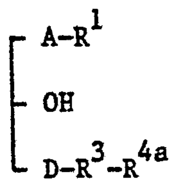

IA-1

(wherein the various symbols are as hereinbefore defined). The acylation may be carried out by methods hereinbefore described for step [m] in Scheme A.

Glycerol derivatives of the general formula I, wherein the group $-B-R^2$ represents the group of the general formula: $-O-\underset{\underset{O}{\|}}{C}-R^6$ and $R^4$ represents an amino group or a group of the general formula: $-NHR^{10}$, i.e. compounds of the general formula:

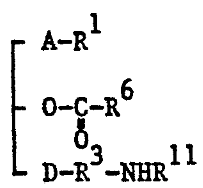

IC-2

(wherein the various symbols are as hereinbefore defined) may be prepared by the series of reactions depicted schematically below in Scheme C, wherein the various symbols are as hereinbefore defined.

Scheme C

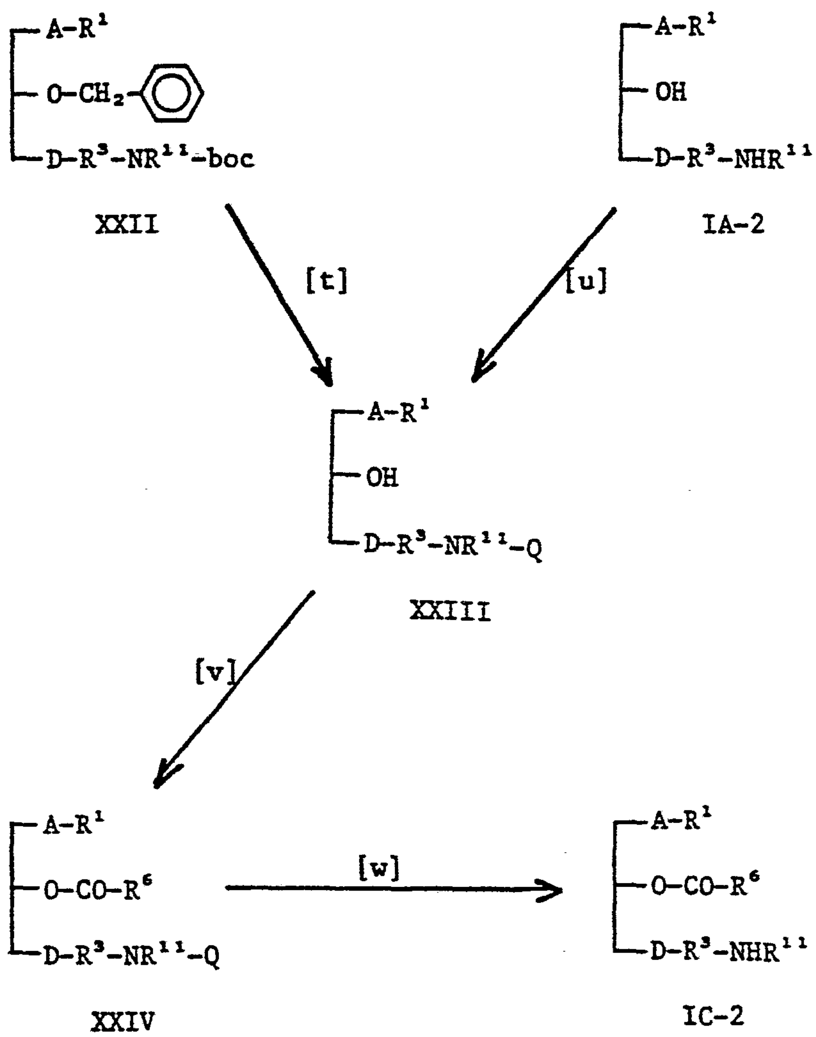


XXII IA-2 XXIII XXIV IC-2

In Scheme C, each step can be effected using methods known per se. For example, step [t] may be carried out by methods hereinbefore described for the conversion of compounds of the general formula IB' to those of the general formual IA.

Step [u] may be carried out by reacting with di-tert-butyldicarbonate in an aqueous solution of sodium hydroxide at room temperature, or in methylene chloride in the presence of a tertiary amine such as triethylamine at room temperature or below, or reacting with benzyl chloroformate in an aqueous solution of sodium hydroxide or sodium carbonate at a temperature from 0°C to 50°C.

Step [v] may be carried out by methods hereinbefore described for step [m] in Scheme A.

Step [w] may be carried out by methods hereinbefore described for step [h] in Scheme A. When Q represents a cbz group, step [w] may also be carried out by the catalytic reduction [i.e. methods hereinbefore described for the conversion of compounds of the general formual IB' to those of the general formual IA] in the presence of acetic acid or hydrochloric acid.

Compounds of the general formula XXIV, wherein D represents the carbonyloxy group may be prepared by esterifying a compound of the general formula:

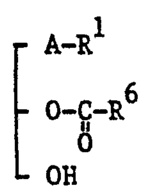

XXV

(wherein the various symbols are as hereinbefore defined) with a compound of the general formula XVIII. The esterification may be carried out by methods hereinbefore described for step [n] in Scheme B.

Starting materials of the general formula XXII is parts of compounds of the general formulae V and IX.

Glycerol derivatives of the general formula I, wherein the group $-B-R^2$ represents the group of the general formula: $-O\underset{\underset{\displaystyle O}{\|}}{C}-NHR^7$, i.e. compounds of the general formula:

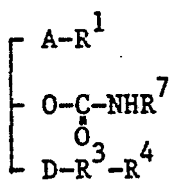

ID

(wherein the various symbols are as hereinbefore defined) may be prepared by the series of reactions depicted schematically below in Scheme D, wherein the various symbols are as hereinbefore defined.

Scheme D

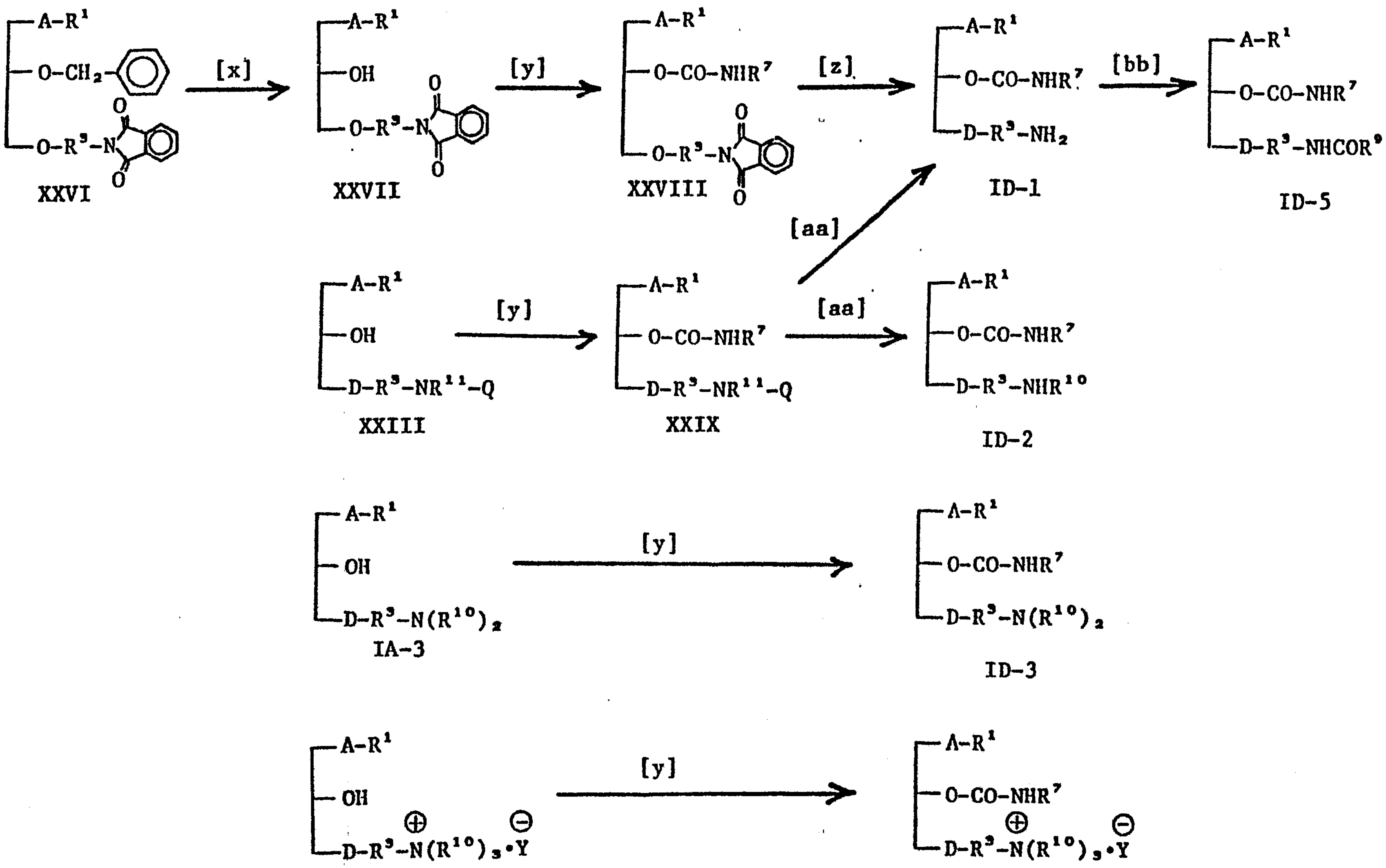


XXVI XXVII XXVIII ID-1 ID-5

XXIII XXIX ID-2

IA-3 ID-3

IA-4 ID-4

In Scheme D, each step can be effected using methods known per se. For example, step [x] may be carried out by methods hereinbefore described for the conversion of compounds of the general formula IB' to those of the general formula IA.

Step [y] may be carried out by reacting compounds of the general formula XXVII, XXIII, IA-3 or IA-4 with isocyanate compounds of the general formula:

$$R^7-N=C=O \qquad XXX$$

(wherein $R^7$ is as hereinbefore defined) in an inert organic solvent such as ethyl acetate, benzene, hexane, diethyl ether, THF, chloroform, methylene chloride or carbon tetrachloride, or two or more of them, in the presence of a tertiary amine such as pyridine or triethylamine at a temperature of 0°C to 50°C.

Step [z] may be carried out by methods hereinbefore described for step [i] in Scheme A.

Step [aa] may be carried out by methods hereinbefore described for step [h] in Scheme A.

Step [bb] may be carried out by methods hereinbefore described for step [m] in Scheme A.

Starting material of the general formula XXVI is a part of compounds of the general formula VI.

Compounds of the general formula XXX are well known per se or may be prepared by methods known per se.

Glycerol derivatives of the general formula I, wherein B represents a sulfur atom and D represents an oxygen atom, i.e. compounds of the general formula:

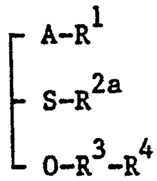

IE

(wherein $R^{2a}$ represents a hydrogen atom, an alkyl group of 1 to 6 carbon atoms or a group of the general formula $-CH_2-$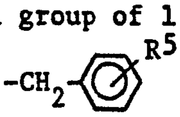 or $-CS-OR^8$, in which the various symbols are as hereinbefore defined, and the other symbols are as hereinbefore defined) may be prepared by the series of reactions depicted schematically below in Scheme E, wherein $R^{2b}$ represents an alkyl group of 1 to 6 carbon atoms or a group of the general formula $-CH_2-$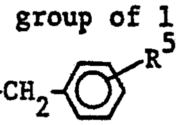 or $-CS-OR^8$, in which the various symbols are as hereinbefore defined, Ac represents acetyl group and the other symbols are as hereinbefore defined.

Scheme E

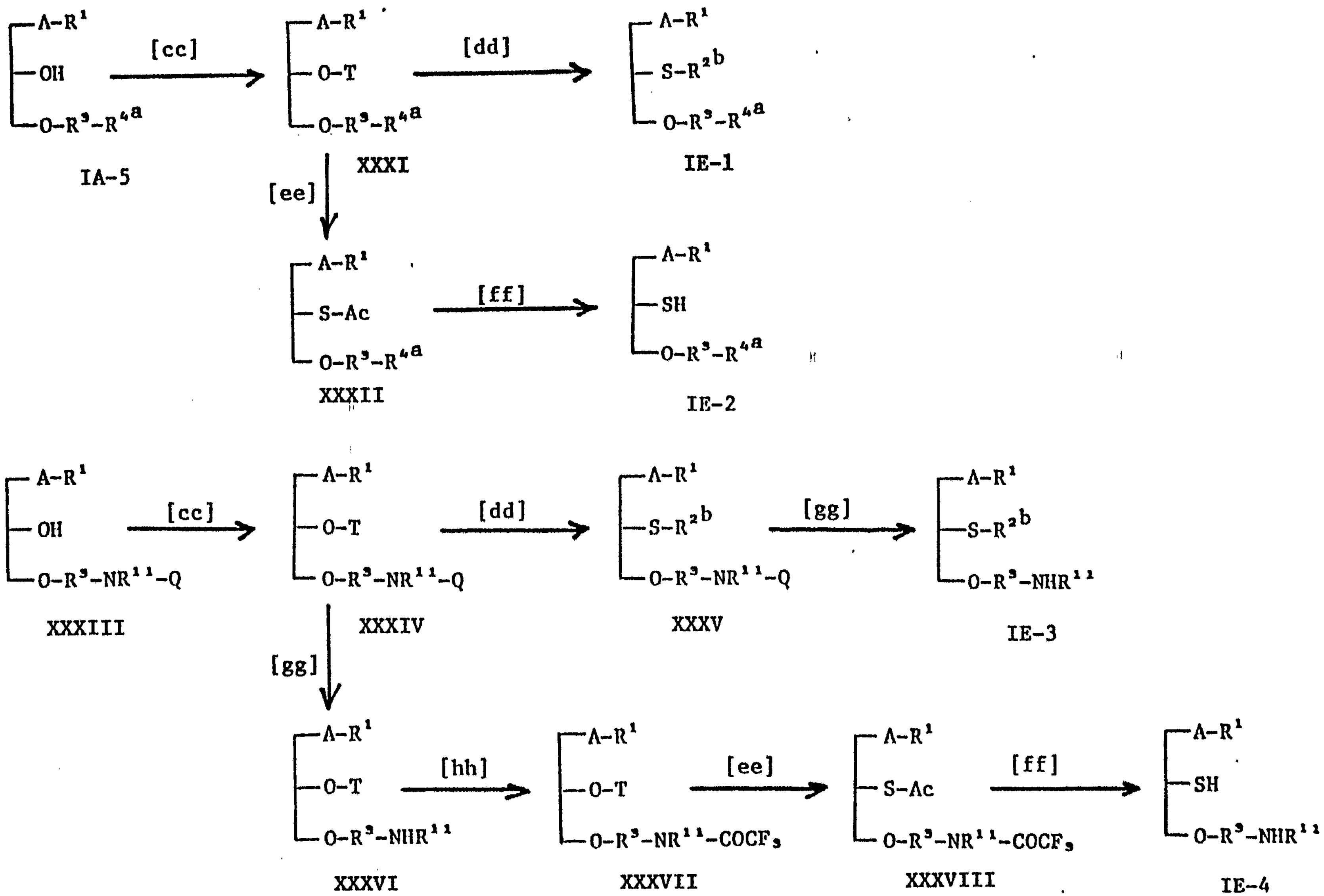


IA-5, XXXI, IE-1, XXXII, IE-2, XXXIII, XXXIV, XXXV, IE-3, XXXVI, XXXVII, XXXVIII, IE-4

In Scheme E, each step can be effected using methods known per se. For example, step [cc] may be carried out by methods hereinbefore described for step [a] in Scheme A.

Step [dd] can be carried out by reacting a compound of the general formula XXXI or XXXIV with a compound of the general formula:

$HS-R^{12}$, 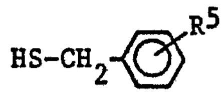 or 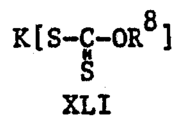

XXXIX XL XLI

(wherein $R^{12}$ represents an alkyl group of 1 to 6 carbon atoms and the other symbols are as hereinbefore defined). The reaction of the compound of the general formula XXXI or XXXIV with the compound of the general formula XXXIX or XL may be effected in the presence of sodium hydride in an inert organic solvent, e.g. an ether such as THF, diethyl ether or dioxane, or DMF or mixture of two or more of them at a temperature from ambient to the reflux temperature of the reaction mixture. The reaction of the compound of the general formula XXXI or XXXIV with the compound of the general formula XLI may be effected in an inert organic solvent such as hexamethyl phosphoramide (abbreviated as HMPA hereafter), DMF or dimethyl sulfoxide (abbreviated as DMSO hereafter) at a temperature from ambient to 100°C.

Step [ee] may be carried out by reacting with thioacetic acid sodium salt or its potassium salt in an inert organic solvent such as DMF, DMSO or HMPA at a temperature from 40°C to 60°C.

Step [ff] may be carried out by using an alkali metal such as sodium or potassium, hydroxide or using a tertialy amine such as triethylamine, in a lower alkanol such as methanol or ethanol at a temperature above ambient, preferably 50°C - 60°C.

Step [gg] may be carried out by methods hereinbefore described for step [h] in Scheme A.

Step [hh] may be carried out by reacting with trifluoroacetic anhydride in an inert organic solvent such as methylene chloride or in the absence of the solvent, in the presence of a tertiary amine such as triethylamine at a temperature below 50°C.

Starting material of the general formula XXXIII is a part of compounds of the general formula XXIII.

Compounds of the general formulae XXXIX, XL and XLI are well known per se or may be prepared by methods known per se.

Glycerol derivatives of the general formula I, wherein B represents a sulfur atom and D represents a carbonyloxy group, i.e. compounds of the general formula:

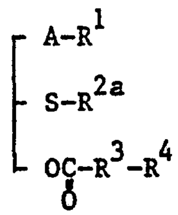

IF

(wherein the various symbols are as hereinbefore defined) may be prepared by the series of reactions depicted schematically below in Scheme F and G, wherein $R^{4b}$ represents a group of the general formula: $-NHCOR^9$ or $-N(R^{10})_2$, in which the various symbols are as hereinbefore defined, THP represents a tetrahydropyran-2-yl group and the other symbols are as hereinbefore defined.

Scheme F

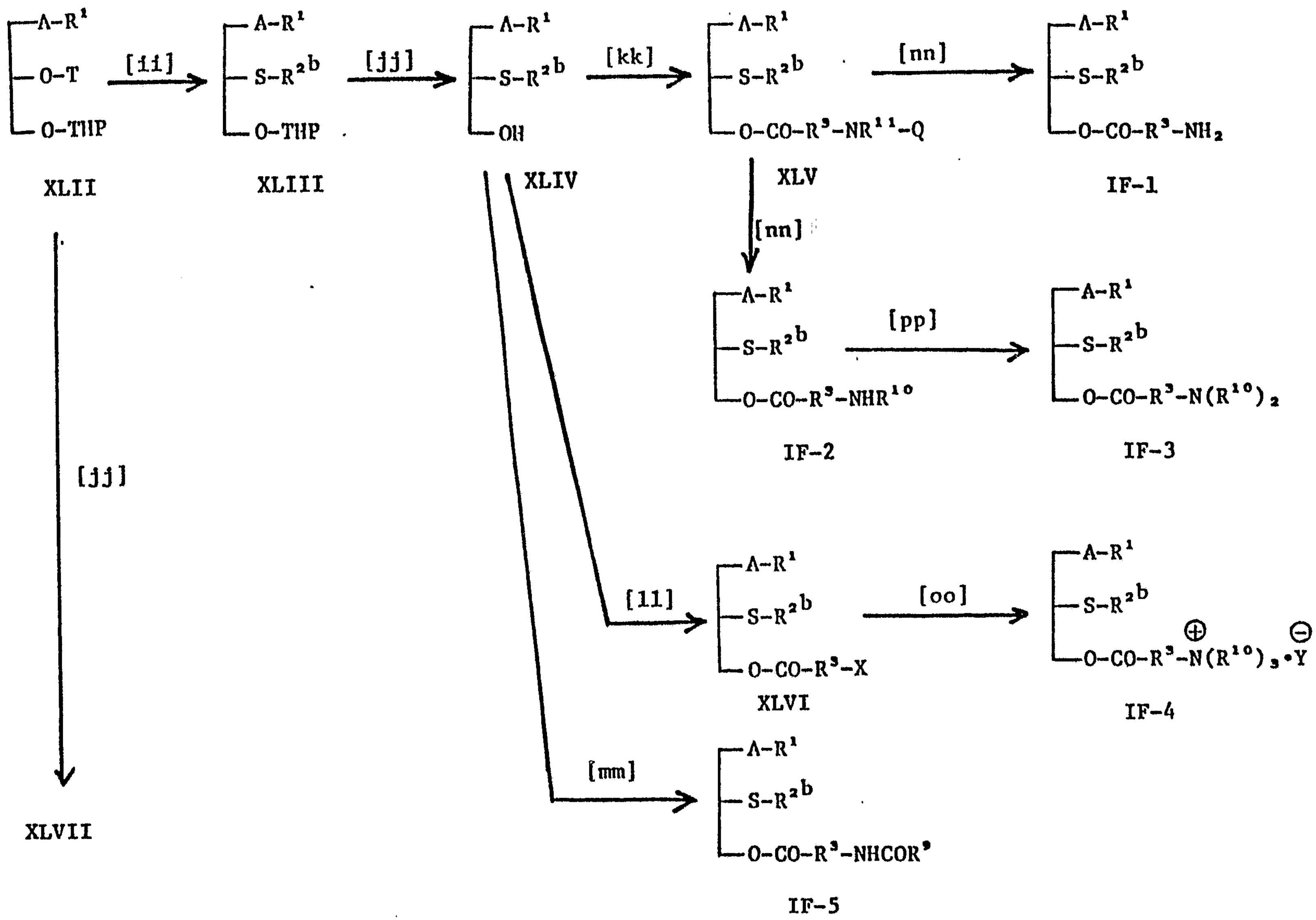


XLII XLIII XLIV XLV IF-1 IF-2 IF-3 XLVI IF-4 IF-5 XLVII

Scheme G

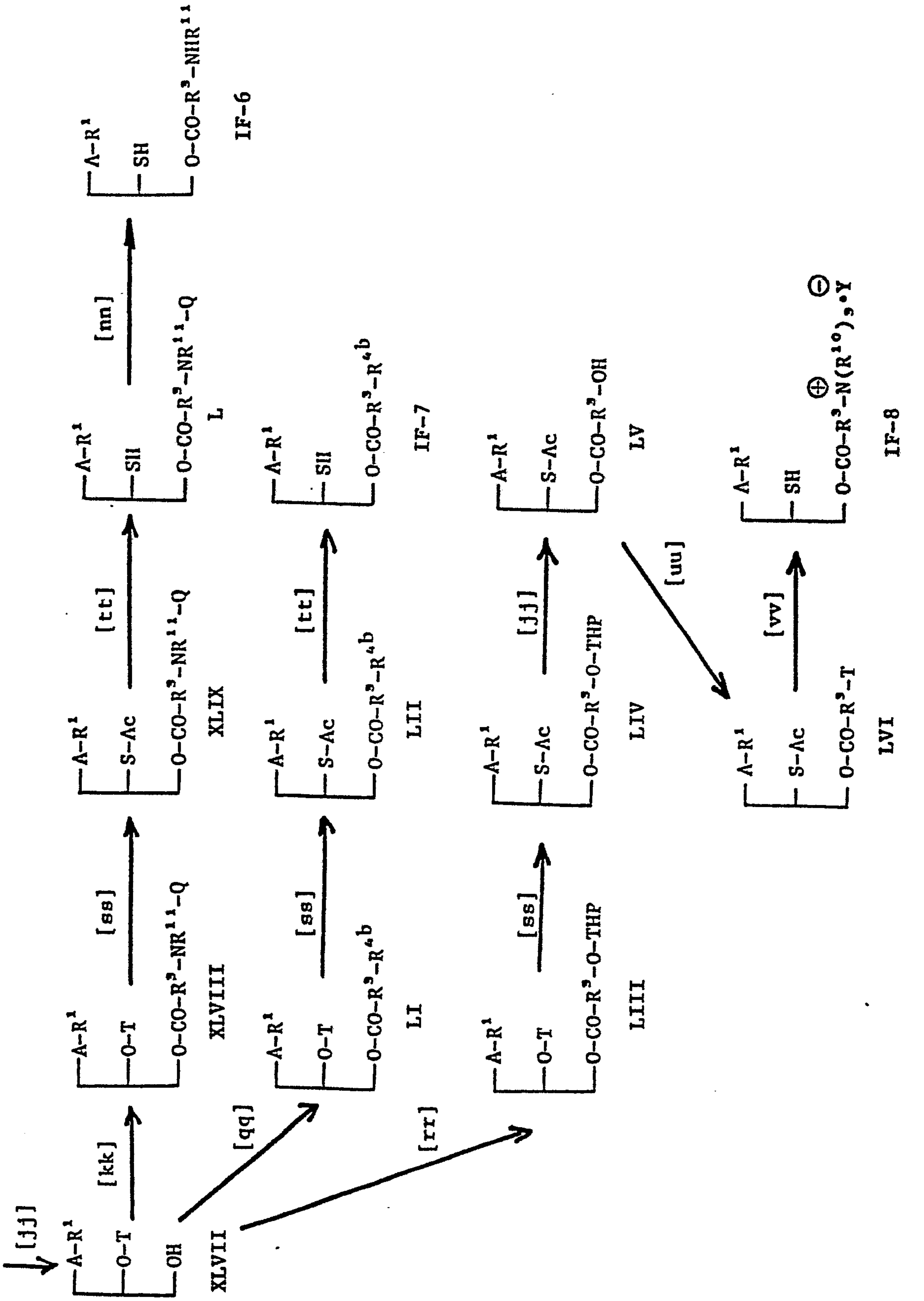

XLII
[jj]
XLVII
[kk]
[qq]
[rr]
XLVIII
LI
LIII
[ss]
[ss]
[ss]
XLIX
LII
LIV
[tt]
[tt]
[jj]
L
IF-7
LV
[uu]
LVI
[vv]
[nn]
IF-6
IF-8

In Scheme F and G, each step can be effected using methods known per se. For example, step [ii] may be carried out by methods hereinbefore described for step [dd] in Scheme E.

Step [jj] may be carried out by using a mixture of tetrahydrofuran, acetic acid and water, a mixture of p-toluenesulfonic acid and anhydrous methanol, or a mixture of diluted hydrochloric acid and THF, at room temperature or with heating.

Step [kk], [ll], [mm], [nn], [oo] and [pp] may be carried out by methods hereinbefore described for step [n], [o], [p], [q], [r] and [s] in Scheme B, respectively.

Step [qq] and [rr] may be carried out by methods hereinbefore described for step [n] in Scheme B, by using compounds of the general formula:

$HOOC-R^3-R^{4b}$ LVII

and $HOOC-R^3-O-THP$ LVIII

(wherein the various symbols are as hereinbefore defined), respectively, instead of the compounds of the general formula XVIII.

Step [ss] may be carried out by the methods hereinbefore described for step [ee] in Scheme E.

Step [tt] may be carried out by using triethylamine in a lower alkanol such as methanol, ethanol, isopropyl alcohol at a temperature from ambient to the reflux temperature of the

reaction mixture.

Step [uu] may be carried out by methods hereinbefore described for step [a] in Scheme A.

Step [vv] may be carried out by reacting with compounds of the general formula: $N(R^{10})_3$, in which $R^{10}$ is as hereinbefore defined, in a lower alkanol such as methanol, ethanol or isopropyl alcohol in the presence of water at a temperature from ambient to the reflux temperature of the reaction mixture to obtain a sulfonate (i.e. compounds of the general formula IF-8, wherein $\overset{\ominus}{Y}$ represents $\overset{\ominus}{O}T$), and, if desired, performing salt exchanging reaction hereinafter described.

Glycerol derivatives of the general formula I, wherein $R^4$ represents a group of the general formula: $-\overset{\oplus}{N}(R^{10})_3 \cdot \overset{\ominus}{Y}$, in which Y represents a halogen atom, i.e. compounds of the general formula:

$$\left[\begin{array}{l} A-R^1 \\ B-R^2 \\ D-R^3-\overset{\oplus}{N}(R^{10})_3 \cdot \overset{\ominus}{Y}{}^1 \end{array}\right. \qquad \text{IG}$$

(wherein $Y^1$ represents a halogen atom and the other symbols are as hereinbefore defined) may be prepared by the reaction of compounds of the general formula:

$$\left[\begin{array}{l} A-R^1 \\ B-R^2 \\ D-R^3-N(R^{10})_2 \end{array}\right. \qquad \text{IH}$$

(wherein the various symbols are as hereinbefore defined) with an alkyl halide such as methyl iodide in a lower alkanol such as methanol or ethanol at a temperature from ambient to the reflux temperature of the reaction mixture.

Glycerol derivatives of the general formula I, wherein the group $-B-R^2$ represents the group of the general formula: $-O-\underset{\underset{O}{\|}}{C}-R^6$ and $R^4$ represents the group of the general formula: $-NHCOR^9$, and further, $R^6$ and $R^9$ represent the same group, i.e. compounds of the general formula:

$$\left[\begin{array}{l} A-R^1 \\ O-CO-R^{13} \\ D-R^3-NHCOR^{13} \end{array}\right. \qquad IJ$$

(wherein two $R^{13}$, being the same, represent alkyl groups of 1 to 6 carbon atoms or phenyl groups, and the other symbols are as hereinbefore defined) may be prepared from a compound of the general formula:

$$\left[\begin{array}{l} A-R^1 \\ OH \\ D-R^3-NH_2 \end{array}\right. \qquad IA\text{-}6$$

(wherein the various symbols are as hereinbefore defined) by methods hereinbefore described for step [m] in Scheme A.

Glycerol derivatives of the general formula IA-6, wherein D represents an oxygen atom, i.e. compounds of the general formula:

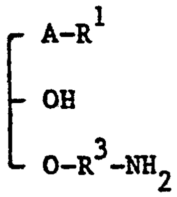

IA-7

(wherein the various symbols are as hereinbefore defined) may be prepared by the reaction of a compound of the general formula:

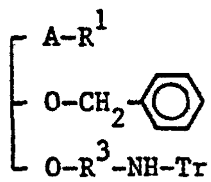

LIX

(wherein the various symbols are as hereinbefore defined) under acidic conditions under which both a benzyl group and a trityl group are eliminated at the same time, for example, by using trifluoroacetic acid at room temperature.

Starting materials in the hereinbefore described procedure may be prepared by various methods known per se, for example, by the series of reactions depicted schematically below in Scheme H, wherein the various symbols are as hereinbefore described.

Scheme H

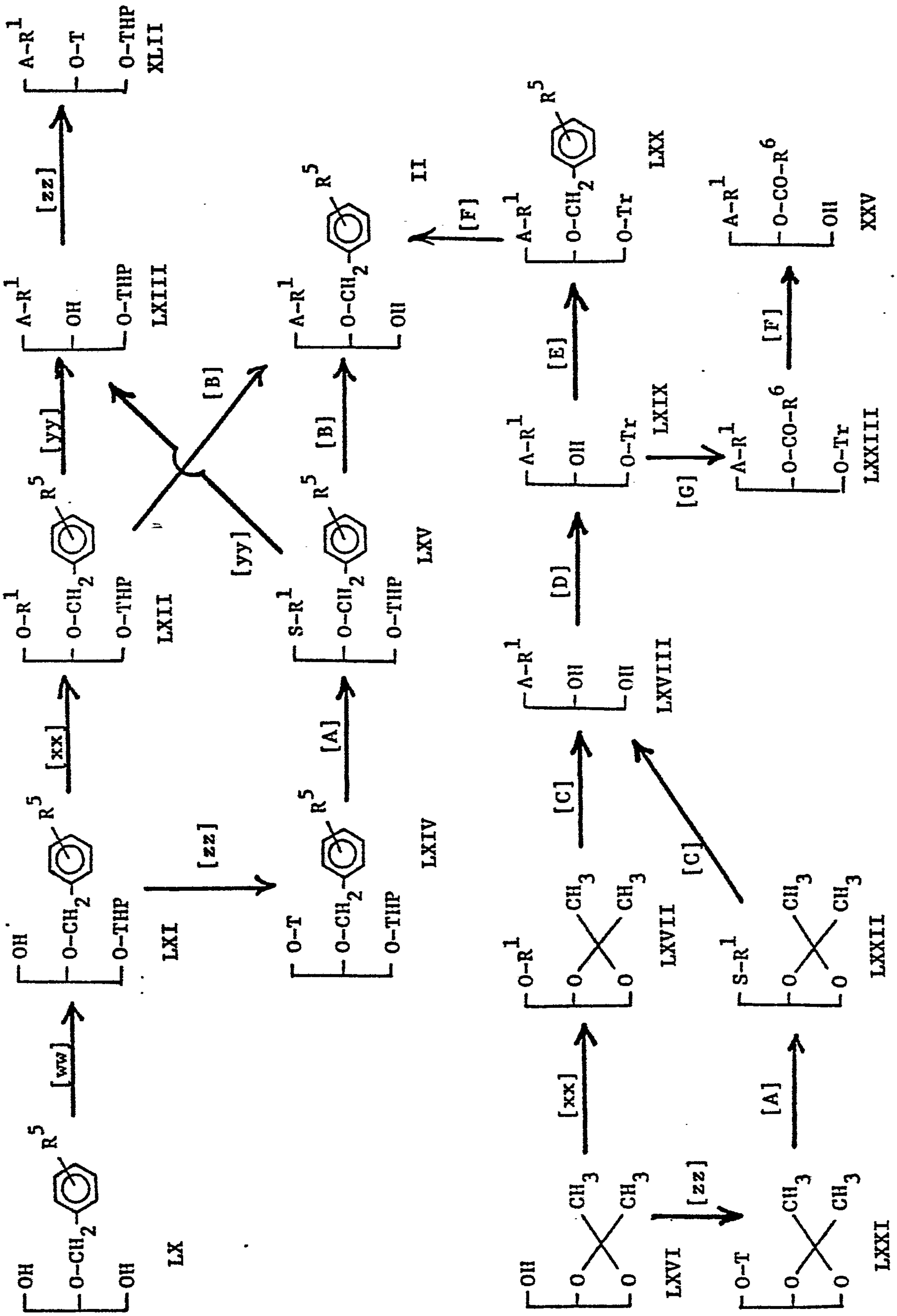


LX LXI LXII LXIII XLII LXIV LXV II LXVI LXVII LXVIII LXIX LXX LXXI LXXII LXXIII XXV

Scheme H (continued)

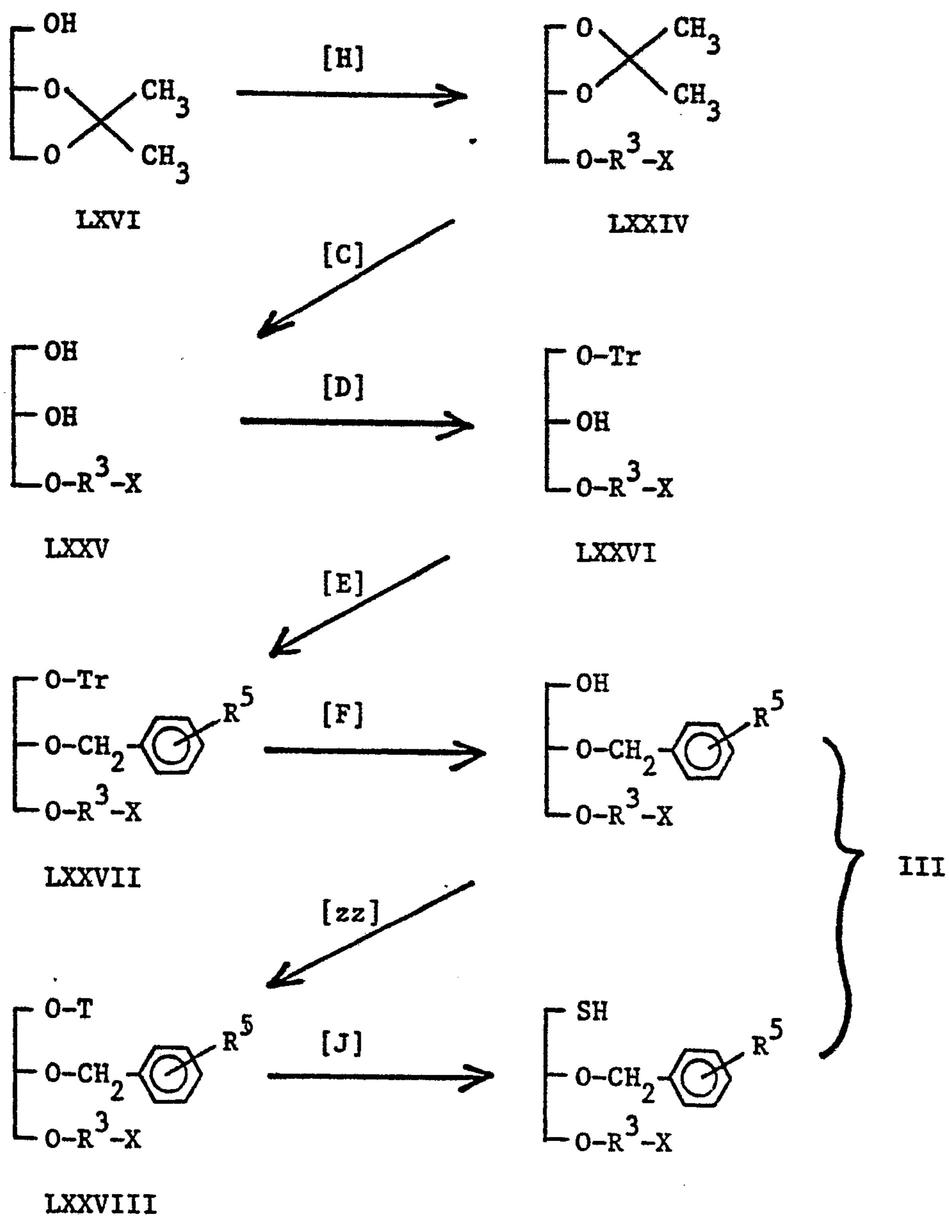


LXVI

LXXIV

LXXV

LXXVI

LXXVII

III

LXXVIII

In Scheme H, each step can be effected using methods known *per se*. For example, step [ww] may be conducted by using 2,3-dihydropyran in methylene chloride in the presence of p-toluenesulfonic acid.

Step [xx] may be carried out by methods hereinbefore described for step [d] in Scheme A.

Step [yy] may be carried out by methods hereinbefore described for step [t] in Scheme C, using a compound of the general formula LXII or LXV, wherein $R^5$ is a hydrogen atom.

Step [zz] may be carried out by methods hereinbefore described for step [a] in Scheme A.

Step [A] may be carried out by methods hereinbefore described for step [dd] by using a compound of the general formula:

$$HS\text{-}R^1 \qquad \text{LXXIX}$$

(wherein $R^1$ is as hereinbefore defined) instead of the compound of the general formula XXXIX.

Step [B] may be carried out by methods hereinbefore described for step [jj] in Scheme F.

Step [C] may be carried out by using concentrated hydrochloric acid in an alcohol such as methanol at a reflux temperature.

Step [D] may be carried out by using trityl chloride in pyridine at a temperature from 50°C to 90°C.

Step [E] may be carried out by reacting with a benzyl halide substituted by $R^5$ in the presence of sodium hydride in an inert organic solvent such as chloroform, methylene chloride, THF or DMF, or mixture of two or more of them at a temperature from 0°C to 40°C, preferably at ambient.

Step [F] may be carried out by methods hereinbefore described for step [g] in Scheme A.

Step [G] may be carried out by methods hereinbefore described for step [v] in Scheme C.

Step [H] may be carried out by methods hereinbefore described for step [c] in Scheme A.

Step [J] may be carried out by methods hereinbefore described for step [ss] and [tt] in Scheme G.

Glycerol derivatives of the general formula I, wherein $R^4$ represents the group of the general formula: $-\overset{\oplus}{N}(R^{10})_3 \cdot \overset{\ominus}{Y}{}^2$, in which $\overset{\ominus}{Y}{}^2$ is an anion of an acid other than a halogen ion and $R^{10}$ is as hereinbefore defined, may be easily prepared from a glycerol derivatives of the general formula I, wherein $R^4$ represents the group of the general formula: $-\overset{\oplus}{N}(R^{10})_3 \cdot \overset{\ominus}{Y}{}^1$, in which $R^{10}$ and $Y^1$ are as hereinbefore defined, by salt-exchange, for example, by neutralizing of a compound of the general formula I, wherein $R^4$ represents the group of the general formula: $-\overset{\oplus}{N}(R^{10})_3 \cdot \overset{\ominus}{Y}{}^1$ with an aqueous solution of sodium

hydroxide, and then reacting with a desirable inorganic or organic acid, or by ion-exchange resin.

Glycerol derivatives of the general formula I, wherein $R^4$ represents an amino group or the group of the general formula: $-NHR^{10}$ or $-N(R^{10})_2$ may be converted into acid addition salts thereof. Preferably, acid addition salts are non-toxic salts and are water-soluble. Suitable acid addition salts, are, for example, inorganic acid salts such as hydrochloride, hydrobromide, hydroiodide, sulfate, phosphorate, nitrate, or organic acid salts such as acetate, lactate, tartrate, benzoate, citrate, methanesulphonate, ethanesulphonate, benzenesulphonate, toluenesulphonate or isethionate. Acid addition salts may be prepared by methods known per se, for example, reacting of stoichiometric quantities of a compound of the general formula I, wherein $R^4$ represents an amino group or the group of the general formula: $-NHR^{10}$ or $-N(R^{10})_2$ and a desirable acid in a suitable solvent.

Glycerol derivatives of the general formula I and acid addition salts thereof have antagonistic effect on PAF, hypotensive effect like PAF, inhibitory effect on phospholipase and further, inhibitory effect on growth of tumour cell or induction of differentiation, and are, therefore, useful as a platelet aggregation inhibitor, anti-asthmatic agent,

anti-allergic agent, anti-inflammatory agent, hypotensive agent and anti-tumour agent for mammals including human beings, especially human beings. These effect were confirmed by standard laboratory test and, for example, antagonistic effect on PAF and inhibitory effect on phospholipase was confirmed by the following screening systems.

(1) Inhibitory effect on PAF-induced platelet aggregation

A mixture of 9 parts of blood obtained from male guinea-pig, and one part of 3.8% trisodium citrate was centrifuged for 10 minutes at 120 x g at room temperature to obtain platelet rich plasma (PRP). Using obtained PRP, PAF (10nM)-induced aggregation was determined by the method of Born [cf. J. Physiol., 162, 67 (1962)].

(2) Inhibitory effect on phospholipase A

The activity of the enzyme was examined by modification of the method of Shakir by using L-ß-[1-$^{14}$C]-arachidonyl-α'-stearoyl-phosphatidylcholine as substrate and phospholipase $A_2$ obtained from lung of guinea-pig as enzyme [cf. Anal. Biochem., 114, 67 (1981)].

(3) Inhibitory effect on phospholipase C

The activity of the enzyme was examined by the method of Rittenhouse-Simmons by using $^{3}H$-myoinsitol labelled phosphatidyl-inositol biosynthesized in microsome of liver in rat as substrate and phospholipase C obtained from lung of guinea-pig as enzyme [cf. J. Clin. Invest., 63, 580 (1979)].

The evaluation is conducted using the concentration of compounds of the present invention required to produce a 50% inhibition of each effect, i.e. $IC_{50}$. The results are shown in the following table.

| Example No. of tested compounds | Inhibitory effect on PAF-induced platelet aggregation ($IC_{50}$, M) | Inhibitory effect on phospholipase $A_2$ ($IC_{50}$, M) | Inhibitory effect on phospholipase C ($IC_{50}$, M) |
|---|---|---|---|
| 1 (g) | $9.4 \times 10^{-6}$ | $1.3 \times 10^{-4}$ | $1.8 \times 10^{-4}$ |
| 1 (v) | $8.5 \times 10^{-6}$ | $1.2 \times 10^{-4}$ | $2.5 \times 10^{-4}$ |
| 5 (d) | $1.1 \times 10^{-5}$ | $2.4 \times 10^{-4}$ | $6.6 \times 10^{-4}$ |
| 12 (b) | $8.7 \times 10^{-6}$ | | |
| 12 (n) | $7.0 \times 10^{-6}$ | | |
| 12 (o) | $8.9 \times 10^{-6}$ | | |
| 12 (s) | $4.3 \times 10^{-6}$ | $2.0 \times 10^{-4}$ | $2.0 \times 10^{-4}$ |
| 12 (x) | $3.8 \times 10^{-6}$ | $2.4 \times 10^{-4}$ | $2.0 \times 10^{-4}$ |

On the other hand, it was confirmed that the acute toxicity of all extent of the compounds of the present invention was more than 30 mg/kg by intravenous administration. Therefore, glycerol derivatives of the present invention may be considered to be sufficiently safe and suitable for medical use. For example, the mortality (dead/used) in glycerol derivatives of the present invention, i.e. compounds prepared in Example 1(g), 1(v), 5(d), 12(b), 12(n), 12(o), 12(s) and 12(x) was 0/10, 0/10, 0/10, 1/10/ 0/10, 0/10, 0/10 and 0/10, respectively, when each glycerol compound was intravenously administered to tail vein in ten mice at the dose of 30 mg/kg.

For the purpose hereinbefore described, glycerol derivatives of the general formula I, or non-toxic acid addition salt thereof may normally be administered systemically or partially usually by oral or parenteral (e.g. intravenous, subcutaneous or intramuscular) administration. The dose to be administered is determined depending upon, for example, age, body weight, symptoms, the desired therapeutic effect, the route of administration, and the duration of the treatment.

In the human adult, the doses per person are generally between 1 mg and 1 g, preferably between 20 and 200 mg by oral administration, and between 100 μg and 100 mg, preferably between and 1 and 10 mg by parenteral administration and can be administered up to several times per day.

As mentioned above, the doses to be used depend on various conditions. Therefore, there may be cases in which doses greater than the ranges specified above, or lower than the ranges specified above, may be used.

The present invention includes within its scope pharmaceutical compositions which comprise at least one glycerol derivatives of the general formula I, or non-toxic acid addition salt thereof, together with a pharmaceutical carrier or coating.

Solid compositions according to the present invention for oral administration include compressed tablets, pills, dispersible powders and granules. In such solid compositions, one or more of the active compound(s) is, or are, admixed with at least one inert diluent such as lactose, mannitol, glucose, hydroxypropyl cellulose, microcrystalline cellulose, starch, polyvinylpyrrolidone or magnesium metasilicate aluminate. The compositions may also comprise, as is normal practice, additional substances other than inert diluents e.g. lubricating agents such as magnesium stearate, and, disintegrating agents such as cellulose calcium gluconate. The tablets or pills may, if desired, be made into enteric film-coated or gastric film-coated tablets or pills, such as sugar-coated, gelatin-coated, hydroxypropylcellulose-coated or hydroxypropylmethylcellulose phthalate-coated tablets or pills; two or more layers may be used.

The compositions for oral administration also include capsules of absorbable material such as gelatin, containing one or more of the active substances with or without the addition of diluents or excipients.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise at least one compound of the present invention.

Preparations according to the present invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, Polysorbate 80 (registered Trade Mark). These compositions may also include ajuvants such as preserving,

wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions include, for parenteral administration, liquids for external use, and endermic liniments such as ointments; suppositories for rectal administration; and pessaries for vaginal administration. Such compositions are prepared by known methods.

The percentage of active ingredient in the compositions of the invention may be varied, it being necessary that it should constitute a proportion such that a suitable dosage for the therapeutic effect desired shall be obtained. Obviously several unit dosage forms may be administered at about the same time. In general, the preparations should normally contain at least 0.025% by weight of active substance when required for administration by injection; for oral administration the preparations will normally contain at least 0.1% by weight of active substance.

Preferred glycerol derivatives of the present invention are as follows:

(2RS)-1-O-hexadecyl-3-O-(4-aminobutyl)glycerol,

(2RS)-1-O-hexadecyl-3-O-[4-(N,N-dimethylamino)butyl]glycerol,

(2RS)-1-O-hexadecyl-3-O-[4-(N,N,N-trimethylammonio)butyl]glycerol halide,

(2RS)-1-O-hexadecyl-3-O-[8-(N,N-dimethylamino)octyl]glycerol,

(2RS)-1-hexadecylthio-3-O-[4-(N,N-dimethylamino)butyl]-2,3-propanediol,

(2RS)-1-O-hexyl-2-O-benzyl-3-O-[4-(N,N-dimethylamino)butyl]-glycerol,

(2RS)-1-O-hexyl-2-O-benzyl-3-O-[4-(N,N,N-trimethylammonio)butyl]-glycerol halide,

(2RS)-1-O-dodecyl-2-O-benzyl-3-O-[4-(N,N-dimethylamino)butyl]-glycerol,

(2RS)-1-O-dodecyl-2-O-benzyl-3-O-[4-(N,N,N-trimethylammonio)butyl]-glycerol halide,

(2RS)-1-O-hexadecyl-2-O-benzyl-3-O-(4-aminobutyl)glycerol,

(2RS)-1-O-hexadecyl-2-O-benzyl-3-O-[4-(N,N-dimethylamino)butyl]-glycerol,

(2RS)-1-O-hexadecyl-2-O-benzyl-3-O-[4-(N,N,N-trimethylammonio)-butyl]glycerol halide,

(2RS)-1-O-hexadecyl-2-O-benzyl-3-O-[4-(N,N-diethylamino)butyl]-glycerol,

(2RS)-1-O-hexadecyl-2-O-benzyl-3-O-[4-(N,N-diethyl-N-methylammonio)butyl]glycerol halide,

(2RS)-1-O-hexadecyl-2-O-benzyl-3-O-[4-(N,N,N-triethylammonio)-butyl]glycerol halide,

(2RS)-1-O-hexadecyl-2-O-benzyl-3-O-[4-(N,N-dipropylamino)butyl]-glycerol,

(2RS)-1-O-hexadecyl-2-O-benzyl-3-O-[4-(N,N-dipropyl-N-methylammonio)butyl]glycerol halide,

(2RS)-1-O-hexadecyl-2-O-benzyl-3-O-[4-(N,N-di-isopropylamino)-butyl]glycerol,

(2RS)-1-O-hexadecyl-2-O-benzyl-3-O-[4-(N,N-di-isopropyl-N-methylammonio)butyl]glycerol halide,

(2RS)-1-O-hexadecyl-2-O-benzyl-3-O-[4-(N,N-dibutylamino)butyl]-glycerol,

(2RS)-1-O-hexadecyl-2-O-benzyl-3-O-[4-(N,N-dibutyl-N-methylammonio)butyl]glycerol halide,

(2RS)-1-O-hexadecyl-2-O-benzyl-3-O-[4-(N,N-dipentylamino)butyl]-glycerol,

(2RS)-1-O-hexadecyl-2-O-benzyl-3-O-[4-(N,N-dipentyl-N-methylammonio)butyl]glycerol halide,

(2RS)-1-O-hexadecyl-2-O-benzyl-3-O-[4-(N,N-dihexylamino)butyl]-glycerol,

(2RS)-1-O-hexadecyl-2-O-benzyl-3-O-[4-(N,N-dihexyl-N-methylammonio)butyl]glycerol halide,

(2RS)-1-O-hexadecyl-2-O-benzyl-3-O-[5-(N,N-dimethylamino)pentyl]-glycerol,

(2RS)-1-O-hexadecyl-2-O-benzyl-3-O-[5-(N,N,N-trimethylammonio)-pentyl]glycerol halide ,

(2RS)-1-O-hexadecyl-2-O-benzyl-3-O-[6-(N,N,N-trimethylammonio)-hexyl]glycerol halide ,

(2RS)-1-O-hexadecyl-2-O-benzyl-3-O-[7-(N,N,N-trimethylammonio)-heptyl]glycerol halide ,

(2RS)-1-O-hexadecyl-2-O-benzyl-3-O-[8-(N,N-dimethylamino)octyl]-glycerol,

(2RS)-1-O-hexadecyl-2-O-benzyl-3-O-[8-(N,N,N-trimethylammonio)-octyl]glycerol halide ,

(2RS)-1-O-hexadecyl-2-O-benzyl-3-O-[9-(N,N,N-trimethylammonio)-nonyl]glycerol halide ,

(2RS)-1-O-hexadecyl-2-O-benzyl-3-O-[10-(N,N,N-trimethylammonio)-decyl]glycerol halide ,

(2RS)-1-O-hexadecyl-2-O-benzyl-3-O-(3-aminopropionyl)glycerol ,

(2RS)-1-O-hexadecyl-2-O-benzyl-3-O-[3-(N,N,N-trimethylammonio)-propionyl]glycerol halide ,

(2RS)-1-O-hexadecyl-2-O-benzyl-3-O-[4-(N,N,N-trimethylammonio)-butyryl]glycerol halide ,

(2RS)-1-O-octadecyl-2-O-(4-chlorophenyl)methyl-3-O-[4-(N,N-dimethylamino)butyl]glycerol ,

(2RS)-1-O-octadecyl-2-O-(4-chlorophenyl)methyl-3-O-[4-(N,N,N-trimethylammonio)butyl]glycerol halide,

(2RS)-1-O-eicosyl-2-O-benzyl-3-O-[4-(N-methylamino)butyl]glycerol,

(2RS)-1-hexadecylthio-2-O-benzyl-3-O-[4-(N,N-dimethylamino)butyl]-2,3-propanediol,

(2RS)-1-hexadecylthio-2-O-benzyl-3-O-[4-(N,N,N-trimethylammonio)-butyl]-2,3-propanediol halide,

(2RS)-1-O-hexadecyl-2-O-acetyl-3-O-(3-aminopropyl)glycerol,

(2RS)-1-O-hexadecyl-2-O-acetyl-3-O-[3-(N-acetylamino)propyl]-glycerol,

(2RS)-1-O-hexadecyl-2-O-acetyl-3-O-[4-(N-acetylamino)butyl]-glycerol,

(2RS)-1-O-hexadecyl-2-O-acetyl-3-O-[4-(N,N-dimethylamino)butyl]-glycerol,

(2RS)-1-O-hexadecyl-2-O-acetyl-3-O-[4-(N,N,N-trimethylammonio)-butyl]glycerol halide ,

(2RS)-1-O-hexadecyl-2-O-butyryl-3-O-[4-(N,N-dimethylamino)butyl]-glycerol,

(2RS)-1-O-hexadecyl-2-O-butyryl-3-O-[4-(N,N,N-trimethylammonio)-butyl]glycerol halide,

(2RS)-1-O-hexadecyl-2-O-hexanoyl-3-O-[4-(N,N-dimethylamino)butyl]-glycerol,

(2RS)-1-O-hexadecyl-2-O-hexanoyl-3-O-[4-(N,N,N-trimethylammonio)-butyl]glycerol halide,

(2RS)-1-O-hexadecyl-2-O-benzoyl-3-O-[4-(N,N-dimethylamino)butyl]-glycerol,

(2RS)-1-O-hexadecyl-2-O-benzoyl-3-O-[4-(N,N,N-trimethylammonio)-butyl]glycerol halide,

(2RS)-1-O-octadecyl-2-O-acetyl-3-O-(3-aminopropionyl)glycerol,

(2RS)-1-O-hexadecyl-2-O-(N-methylcarbamoyl)-3-O-[4-(N,N-dimethyl-amino)butyl]glycerol,

(2RS)-1-O-hexadecyl-2-O-(N-methylcarbamoyl)-3-O-[4-(N,N,N-trimethylammonio)butyl]glycerol halide,

(2RS)-1-O-hexadecyl-2-O-(N-propylcarbamoyl)-3-O-[4-(N,N-dimethyl-amino)butyl]glycerol,

(2RS)-1-O-hexadecyl-2-O-(N-propylcarbamoyl)-3-O-[4-(N,N,N-trimethylammonio)butyl]glycerol halide,

(2RS)-1-O-hexadecyl-2-O-(N-butylcarbamoyl)-3-O-[4-(N,N-dimethyl-amino)butyl]glycerol,

(2RS)-1-O-hexadecyl-2-O-(N-butylcarbamoyl)-3-O-[4-(N,N,N-trimethylammonio)butyl]glycerol halide,

(2RS)-1-O-hexadecyl-2-O-(N-phenylcarbamoyl)-3-O-[4-(N,N-dimethylamino)butyl]glycerol,

(2RS)-1-O-hexadecyl-2-O-(N-phenylcarbamoyl)-3-O-[4-(N,N,N-trimethylammonio)butyl]glycerol halide,

(2RS)-1-O-hexadecyl-2-mercapt-3-O-[4-(N,N-dimethylamino)butyl]-1,3-propanediol,

(2RS)-1-O-hexadecyl-2-ethylthio-3-O-[4-(N,N-dimethylamino)butyl]-1,3-propanediol,

(2RS)-1-O-hexadecyl-2-ethylthio-3-O-[4-(N,N,N-trimethylammonio)-butyl]-1,3-propanediol halide,

(2RS)-1-O-hexadecyl-2-pentylthio-3-O-[8-(N,N-dimethylamino)octyl]-1,3-propanediol,

(2RS)-1-O-hexadecyl-2-pentylthio-3-O-[8-(N,N,N-trimethylammonio)-octyl]-1,3-propanediol halide,

(2RS)-1-O-hexadecyl-2-benzylthio-3-O-(4-aminobutyl)-1,3-propanediol,

(2RS)-1-O-hexadecyl-2-benzylthio-3-O-[4-(N-acetylamino)butyl]-1,3-propanediol,

(2RS)-1-O-hexadecyl-2-benzylthio-3-O-[4-(N,N-dimethylamino)-butyl]-1,3-propanediol,

(2RS)-1-O-hexadecyl-2-benzylthio-3-O-[4-(N,N,N-trimethylammonio)-butyl]-1,3-propanediol halide,

(2RS)-1-O-hexadecyl-2-(4-chlorophenyl)methylthio-3-O-[4-(N,N-dimethylamino)butyl]-1,3-propanediol,

(2RS)-1-O-hexadecyl-2-(4-chlorophenyl)methylthio-3-O-[4-(N,N,N-trimethylammonio)butyl]-1,3-propanediol halide,

(2RS)-1-O-hexadecyl-2-[ethoxy(thiocarbonyl)thio]-3-O-[4-(N,N-dimethylamino)butyl]-1,3-propanediol,

(2RS)-1-O-hexadecyl-2-[ethoxy(thiocarbonyl)thio]-3-O-[4-(N,N,N-trimethylammonio)butyl]-1,3-propanediol halide,

(2RS)-1-O-hexadecyl-2-[phenoxy(thiocarbonyl)thio]-3-O-[4-(N,N-dimethylamino)butyl]-1,3-propanediol,

(2RS)-1-O-hexadecyl-2-[phenoxy(thiocarbonyl)thio]-3-O-[4-(N,N,N-trimethylammonio)butyl]-1,3-propanediol halide,
(2RS)-1-hexadecylthio-2-benzylthio-3-O-[4-(N,N-dimethylamino)-butoxy]propane and
(2RS)-1-hexadecylthio-2-benzylthio-3-O-[4-(N,N,N-trimethylammonio)-butoxy]propane halide
and non-toxic acid addition salts thereof.

The following Reference Examples and Examples illustrate, but not limit, the preparation of compounds of the present invention. In Reference Examples and Examples, "washed" indicates that a solution was washed with any one of suitable solutions such as water, a saturated aqueous solution of sodium chloride, diluted hydrochloric acid and a saturated aqueous solution of sodium bicarbonate, or two or more of them, in suitable rotation. "Dried" indicates that a solution was dried over any one of suitable drying agents such as magnesium sulfate, sodium sulfate and potassium carbonate. "Concentrated" indicates that a solution was concentrated under reduced pressure. The solvents in parentheses in chromatographic separation show the eluent or the developing solvent used. Where solvent ratios are specified in TLC, the ratios are by volume. Except when specified otherwise, IR is recorded by the liquid film method, and NMR is recorded in deuterochloroform ($CDCl_3$) solution.

Various abbreviated words in Reference Example and Examples donate the following words:

THP : 2-tetrahydropyranyl group,

Tr : trityl group,

Ts : tosyl group,

cbz : benzyloxycarbonyl group,

boc : tert-butoxycarbonyl group,

Ms : mesyl group,

AcOEt: ethyl acetate,

DMF : dimethylformamide,

$CH_2Cl_2$: methylene chloride,

MeOH : methanol,

AcOH : acetic acid,

THF : tetrahydrofuran,

HMPA : hexamethylphosphoramide,

$CHCl_3$: chloroform,

TLC : thin layer chromatography,

IR : Infrared absorption spectrum,

NMR : nuclear magnetic resonance spectrum,

Mass (or MS): Mass spectrum.

Reference Example 1

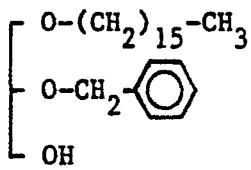

(i) A mixture of 49 g of 2-O-benzylglycerol, 22.6 g of 2,3-dihydropyran, 200 mg of p-toluenesulfonic acid and 300 ml of $CH_2Cl_2$ was stirred for 30 minutes at room temperature. The reaction mixture was neutralized with a saturated aqueous solution of sodium dicarbonate and the solution was extracted with AcOEt. The extract was washed, dried and concentrated. The residue was purified by column chromatography on silica gel (a mixture of AcOEt and cyclohexane) to give 37.4 g of (2RS)-2-O-benzyl-3-O-(2-tetrahydropyranyl)glycerol having the following physical data:

TLC ($CHCl_3$:acetone=10:1): Rf=0.39.

(ii) Under an atmosphere of argon, to a suspension of 10.4 g of sodium hydride (content: 64.1%) in 500 ml of dry DMF was added a solution of 37 g of the glycerol compound prepared in the above (i) in 150 ml of dry DMF. The mixture was stirred for one hour at 60°C. After cooling to 40°C, thereto was added a solution of 84.8 g of hexadecyl bromide in 150 ml of dry DMF and

the mixture obtained was stirred for 10 minutes at room temperature and further, for 3 hours at 60 - 80°C. The reaction mixture was washed and concentrated. The residue was purified by column chromatography on silica gel (a mixture of $CH_2Cl_2$ and n-hexane, following $CH_2Cl_2$ only) to give 57 g of (2RS)-1-O-hexadecyl-2-O-benzyl-3-O-(2-tetrahydropyranyl)glycerol having the following physical data:

TLC (AcOEt:cyclohexane=1:5): Rf=0.63.

(iii) A mixture of 57 g of the 3-O-(2-tetrahydropyranyl)-glycerol compound prepared in the above (ii), 150 ml of $CH_2Cl_2$, 300 ml of MeOH and 500 mg of p-toluenesulfonic acid was stirred for 4 hours at room temperature. To the reaction mixture was added 100 ml of a saturated aqueous solution of sodium bicarbonate and concentrated to distill off MeOH. To the residue was added 2 liters of AcOEt and the solution was washed and concentrated. The residue was purified by column chromatography on silica gel (a mixture of $CH_2Cl_2$ and AcOEt) to give 34.8 g of the title compound having the following physical data:

TLC (AcOEt:cyclohexane=1:5): Rf=0.24;

NMR: δ=7.2 (5H, s), 4.6 (2H, s), 3.8-3.2 (7H, m), 2.3-2.0 (1H, m), 1.7-0.7 (31H, m);

IR: ν=3450, 2920, 2840, 1460, 1110 $cm^{-1}$.

Reference Example 2

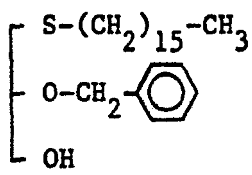

(i) A mixture of 1.02 g of (2RS)-2-O-benzyl-3-O-(2-tetrahydropyranyl)glycerol [prepared in Reference Example 1(i)], 1 ml of triethylamine 15 ml of $CH_2Cl_2$ and 0.37 ml of mesyl chloride was stirred for, 15 minutes with cooling in an ice-bath. To the mixture was added 80 ml of AcOEt and the solution thus obtained was washed, dried and concentrated to give (2RS)-1-O-mesyl-2-O-benzyl-3-O-(2-tetrahydropyranyl)-glycerol as crude product having the following physical data: TLC ($CH_2Cl_2$:AcOEt=10:1): Rf=0.58.

(ii) Under an atmosphere of argon, a mixture of 4.8 g of hexadecanthiol, 750 mg of sodium hydride (content: 64.1%) and 35 ml of dry THF was stirred for one hour at 40-50°C, and thereto was added 10 ml of HMPA, and further added a solution of the 1-O-mesylglycerol compound prepared in the above (i) in 10 ml of dry THF. The mixture thus obtained was refluxed for 2 hours. The reaction mixture was poured into an ice-water and extracted

with AcOEt and the extract was dried and concentrated to give (2RS)-1-hexadecylthio-2-O-benzyl-3-O-(2-tetrahydropyranyl)-2,3-propanediol as a crude product having the following physical data:

TLC (AcOEt:n-hexane): Rf=0.55.

(iii) By the same procedure as described in Reference Example 1(iii), 1.0 g of the title compound having the following physical data was prepared from the 3-O-(2-tetrahydropyranyl)-propanediol compound prepared in the above (ii):

TLC (AcOEt:n-hexane=1:5): Rf=0.22;

NMR: δ=7.2 (5H, s), 4.56 (2H, q), 3.86-3.43 (3H, m), 2.8-2.2 (4H, m), 2.2-0.7 (32H, m);

IR: ν=3450, 2930, 2860, 1460 $cm^{-1}$;

Mass: m/e=422 ($M^+$), 391, 315.

Reference Example 3

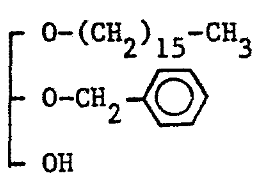

(i) By the same procedure as described in Reference Example 1 (ii), (2RS)-1-O-hexadecylglycerol dimethylketal having

the following physical data was prepared from glycerol dimethylketal.

TLC (cyclohexane:AcOEt=5:1): Rf=0.64.

(ii) A mixture of 11 g of the dimethylketal compound prepared in the above (i), 90 ml of MeOH and 11 ml of a concentrated hydrochloric acid was refluxed for one hour. After cooling, the reaction mixture was concentrated to distill off MeOH. The residue was extracted with diethyl ether and the extract was washed, and concentrated to give 9.76 g of (2RS)-1-O-hexadecylglycerol as white crystals having the following physical data:

IR (KBr method): ν=3350, 2910, 2840, 1460, 1320, 1120, 1055 $cm^{-1}$.

(iii) A mixture of 13.36 g of the glycerol compound prepared in the above (ii), 84 ml of pyridine and 17.7 g of trityl chloride was stirred for 2 hours at 80°C. The reaction mixture was poured into 240 ml of ice-water and acidified with a concentrated hydrochloric acid and extracted with diethyl ether. The extract was washed, dried and concentrated. The residue was purified by column chromatography on silica gel (a mixture of cyclohexane and ethyl acetate) to give 23 g of (2RS)-1-O-hexadecyl-3-O-tritylglycerol having the following physical data:

NMR: δ=7.50-7.10 (15H, m), 3.66-2.33 (7H, m), 1.66-1.10 (28H, m), 0.87 (3H, t).

(iv) Under an atmosphere of nitrogen, 1.697 g of sodium hydride (content: 64.1%) was added all at once to a solution of 23 g of the trityl compound prepared in the above (iii) in 123 ml of DMF. After completion of the evolution of hydrogen gas, 5.63 ml of benzyl bromide was added dropwise to the solution at room temperature. The solution was stirred for one hour at 80°C and concentrated to distill off DMF. The residue was dissolved in 600 ml of diethyl ether and washed, dried and concentrated to give 28 g of (2RS)-1-O-hexadecyl-2-O-benzyl-3-O-tritylglycerol as crude compound having the following physical data:
TLC (cyclohexane:ethyl acetate=5:1): Rf=0.84.

(v) To a solution of 28 g of the trityl compound prepared in the above (iv) in a mixture of 200 ml of $CHCl_3$ and 40 ml of MeOH, was added dropwise 41.2 ml of a 30% solution of hydrogen bromide in AcOH at 0°C. The solution was stirred for 30 minutes at 0°C and poured into 200 ml of ice-water and extracted with diethyl ether. The extract was washed, dried and concentrated. The residue was purified by column chromatography on silica gel (a mixture of cyclohexane and AcOEt) to give 11.5 g of the title

compound having the same physical data as the compound prepared in Reference Example 1.

Reference Example 4

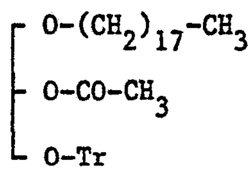

A mixture of 1 g of (2RS)-1-O-octadecyl-3-O-tritylglycerol [prepared by the same procedure as described in FEBS LETTERS, 116, 161 (1980)], 2 ml of acetic anhydride and 8 ml of pyridine was stirred overnight. AcOEt was added to the reaction mixture and the solution thus obtained was washed, dried and concentrated to give 1.1 g of the title compound having the following physical data:

TLC (AcOEt:cyclohexane=1:5): Rf=0.54;

NMR: δ=7.6-6.7 (15H, m), 5.3-4.7 (1H, m), 3.7-2.9 (6H, m), 2.0 (3H, s), 1.6-0.5 (35H, m);

Mass: m/e=628 ($M^+$), 552, 539, 385, 369.

Reference Example 5

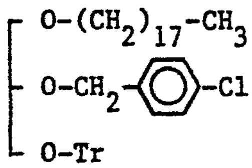

To a mixture of 293 mg of (2RS)-1-O-octadecyl-3-O-tritylglycerol [prepared by the same procedure as described in FEBS LETTERS, 116, 161 (1980)], 24 mg of sodium hydride, 18.4 mg of tetrabutylammonium iodide and 10 ml of THF, were added 161 mg of p-chlorobenzyl chloride, and the mixture was stirred for 20 hours at 50-60°C. After addition of 48 mg of sodium hydride and 322 mg of p-chlorobenzyl chloride, the mixture was stirred for 48 hours at 50-60°C. To the reaction mixture was added 20 ml of water and the mixture was extracted with AcOEt. The extract was dried and concentrated. The residue was purified by column chromatography on silica gel (a mixture of AcOEt and cyclohexane) to give 180 mg of the title compound having the following physical data:

TLC (AcOEt:cyclohexane=10:1): Rf=0.49;

NMR: ν=7.7-6.7 (19H, m), 4.56 (2H, s), 3.8-3.2 (7H, m), 1.9-0.7 (35H, m);

IR: δ=2910, 2850, 1440, 1110 $cm^{-1}$;

Mass: m/e=585, 508.

Reference Example 6

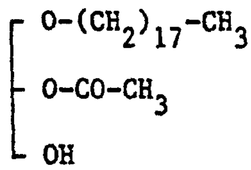

A solution of 500 mg of the trityl compound (prepared in Reference Example 4) in 8 ml of $CHCl_3$ was cooled to -40°C. To the solution were added 0.25 ml of a 30% solution of hydrogen bromide in acetic acid and the obtained solution was stirred for 30 minutes. The reaction mixture was neutralized with triethylamine and extracted with AcOEt. The extract was washed, dried and concentrated. The residue was purified by column chromatography on silica gel (a mixture of AcOEt and cyclohexane) to give 285 mg of the title compound having the following physical data:

TLC ($CHCl_3$:acetone=10:1): Rf=0.39;

NMR: δ=5.24-4.95 (1H, m), 3.7-3.3 (6H, m), 2.09 (3H, s), 1.6-1.1 (33H, m), 1.0-0.74 (3H, m);

Mass: m/e=386 ($M^+$), 368, 355, 326, 283, 281, 253, 250.

By the same procedure as described in Reference Example 6, the compound having the following physical data was prepared.

(a) 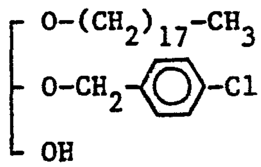

Starting material: the trityl compound prepared in Reference Example 5;

TLC ($CHCl_3$:MeOH=10:1): Rf=0.69;

NMR: δ=7.16 (4H, s), 4.56 (2H, s), 3.8-3.2 (7H, m), 2.3-1.9 (1H, m), 1.9-0.7 (35H, m);

IR: ν=3400, 2920, 2850, 1460, 1110 $cm^{-1}$;

Mass: m/e=468 ($M^+$), 343, 310.

Reference Example 7

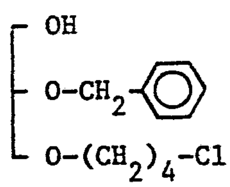

(i) Under an atmosphere of argon, to a suspension of 1.2 g of sodium hydride (content: 64.1%) in 50 ml of dry DMF, was added a solution of 4.1 g of glycerol dimethylketal in 25 ml of dry DMF, and the mixture was stirred for 15 minutes at 60°C. After cooling to room temperature, a solution of 8.1 g of 4-(p-toluene sulfonyloxy)butyl chloride in 25 ml of dry DMF was added to the mixture, and the mixture obtained was stirred for one hour at 60°C and further stirred 2 hours at 80°C. To the reaction mixture were added 5 ml of water and concentrated. To the residue were added 50 ml of water and the solution was extracted with diethyl ether. The extract was washed, dried and concentrated to give 3-O-(4-chlorobutyl)glycerol dimethylketal as crude compound having the following physical data:

TLC (AcOEt:cyclohexane=1:5): Rf=0.35.

(ii) A mixture of the glycerol compound prepared in the above (i), 50 ml of MeOH and 9 ml of a concentrated hydrochloric acid was refluxed for one hour. The reaction mixture was concentrated to give (2RS)-3-O-(4-chlorobutyl)glycerol as crude compound.

(iii) A mixture of the glycerol compound prepared in the above (ii), 13 g of trityl chloride and 100 ml of pyridine was stirred for 2 hours at 80°C. The reaction mixture was concentrated and the residue thus obtained was purified by column chromatography on silica gel ($CH_2Cl_2$ and n-hexane) to give 5 g of (2RS)-1-O-trityl-3-O-(4-chlorobutyl)glycerol having the following physical data:

TLC ($CH_2Cl_2$): Rf=0.25;

NMR: δ=7.7-6.9 (15H, m), 4.1-3.7 (1H, m), 3.7-3.0 (8H, m), 2.0-1.4 (4H, m).

(iv) Under an atmosphere of argon, to a suspension of 854 mg of sodium hydride (content: 64.1%) in 80 ml of dry DMF, were added a solution of 4.8 g of the chlorobutylglycerol compound prepared in the above (iii) in 80 ml of dry DMF, and further 4 ml of benzyl bromide. The mixture obtained was stirred for 2 hours at 60°C and to the reaction mixture were added 2 ml of water, and the mixture was concentrated. The residue was purified by column chromatography on silica gel (a mixture of AcOEt and n-hexane) to give 3.9 g of (2RS)-1-O-trityl-2-O-benzyl-3-O-(4-chlorobutyl)glycerol having the following physical data:

TLC ($CH_2Cl_2$:n-hexane=2:1): Rf=0.40;

NMR: δ=7.6-7.0 (20H, m), 4.6 (2H, s), 3.9-3.1 (9H, m), 2.0-1.5 (4H, m).

(v) A mixture of 3.9 g of the tritylglycerol compound prepared in the above (iv), 50 ml of $CHCl_3$, 20 ml of MeOH and 6 ml of a 30% solution of hydrogen bromide in acetic acid was stirred for 30 minutes with cooling in an ice-bath. To the reaction mixture were added 10 ml of water, and the solution was extracted with AcOEt. The extract was washed, dried and

concentrated. The residue was purified by column chromatography on silica gel ($CH_2Cl_2$ and a mixture of $CH_2Cl_2$ and AcOEt, successively) to give 1.8 g of the title compound having the following physical data:

TLC ($CHCl_3$:acetone=10:1): Rf=0.49;

NMR: $\delta$=7.2 (5H, s), 4.6 (2H, s), 3.8-3.2 (9H, m), 2.3-2.1 (1H, m), 2.0-1.5 (4H, m);

IR: $\nu$=3450, 2920, 2850, 1440, 1110 $cm^{-1}$;

Mass: m/e=272 ($M^+$).

Reference Example 8

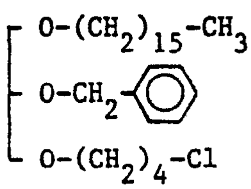

Under an atmosphere of argon, 406 mg of the 3-hydroxy compound (prepared in Reference Example 1) and 56 mg of sodium hydride were added to 4 ml of DMF and the mixture was warmed to 70°C. After completing of the evolution of hydrogen gas, the mixture was cooled to 40°C and 362 mg of 4-(p-toluene-sulfonyloxy)butyl chloride in 2 ml of DMF was added thereto and then the mixture was stirred for 5 minutes at 70°C. After

cooling, water was added to the reaction mixture and the mixture was extracted with AcOEt. The extract was washed, dried and concentrated. The residue was purified by column chromatography on silica gel (a mixture of AcOEt and cyclohexane) to give 261 mg of the title compound having the following physical data:

TLC (AcOEt:cyclohexane:$CH_2Cl_2$=2:10:1): Rf=0.65;

NMR: δ=7.18 (5H, s), 4.62 (2H, s), 3.8-3.13 (11H, m), 2.13-0.63 (37H, m);

IR: ν=2920, 2850, 1460, 1450, 1115, 735, 695 $cm^{-1}$.

By the same procedure as described in Reference Example 8, the compounds having the following physical data in Table 1 were prepared.

**Table 1**

| Reference Example No. | Reference Example No. of Starting Material | Structure of Product | Physical Data | | | |
|---|---|---|---|---|---|---|
| | | | TLC (developing solvent) | NMR | IR | MS |
| (a) | 1 | 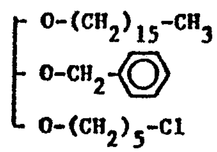 | Rf=0.60 (AcOH: cyclohexane: $CH_2Cl_2$ =2:10:1) | 6.83(5H,s), 4.57(2H,s), 3.77-3.05(11H,m), 2.30-0.97(37H,m), 0.97-0.50 (3H,t) | 3060, 3030, 2920, 2850, 1460, 1450, 1375, 1305, 1205, 1115, 1060, 730, 695 | |
| (b) | 6(a) | 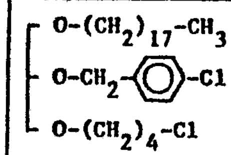 | Rf=0.70 (AcOEt: n-hexane=1:4) | 7.2(4H,s), 4.6(2H,s), 3.8-3.2(11H,m), 2.0-0.7(39H,m) | 2920, 2840, 1450, 1110 | 544($M^+$), 419 |
| (c) | 2 | 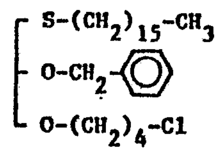 | Rf=0.59 (AcOEt: n-hexane=1:5) | 7.3-7.0(5H,m), 4.58 (2H,s), 3.76-3.2 (7H,m), 2.8-2.3(4H,m), 2.0-0.7(35H,m) | 2940, 2870, 1460, 1120 | |

Reference Example 9

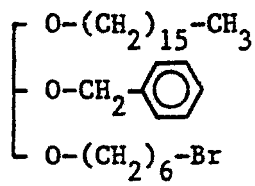

Under an atmosphere of nitrogen, a mixture of 235 mg of the 3-hydroxy compound (prepared in Reference Example 1), 26 mg of sodium hydride (contents: 64.1%) and 6 ml of DMF was stirred for 30 minutes at 80°C. To the mixture was added 0.44 ml of 1,6-dibromohexane, and the mixture was stirred for 3 hours at 80°C. The reaction mixture was adjusted to pH 4 - 5 by adding 1 ml of water and further a 1N hydrochloric acid, and extracted with AcOEt. The extract was washed and concentrated. The residue was purified by column chromatography on silica gel (a mixture of AcOEt and n-hexane) to give 85 mg of the title compound having the following physical data:

TLC (AcOEt:n-hexane=1:5): Rf=0.22;

MS: m/e=570($M^+$), 568($M^+$).

By the same procedure as described in Reference Example 9, the following compounds having physical data in Table 2 were prepared.

Table 2

| Reference Example No. | Reference Example No. of Starting Material | Structure of Product | Physical Data: TLC (developing solvent) | Physical Data: MS |
|---|---|---|---|---|
| (a) | 1 | 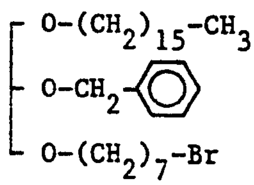 | Rf=0.58 (AcOEt:n-hexane =1:5) | 584($M^+$) 582($M^+$) 493, 491 |
| (b) | 1 | 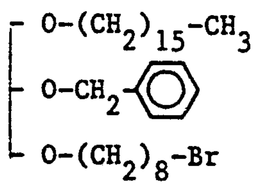 | Rf=0.60 (AcOEt:n-hexane =1:5) | 598($M^+$) 596($M^+$) |
| (c) | 1 | 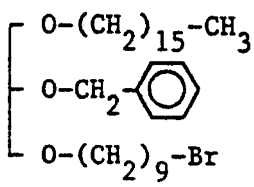 | Rf=0.61 (AcOEt:n-hexane =1:5) | 612($M^+$) 610($M^+$) |
| (d) | 1 | 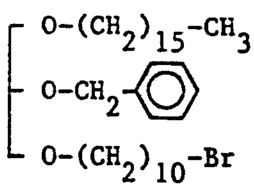 | Rf=0.65 (AcOEt:n-hexane =1:5) | 626($M^+$) 624($M^+$) |

Reference Example 10

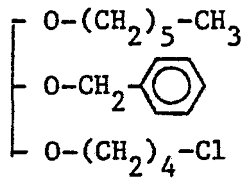

Under an atmosphere of argon, 55 mg of sodium hydride (content: 64.1%), 0.29 ml of n-hexyl bromide and 6 ml of dry DMF were added to 200 mg of the 1-hydroxy compound (prepared in Reference Example 7) at room temperature. The mixture was stirred for 2.5 hours at 65°C. To the reaction mixture were added one ml of water and the mixture was concentrated. The residue was purified by column chromatography on silica gel (a mixture of $CH_2Cl_2$ and n-hexane) to give 172 mg of the title compound having the following physical data:

TLC ($CH_2Cl_2$): Rf=0.33;

NMR: δ=7.1 (5H, s), 4.6 (2H, s), 4.0-3.1 (11H, m), 2.1-0.7 (15H, m);

IR: ν=2920, 2850, 1450, 1110 $cm^{-1}$;

Mass: m/e=356 ($M^+$), 265.

By the same procedure as described in Reference Example 10, the compounds having the following physical data were prepared.

(a) 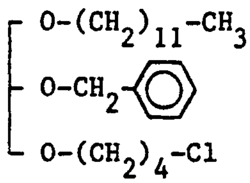

Starting Material: the 1-hydroxy compound prepared in Reference Example 7;

TLC ($CH_2Cl_2$): Rf=0.56;

NMR: δ=7.2 (5H, s), 4.6 (2H, s), 3.8-3.1 (11H, m), 2.0-0.8 (27H, m);

IR: ν=2920, 2850, 1450, 1110 $cm^{-1}$;

Mass: m/e=440 ($M^+$), 404, 349.

(b) 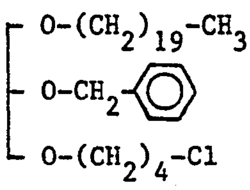

Starting Material: the 1-hydroxy compound prepared in Reference Example 7;

TLC (AcOEt:n-hexane=1:5): Rf=0.70;

NMR: δ=7.21 (5H, s), 4.63 (2H, s), 3.8-3.1 (11H, m), 1.95-0.7 (43H, m);

IR: ν=2920, 2840, 1110 $cm^{-1}$;

Mass: m/e=516 ($M^+$), 425, 318.

Reference Example 11

$O-(CH_2)_{17}-CH_3$
$O-CO-CH_3$
$O-CO-(CH_2)_2-NH-cbz$

Under an atmosphere of nitrogen, a mixture of 280 mg of the 3-hydroxy compound (prepared in Reference Example 6), 194 mg of (3-benzyloxycarbonylamine)propionic acid, 224 mg of dicyclohexylcarbodiimide, 7 mg of 4-dimethylaminopyridine and 10 ml of dry THF was stirred for 3 hours at room temperature, and the reaction mixture was concentrated. The precipitating crystals were filtered off and the filtrate was concentrated. The residue was purified by column chromatography on silica gel (a mixture of AcOEt and cyclohexane) to give 400 mg of the title compound having the following physical data:

TLC ($CHCl_3$:acetone=10:1): Rf=0.78;

NMR: δ=7.6 (5H, s), 5.6-4.8 (4H, m), 4.5-3.9 (2H, m), 3.8-3.0 (6H, m), 2.8-2.3 (2H, m), 2.0 (3H, s), 1.9-0.7 (35H, m);

IR: ν=3340, 2900, 2850, 1740, 1690, 1540 $cm^{-1}$;

Mass: m/e=591 ($M^+$), 547, 531, 484, 456, 442, 424, 369.

By the same procedure as described in Reference Example 11, the compound having the following physical data in Table 3 were prepared.

**Table 3**

| Reference Example No. | Reference Example No. of Starting Material | Structure of Product | Physical Data | | | |
|---|---|---|---|---|---|---|
| | | | TLC (developing solvent) | NMR | IR | MS |
| (a) | 1 | 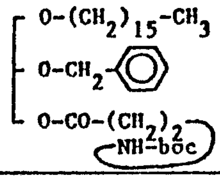 | Rf=0.61 (AcOEt: cyclohexane: =1:2) | 7.2(5H,s), 5.2-4.6(1H,m), 4.6(2H,s), 4.16(2H,t), 4.0-3.0(7H,m), 2.46(2H,t), 1.8-0.6(31H,m), 1.4(9H,s) | 3500-3100, 2920, 2850, 1730, 1710, 1500 | 520, 504, 477, 414 |
| (b) | 1 | 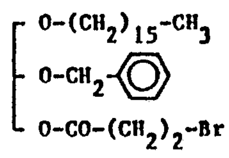 | Rf=0.54 (AcOEt: cyclohexane =1:5) | 7.2(5H,s), 4.6(2H,s), 4.4-4.1(2H,m), 4.0-3.2 (7H,m), 2.9(2H,t), 1.9-0.7(31H,m) | 2910, 2840, 1730, | 542($M^+$), 540($M^+$), 450, 435, 433, 405 |
| (c) | 1 | 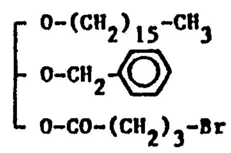 | Rf=0.49 (AcOEt: cyclohexane =1:5) | 7.2(5H,s), 4.6 (2H,s), 4.4-4.0(2H,m), 4.0-3.1(7H,m), 2.7-1.9 (4H,m), 1.9-0.6(31H,m) | 2920, 2840, 1735, 1450, 1110 | 555($M^+$) 554($M^+$) 447, 403 |

Reference Example 12

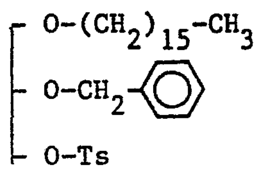

To a solution of 2.49 g of the 3-hydroxy compound (prepared in Reference Example 1) in 30 ml of $CH_2Cl_2$, was added 3 ml of pyridine and further 1.93 g of p-toluenesulfonyl chloride was added thereto with cooling in an ice. After the mixture was stirred for 2 days at room temperature, the reaction mixture was concentrated to distill off the solvent. To the residue obtained, was added water and the mixture was extracted with AcOEt. The extract was washed, dried and concentrated. The residue was purified by column chromatography on silica gel (a mixture of AcOEt and cyclohexane) to give 2.73 g of the title compound having the following physical data:

TLC (AcOEt:cyclohexane=1:8): Rf=0.34;

NMR: $\delta$=7.81 (2H, d), 7.50-7.20 (7H, m), 4.60 (2H, s), 4.30-3.20 (7H, m), 2.47 (3H, s), 2.70-2.10 (28H, m), 0.96 (3H, t);

IR: $\nu$=2930, 2860, 1600, 1455, 1370, 1190, 1180, 1120, 1100, 990 $cm^{-1}$.

Reference Example 13

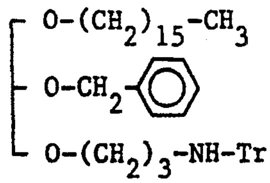

Under an atmosphere of nitrogen, 40 mg of sodium hydride and 164 mg of 3-(N-tritylamino)propyl alcohol were added to 3 ml of DMF and the suspension was stirred for 30 minutes at 40°C and further stirred for 15 minutes at 60°C. Then a solution of 200 mg of p-toluenesulfonylglycerol compound (prepared in Reference Example 12) in 3 ml of DMF was added to the mixture at 40°C and the mixture was stirred for 5 hours at the same temperature. The reaction mixture was extracted with AcOEt and the extract was washed, dried and concentrated. The residue was purified by column chromatography on silica gel (a mixture of AcOEt and cyclohexane) to give 158 mg of the title compound having the following physical data:

TLC (AcOEt:cyclohexane=1:5): Rf=0.63;

NMR: $\delta$=7.6-7.0 (20H, m), 4.6 (2H, s), 3.8-3.2 (9H, m), 2.5-2.1 (2H, m), 2.0-0.7 (34H, m);

IR: $\nu$=2920, 2850, 1590, 1440 $cm^{-1}$

Reference Example 14

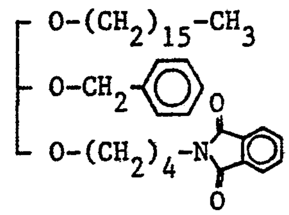

A solution of 131 mg of the chlorobutylglycerol compound (prepared in Reference Example 8) and 64 mg of potassium phthalimide in 0.5 ml of DMF was stirred for 5 hours on a water bath at 100°C. To the reaction mixture was added a small proportion of water and the mixture was extracted with AcOEt. The extract was concentrated. The residue was purified by column chromatography on silica gel (a mixture of AcOEt and cyclohexane) to give 128 mg of the title compound having the following physical data:

TLC (AcOEt:cyclohexane=1:5): Rf=0.31;

NMR: δ=7.88-7.38 (4H, m), 7.15 (5H, s), 4.58 (2H, s), 4.01-3.08 (11H, m), 2.08-0.58 (35H, m);

IR: ν=2925, 2850, 1775, 1715, 1465, 1395, 1365, 1115, 1050, 720 $cm^{-1}$.

Example 1

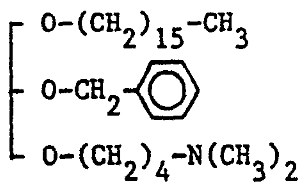

A mixture of 1.911 g of the chlorobutylglycerol compound (prepared in Reference Example 8), 10 ml of a 40% aqueous solution of dimethylamine and 35 ml of ethanol was stirred overnight on a water bath at 75°C. The reaction mixture was concentrated and to the residue thus obtained was added an aqueous solution of sodium hydroxide and then the mixture was extracted with AcOEt. The extract was washed, dried and concentrated. The residue was purified by column chromatography on silica gel (a mixture of $CHCl_3$, MeOH and trimethylamine) to give 1.7 g of the title compound having the following physical data:

TLC (AcOEt:AcOH:water=3:1:1): Rf=0.67;

NMR: $\delta$=7.22 (5H, s), 4.63 (2H, s), 3.87-3.20 (9H, m), 2.50-2.0 (2H, m and 6H, s), 1.83-0.67 (35H, m);

IR: $\nu$=3070, 3045, 2940, 2860, 1470, 1455, 1380, 1120, 740, 700 $cm^{-1}$.

By the same procedure as described in Example 1, the following compound having physical data in Table 4 were prepared.

Table 4(1)

| Example No. | Reference Example No. of Starting Material | Structure of Product | Physical Data | | | |
|---|---|---|---|---|---|---|
| | | | TLC (developing solvent) | NMR | IR | MS |
| (a) | 8 | 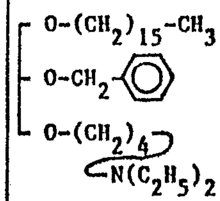 | Rf=0.66 (AcOEt:AcOH: water=3:1:1) | 7.42-7.22(5H,m), 4.68 (2H,s), 3.78-3.64(1H,m), 3.60-3.36(8H,m), 2.52 (4H,q), 2.55-2.38(2H,m), 1.80-1.40(6H,m), 1.26 (26H, m), 1.01(6H,t), 0.87(3H,m) | 2930, 2850, 1460, 1110 | 533($M^+$), 519, 504, 442, 426 |
| (b) | 8 | 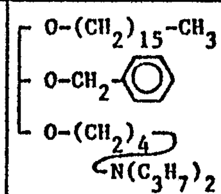 | Rf=0.82 (AcOEt:AcOH: water=3:1:1) | 7.41-7.22(5H,m), 4.68 (2H,s), 3.78-3.64(1H,m), 3.59-3.35(8H,m), 2.48-2.28(6H,m), 1.78-1.15 (36H,m), 0.85(6H,t), 0.87(3H,m) | 2940, 2860, 1460, 1120 | 561($M^+$), 532, 470, 454 |
| (c) | 8 | 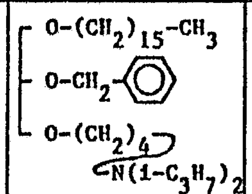 | Rf=0.74 (AcOEt:AcOH: water=3:1:1) | | | 561($M^+$), 546, 504, 454 |
| (d) | 8 | 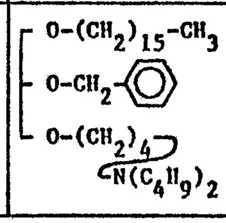 | Rf=0.87 (AcOEt:AcOH: water=3:1:1) | 7.43-7.22(5H,m), 4.69(2H, s), 3.78-3.65(1H,m), 3.62-3.37(8H,m), 2.49-2.33(6H,m), 1.78-1.20 (40H,m), 0.90(6H,t), 0.88 (3H,m) | 2930, 2860, 1460, 1110 | 589($M^+$), 564, 532, 482 |

Table 4(2)

| Example No. | Reference Example No. of Starting Material | Structure of Product | Physical Data | | | |
|---|---|---|---|---|---|---|
| | | | TLC (developing solvent) | NMR | IR | MS |
| (e) | 8 | 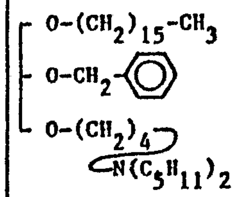 | Rf=0.34 (AcOEt:AcOH: MeOH=20:1:1) | 7.44-7.25(5H,m), 4.69 (2H,s),3.79-3.64(1H,m), 3.60-3.27(8H,m), 2.50-2.30(6H,m),1.78-1.16 (36H, m), 1.04-0.82 (9H,m). | 3300, 2930, 2850, 1160, 1460, 1110 | 617($M^+$), 560, 546, 510 |
| (f) | 8 | 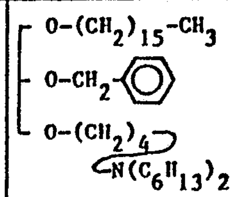 | Rf=0.48 (AcOEt:AcOH: MeOH=20:1:1) | 7.42-7.25(5H,m), 4.69 (2H,s), 3.78-3.63(1H,m), 3.62-3.36(8H,m), 2.50-2.30(6H,m) | | 645($M^+$), 574, 538 |
| (g) | 8 | 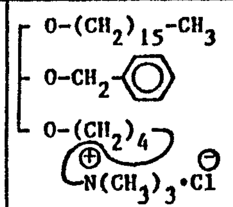 | Rf=0.41 (AcOEt:AcOH: water=3:1:1) | 7.44-7.28(5H,m), 4.68(2H, dd), 3.80-3.65(1H,m), 3.65-3.38(10H,m), 3.32 (9H,s), 1.93-1.46(6H,m), 1.45-1.13(26H,m), 0.88 (3H,t) | 2925, 2850, 1480, 1460, 1450, 1115, 970, 910, 750 | |
| (h) | 8(a) | 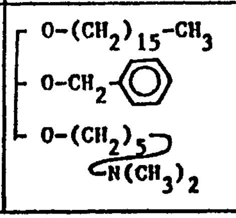 | Rf=0.66 (AcOEt:AcOH: water=3:1:1) | 7.22(5H,s), 4.63(2H, s), 3.87-3.20(9H,m), 2.50-2.0(2H,m and 6H,s), 1.85-0.67(37H,m) | | |

Table 4(3)

| Example No. | Reference Example No. of Starting Material | Structure of Product | Physical Data | | | |
|---|---|---|---|---|---|---|
| | | | TLC (developing solvent) | NMR | IR | MS |
| (i) | 8(a) | 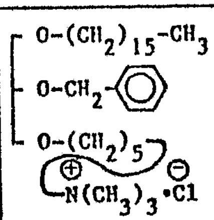 | Rf=0.58 (AcOEt:AcOH: water=3:1:1)<br>Melting point: 33-36°C | 7.42-7.24(5H,m), 4.67 (2H,s), 3.76-3.62(1H,m), 3.62-3.22(19H,m), 1.86-1.40(8H,m), 1.40-1.10 (26H, m), 0.88(3H,t), | 2925, 2850, 1630, 1485, 1470, 1390 1125, 970, 950, 750 (KBr method) | |
| (j) | 9 | 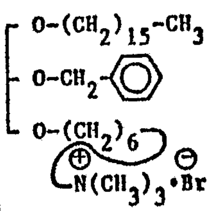 | Rf=0.58 (AcOEt:AcOH: water=3:1:1)<br>Melting point: 68-74°C | 7.42-7.25(5H,m), 4.68 (2H,s), 3.80-3.64(1H,m), 3.64-3.35(10H,m), 3.40 (9H,s), 1.85-1.38 (10H,m), 1.26(26H,m), 0.88(3H,m) | 3450, 2920, 2850, 1460,1120 (KBr method) | 533, 422, 426 |
| (k) | 9(a) | 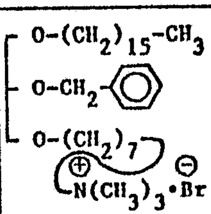 | Rf=0.37 (AcOEt:AcOH: water=3:1:1)<br>Melting point: 59-63°C | 7.42-7.26(5H,m), 4.68 (2H,s), 3.78-3.65(1H,m), 3.60-3.35(19H,m), 1.82-1.65(2H,m), 1.65-1.46 (4H,m), 1.46-1.33 (6H,m) 1.25(26H,m), 0.87(3H,m) | 3450, 2920, 2850 1460, 1120<br>(KBr method) | 547, 456, 440 |
| (l) | 9(b) | 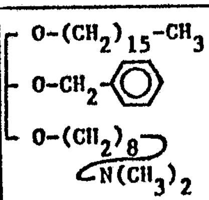 | Rf=0.40 (AcOEt:AcOH: water=3:1:1) | 7.4-7.25(5H,m), 4.69(2H, s), 3.8-3.4(9H,m), 2.5-2.1(2H,m and 6H,s), 2.0-0.7(43H,m) | 2920, 2840, 1450, 1110 | 561($M^+$), 470, 454 |

**Table 4(4)**

| Example No. | Reference Example No. of Starting Material | Structure of Product | Physical Data | | | |
|---|---|---|---|---|---|---|
| | | | TLC (developing solvent) | NMR | IR | MS |
| (m) | 9(b) | 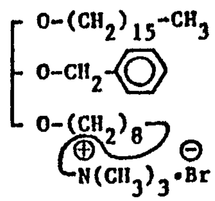 | Rf=0.37 (AcOEt:AcOH: water=3:1:1)<br><br>Melting point: 135-138°C | 7.40-7.26(5H,m), 4.69 (2H,s), 3.78-3.65(1H,m), 3.60-3.36(19H,m), 1.83-1.68(2H,m), 1.64-1.46, (4H,m), 1.46-1.20 (34H, m), 0.08(3H,m) | 3400, 2920, 2850, 1460, 1120<br><br>(KBr method) | 561, 470, 454 |
| (n) | 9(c) | 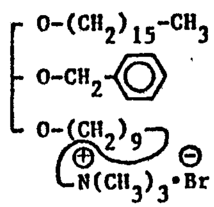 | Rf=0.35 (AcOEt:AcOH: water=3:1:1)<br><br>Melting point: 125-130°C | 7.44-7.26(5H,m), 4.70 (2H,s), 3.79-3.65(1H,m), 3.64-3.36(19H,m), 1.84-1.67(2H,m), 1.65-1.48 (4H,m), 1.27(36H,m), 0.88(3H,m) | 3450, 2940, 2860, 1470, 1120<br><br>(KBr method) | 575, 484, 468 |
| (o) | 9(d) | 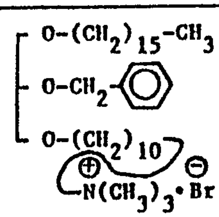 | Rf=0.38 (AcOEt:AcOH: water=3:1:1)<br><br>Melting point: 41-44°C | 7.41-7.26(5H,m), 4.69 (2H,s), 3.78-3.64(2H,m) 3.62-3.34(19H,m), 1.84-1.65(2H,m), 1.64-1.45 (4H,m), 1.25(38H,m), 0.87(3H,m) | 3400, 2930, 2860, 1470, 1110 | 589, 498, 482 |
| (p) | 8(b) | 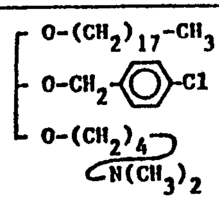 | Rf=0.59 (AcOEt:AcOH: water=3:1:1) | 7.40-7.20(4H,m), 4.69(2H, s), 3.80-3.64(2H,m), 3.58-3.36(7H,m), 2.33-2.16(2H,m), 2.20(6H,s), 1.68-1.45(6H,m), 1.26 (30H,m), 0.88(3H,m) | 2920, 2850, 1450, 1110 | 553($M^+$), 538, 428, 412 |

Table 4(5)

| Example No. | Reference Example No. of Starting Material | Structure of Product | Physical Data | | | |
|---|---|---|---|---|---|---|
| | | | TLC (developing solvent) | NMR | IR | MS |
| (q) | 8(c) | 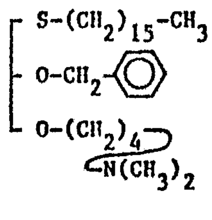 | Rf=0.44 (AcOEt:AcOH: water=3:1:1) | 7.4-7.23(5H,m), 4.66 (2H,s), 3.74-3.62(1H,m), 3.62-3.53(2H,m), 3.52-3.40(2H,m), 2.75-2.67 (2H,m), 2.51(2H,t), 2.35-2.2((2H, m), 2.21 (6H,s), 1.66-1.45(6H,m) 1.25(26H,m), 0.88(3H,m) | 2930, 2860, 1460, 1120 | 521($M^+$), 414, 386 |
| (r) | 10 | 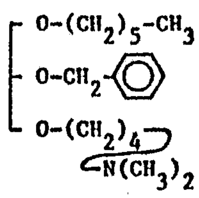 | Rf=0.61 (AcOEt:AcOH: water=3:1:1) | 7.2(5H,s), 4.6(2H,s) 3.8-3.1(9H,m), 2.6-2.0 (2H,m and 6H,s), 2.0-0.7(15H,m) | 2920, 2850, 1450, 1110 | 365($M^+$), 350, 280, 258, 250 |
| (s) | 10(a) | 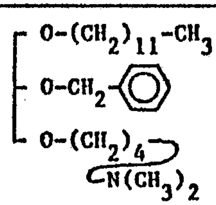 | Rf=0.60 (AcOEt:AcOH: water=3:1:1) | 7.2(5H,s), 4.66(2H,s), 3.8-3.1(9H,m), 2.4-2.0 (2H,m and 6H,s), 1.8-0.7(27H,m) | 2920, 2850, 1460, 1110 | 449($M^+$), 434, 420, 406, 392, 378, 358, 342 |
| (t) | 10(b) | 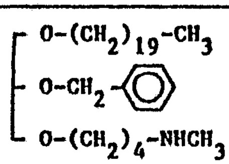 | Rf=0.60 (AcOEt:AcOH: water=3:1:1) | 7.41-7.20(5H,m), 4.68(2H, s), 3.78-3.64(1H,m), 3.58-3.36(8H,m), 2.44-2.26(3H,m and 3H,s), 1.68-1.44(6H,m), 1.26 (34H,m), 0.88(3H,m) | 2920, 2850, 1460, 1110 | 511($M^+$), 420, 404 |

Table 4(6)

| Example No. | Reference Example No. of Starting Material | Structure of Product | Physical Data: TLC (developing solvent) | Physical Data: NMR | Physical Data: IR | Physical Data: MS |
|---|---|---|---|---|---|---|
| (u) | 11(b) | 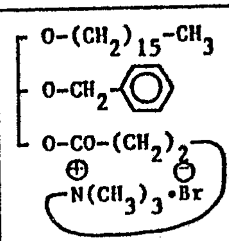 | Rf=0.17 ($CHCl_3$:MeOH: water =50:10:1) | 7.36(5H,s), 4.78-4.55 (2H,q), 4.50-4.15(2H,m), 4.00-3.70(1H,m), 3.64-3.30(7H,m), 3.36(9H,s), 3.00-2.80(2H, m), 1.94-1.70(2H,m), 1.68-1.45 (2H,m), 1.42-1.10(26H, m), 0.88(3H,t) | 2900, 2840, 1730 | 507, 460, 405, 398, 353 |
| (v) | 11(c) | 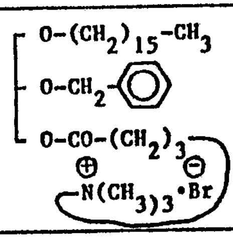 | Rf=0.61 ($CHCl_3$:MeOH: water =65:35:6) | 7.35(5H,s), 4.75-4.58 (2H,q), 4.48-4.09(2H,m), 3.86-3.25(7H,m), 3.37 (9H,s), 2.51(2H,t), 2.2-1.95(2H,m), 1.67-1.45(2H,m), 1.45-1.15 (26H,m), 1.0-0.8(3H,m) | 3600-3100, 2930, 2850, 1740 (KBr method) | 519, 504, 428, 412 |

Example 2

$O-(CH_2)_{17}-CH_3$

$O-CO-CH_3$

$O-CO-(CH_2)_2-NH_2 \cdot CH_3COOH$

Under an atmosphere of hydrogen, a mixture of 336 mg of the cbz compound (prepared in Reference Example 11), 200 mg of palladium on carbon (content: 10%), 0.8 ml of a concentrated hydrochloric acid and 20 ml of AcOEt was stirred for 30 minutes at room temperature. Catalyst was filtered off and the filtrate was concentrated. The residue was purified by column chromatography on silica gel (a mixture of $CHCl_3$, MeOH and AcOH) to give 119 mg of the title compound having the following physical data:

TLC ($CHCl_3$:MeOH:AcOH=10:1:1): Rf=0.46;

[The Rf value shows that of the corresponding hydrochloric acid salt before purification.]

NMR: δ=5.32-5.06 (1H, m), 4.5-4.0 (2H, m), 3.68-3.25 (6H, m), 2.8-2.49 (2H, m), 2.2-1.85 (6H, m), 1.65-1.08 (32H, m), 1.08-0.7 (3H, m);

IR ($CHCl_3$ solution): ν=2920, 2850, 1730 $cm^{-1}$;

Mass: m/e=457, 428, 397, 386, 355, 325, 308, 283, 281, 253.

Example 3

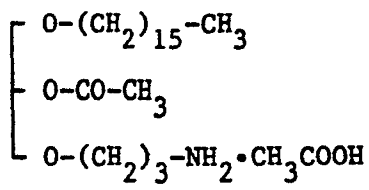

A mixture of 128 mg of the tritylaminoglycerol compound (prepared in Reference Example 13) and 2 ml of trifluoroacetic acid was stirred for 3 hours at room temperature. The reaction mixture was concentrated and the residue was purified twice by column chromatography on silica gel (a mixture of $CHCl_3$, MeOH and AcOH for both the first and the second purification) to give 37 mg of the title compound having the following physical data:

TLC ($CHCl_3$:MeOH:AcOH=10:1:1): Rf=0.57;

IR: ν=2920, 2840, 1735, 1680, 1550, 1480, 1240 $cm^{-1}$.

Example 4

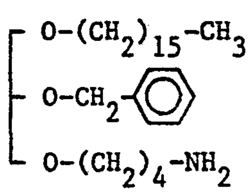

A solution of 128 mg of the phthalimidoglycerol compound (prepared in Reference Example 14) and 40 mg of hydrazine hydrate in one ml of ethanol was stirred for 20 minutes on a water bath at 70°C. After cooling, diethyl ether was added to the reaction mixture and then the mixture was filtered. The filtrate was concentrated and the residue was purified by column chromatography on silica gel (a mixture of $CHCl_3$, MeOH and triethylamine) to give 89 mg of the title compound having the following physical data:

TLC ($CHCl_3$:MeOH:triethylamine=25:5:0.1): Rf:0.24;

NMR: δ=7.50-7.26 (5H, m), 4.71 (2H, s), 3.72 (1H, m), 3.65-3.33 (8H, m), 2.73 (2H, t), 2.32 (2H, broad s), 1.70-1.44 (6H, m), 1.44-1.18 (26H, m), 0.88 (3H, t);

IR: ν=2920, 2850, 1455, 1110, 730, 690 $cm^{-1}$.

Example 5

$O-(CH_2)_{15}-CH_3$
OH
$O-(CH_2)_4-NH_2$ and its acetate

To a mixture of 5 ml of ethanol and 0.1 ml of AcOH were added 70 mg of the benzylglycerol compound (prepared in Example 4) and a proper amount of palladium on carbon, and the

mixture was stirred for 3 hours at room temperature with blowing hydrogen gas. After the completion of the reaction, the catalyst was filtered off and the filtrate was concentrated. The residue was purified by column chromatography on silica gel (a mixture of $CHCl_3$, MeOH and triethylamine) to give 45 mg of the title compound (as free amine compound). A part of the compound was converted into its acetate by using acetic acid to give the title compound (as acetate of amine) having the following physical data:

TLC (AcOEt:AcOH:water=3:1:1): Rf=0.42;

NMR: δ=4.10-3.90 (1H, m), 3.68-3.33 (8H, m), 3.18-3.86 (2H, m), 2.02 (3H, s), 1.95-1.66 (4H, m), 1.64-1.46 (2H, m), 1.42-1.16 (26H, m), 0.88 (3H, t);

IR (KBr method): ν=3420, 2925, 2855, 2800-2250, 1570, 1470, 1405, 1120 $cm^{-1}$.

By the same procedure as described in Example 5, the following compounds having physical data in Table 5 were prepared.

Table 5

| Example No. | Example No. of Starting Material | Structure of Product | Physical Data: TLC (developing solvent) | Physical Data: NMR | Physical Data: IR | Physical Data: MS |
|---|---|---|---|---|---|---|
| (a) | 1 | 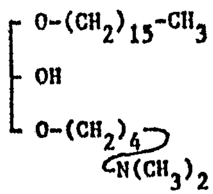 | Rf=0.61 (AcOEt:AcOH: water=3:1:1) | 4.08-3.63(1H,m), 3.63-3.15(8H,m), 2.93(1H, s), 2.45-2.08(8H,m), 2.18 (6H,s), 1.92-0.67(35H,m) | 3420, 2925, 2855, 1465, 1375, 1265, 1115, 755 | |
| (b) | 1(l) | 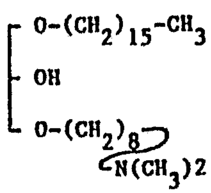 | Rf=0.36 (AcOEt:AcOH: water=3:1:1) | 4.1-3.7(1H,m), 3.65-3.2 (8H,m), 2.7(1H,s), 2.5-2.0(2H,m and 6H,s), 1.9-0.7(43H,m) | 3450, 2920, 2840, 1450, 1110 | 471($M^+$) |
| (c) | 1(q) | 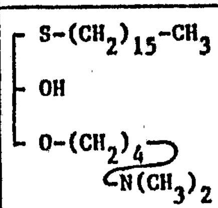 | Rf=0.35 ($CH_2Cl_2$:MeOH =10:1) | 3.90-3.65(1H,m), 3.55-3.14(4H,m), 2.75-2.55 (2H,m), 2.24(6H,s), 0.87(3H,broad t) | 3450, 1125 | 431($M^+$), 416 |
| (d) | 1(g) | 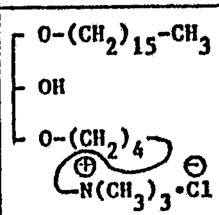 | Rf=0.37 (AcOEt:AcOH: water=3:1:1)<br>Melting point: 83-85°C | 4.05-3.90(1H,m), 3.82-3.35(20H,m), 2.03-1.45 (6H,m), 1.40-1.14(26H, m), 0.88(3H,t) | 3430, 2920, 2850, 1470, 1120, 975<br>(KBr method) | |

Example 6

$O-(CH_2)_{15}-CH_3$
$O-CO-CH_3$
$O-(CH_2)_4-NHCO-CH_3$

A mixture of 23 mg of the hydroxy compound (prepared in Example 5, the free amine compound), 0.15 ml of acetic anhydride and 1.0 ml of pyridine was stirred at 40°C. After the completion of the reaction, pyridine was distilled off from the reaction solution. The residue thus obtained was purified by column chromatography on silica gel (a mixture of AcOEt and cyclohexane, and acOEt, successively) to give 12 mg of the title compound having the following physical data:

Melting point: 38-40°C;

TLC ($CHCl_3$:MeOH=10:1): Rf=0.51;

NMR: δ=5.24-5.07 (1H, m), 3.66-3.52 (4H, m), 3.52-3.37 (4H, m), 3.37-3.16 (2H, m), 2.08 (3H, s), 1.98 (3H, s), 1.70-1.44 (6H, m), 1.44-1.10 (26H, m), 0.88 (3H, t);

IR (KBr method): ν=3320, 2920, 2850, 1740, 1640, 1545, 1465, 1370, 1240, 1125 $cm^{-1}$.

By the same procedure as described in Example 6, the following compounds having physical data in Table 6 were prepared.

Table 6(1)

| Example No. | Example No. of Starting Material | Structure of Product | Physical Data: TLC (developing solvent) | Physical Data: NMR | Physical Data: IR | Physical Data: MS |
|---|---|---|---|---|---|---|
| (a) | 5(a) | 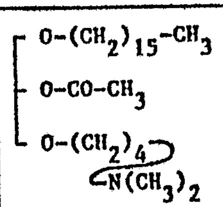 | Rf=0.70 (AcOEt:AcOH: water=3:1:1) | 5.18-5.02(1H,m), 3.62-3.34(8H,m), 2.2(6H,s) 2.34-2.16((2H, m), 2.07 (3H,s), 1.66-1.44(6H,m), 1.25(26H,m), 0.87(3H,m) | 2920, 2850, 1740, 1460, 1370, 1240, 1110 | 457($M^+$), 442, 427, 397 |
| (b) | 5(a) | 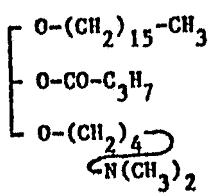 | Rf=0.58 (AcOEt:AcOH: water=3:1:1) | | 1730 | 485($M^+$), 398 |
| (c) | 5(a) | 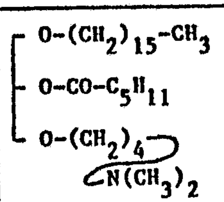 | Rf=0.62 (AcOEt:AcOH: water=3:1:1) | | | |
| (d) | 5(a) | 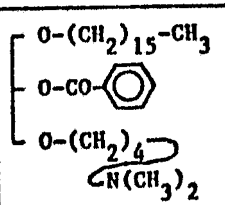 | Rf=0.45 (AcOEt:AcOH: water=3:1:1) | 5.55(1H,m), 2.62(6H, s) | 1715 | |

**Table 6(2)**

| Example No. | Example No. of Starting Material | Structure of Product | Physical Data | | | |
|---|---|---|---|---|---|---|
| | | | TLC (developing solvent) | NMR | IR | MS |
| (e) | 5(d) | 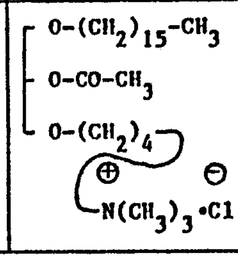 | Rf=0.42 (AcOEt:AcOH: water=3:1:1) | 5.24-5.10(1H,m), 3.74-3.36(10H,m), 3.45(9H,s), 2.09(3H,s), 2.00-1.46 (6H,m), 1.42-1.16 (26H,m), 0.88(3H,t) | 3450, 2920, 2850, 1740, 1465, 1380, 1245, 1120 (KBr method) | |

Example 7

$$\left[\begin{array}{l} O-(CH_2)_{15}-CH_3 \\ O-CONH-CH_3 \\ O-(CH_2)_4-N(CH_3)_2 \end{array}\right.$$

A mixture of 106.5 mg of the hydroxy compound [prepared in Example 5(a)], 0.3 ml of methyl isocyanate, 0.1 ml of pyridine and 5 ml of $CHCl_3$ was stirred for 2 days at 50°C. The reaction mixture was concentrated and the residue was purified by column chromatography on silica gel (a mixture of $CHCl_3$, MeOH and triethylamine) to give 270 mg of the title compound having the following physical data:

TLC (AcOEt:AcOH:water=3:1:1): Rf=0.67.

By the same procedure as described in Example 7, the compounds having the following physical data in Table 7 were prepared.

Table 7

| Example No. | Example No. of Starting Material | Structure of Product | Physical Data | | | |
|---|---|---|---|---|---|---|
| | | | TLC (developing solvent) | NMR | IR | MS |
| (a) | 5(a) | 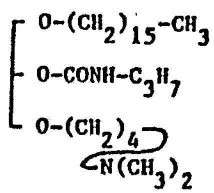 | Rf=0.73 (AcOEt:AcOH: water=3:1:1) | | | 500($M^+$), 485, 470, 398, 259 |
| (b) | 5(a) | 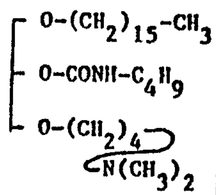 | Rf=0.60 (AcOEt:AcOH: water=3:1:1) Melting point: 37-38°C | 5.04-4.78(2H,m), 3.64-3.36(8H,m), 3.24-3.10 (2H,m), 2.22(6H,s), 3.36-2.20(2H,m), 1.68-1.20(36H,m), 1.92(3H,t), 0.88 (3H,m) | 3340, 2920, 2850, 1710, 1460, 1240, 1110 | 514($M^+$), 499, 485, 471, 398 |
| (c) | 5(a) | 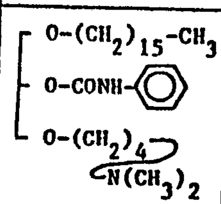 | Rf=0.65 (AcOEt:AcOH: water=3:1:1) | | | |

Reference Example 15

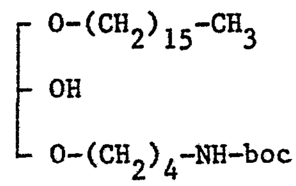

To a mixture of 300 mg of the amino compound (prepared in Example 5), 0.8 ml of triethylamine and 20 ml of $CH_2Cl_2$, was added a solution of 179 mg of di-tert-butyl dicarbonate in 5 ml of $CH_2Cl_2$ with cooling in an ice-bath and the mixture obtained was stirred for 2 hours at room temperature. The reaction mixture was concentrated and the residue was purified by column chromatography on silica gel (a mixture of AcOEt and n-hexane) to give 220 mg of the title compound having the following physical data:

TLC (AcOEt:n-hexane=1:5): Rf=0.60;

NMR: $\delta$=3.95-3.65 (1H, m), 3.60-3.15 (8H, m), 2.80-2.60 (2H, m), 2.25 (9H, s), 0.90 (3H, broad t);

IR: $\nu$=3450, 3300, 1690, 1530 $cm^{-1}$;

Mass: m/e=487 ($M^+$), 430, 414, 386.

Reference Example 16

$$\left[\begin{array}{l} O-(CH_2)_{15}-CH_3 \\ O-Ms \\ O-(CH_2)_4-N(CH_3)_2 \end{array}\right.$$

To a mixture of 3.4 g of the hydroxy compound [prepared in Example 5(a)], 4 ml of triethylamine, and 50 ml of $CH_2Cl_2$, was added 0.76 ml of mesyl chloride at 0°C, and the solution thus obtained was stirred for one hour at 0°C. To the reaction mixture, was added 300 ml of diethyl ether and the mixture thus obtained was washed, dried and concentrated to give 3.86 g of the title compound having the following physical data:

TLC (AcOEt:AcOH:water=3:1:1): Rf=0.61;

NMR: δ=4.93-4.53 (1H, m), 3.80-3.16 (8H, m), 3.0 (3H, s), 2.16 (6H, s), 2.46-2.0 (2H, m), 1.83-0.66 (35H, m);

IR: ν=2920, 2840, 1450, 1350, 1170, 1110 $cm^{-1}$.

By the same procedure as described in Reference Example 16, the following compounds having physical data in Table 8 were prepared.

Table 8

| Reference Example No. | (Reference) Example No. of Starting Material | Structure of Product | Physical Data: TLC (developing solvent) | NMR | IR | MS |
|---|---|---|---|---|---|---|
| (a) | Example 5(b) | $O-(CH_2)_{15}-CH_3$<br>$O-Ms$<br>$O-(CH_2)_8-N(CH_3)_2$ | Rf=0.39 (AcOEt:AcOH: water=3:1:1) | 4.9-4.5(1H,m), 3.7-3.1(8H,m), 3.0(3H,s), 2.4-2.0(2H,m and 6H,s), 2.0-0.7(43H,m) | 2920, 2850, 1460, 1360, 1170, 1120 | 549($M^+$), 470 453 |
| (b) | Example 5(c) | $S-(CH_2)_{15}-CH_3$<br>$O-Ms$<br>$O-(CH_2)_4-N(CH_3)_2$ | Rf=0.40 ($CH_2Cl_2$:MeOH =10:1) | 4.70-4.30(1H,m), 3.60-3.15(4H,m), 2.76-2.61 2H,m), 2.24(6H,s), 0.90(3H, broad t) | 1370, 1190 | 509($M^+$), 430 |
| (c) | Reference Example 15 | $O-(CH_2)_{15}-CH_3$<br>$O-Ms$<br>$O-(CH_2)_4-NH-boc$ | Rf=0.70 (AcOEt: n-hexane=1:5) | 4.75-4.25(1H,m), 3.55-3.14(8H,m), 2.75-2.60 (2H,m), 2.30(9H,s), 0.91(3H, broad t) | 3300, 1690, 1525 | 565($M^+$), 486 |

Example 8

$$\begin{cases} O-(CH_2)_{15}-CH_3 \\ S-C_2H_5 \\ O-(CH_2)_4-N(CH_3)_2 \end{cases}$$

Under an atmosphere of argon, to a suspension of 37 mg of sodium hydride (content: 64.1%) in 3 ml of dry THF, was added 74 µl of ethanethiol and the mixture was stirred for 10 minutes at room temperature. To the mixture was added a solution of 100 mg of the mesyl compound (prepared in Reference Example 16) in 7 ml of dry THF and one ml of HMPA and the solution was refluxed overnight. To the reaction mixture was added 50 ml of AcOEt. The mixture was washed, dried and concentrated. The residue was purified by column chromatography on silica gel ($CHCl_3$, and a mixture of $CHCl_3$, MeOH and triethylamine, successively) to give 75 mg of the tile compound having the following physical data:

TLC (AcOEt:AcOH:water=3:1:1): Rf=0.59;

NMR: δ=3.8-3.1 (8H, m), 3.0-2.8 (1H, m), 2.7-2.5 (2H, m), 2.4-2.0 (2H, m and 6H, s), 1.8-0.8 (38H, m);

Mass: m/e=459 ($M^+$), 430, 398.

By the same procedure as described in Example 8, the compounds having the following physical data in Table 9 were prepared.

**Table 9**

| Example No. | Reference Example No. of Starting Material | Structure of Product | Physical Data: TLC (developing solvent) | Physical Data: NMR | Physical Data: IR | Physical Data: MS |
|---|---|---|---|---|---|---|
| (a) | 16(a) | 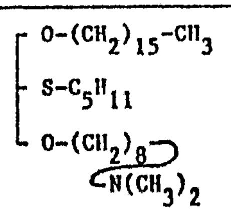 | Rf=0.41 (AcOEt:AcOH: water=3:1:1) | 3.75-3.4(8H,m), 3.04-2.95(1H,m), 2.58-2.46 (2H,t), 2.35-2.18(2H, m and 6H, s), 1.65-1.45(8H,m), 1.26 (38H, m), 0.88(6H,m) | 2930, 2850, 1460, 1120 | 557($M^+$) |
| (b) | 16 | 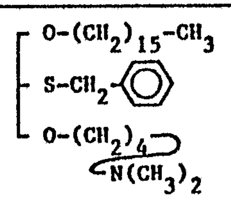 | Rf=0.64 (AcOEt:AcOH: water=3:1:1) | 7.17(5H,broad s), 3.77 (2H,s), 3.7-3.1(8H,m), 3.0-2.7(1H,m), 2.50-2.0(2H,m and 6H,s), 1.9-0.7(35H,m) | 2910, 2850, 1460, 1450, 1110 | 521($M^+$), 430, 398 |
| (c) | 16 | 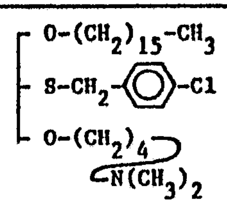 | Rf=0.64 (AcOEt:AcOH: water=3:1:1) | 7.17(4H,s), 3.77(2H, s), 3.7-3.1(8H,m), 3.0-2.7 (1H,m), 2.5-2.0(2H,m and 6H,s), 1.9-0.7 (35H,m) | 2910, 2850, 1460, 1450, 1110 | 555($M^+$), 464, 432 |
| (d) | 16(b) | 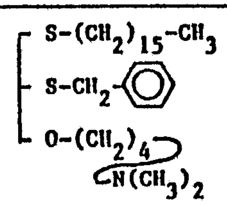 | Rf=0.64 ($CH_2Cl_2$:MeOH =10:1) | 3.78(2H,s), 3.50-3.12 4H,m), 2.73-2.62 (2H,m), 2.24(6H,s), 0.87(3H, broad t) | 2950, 1130 | 537($M^+$), 446 |

Reference Example 17

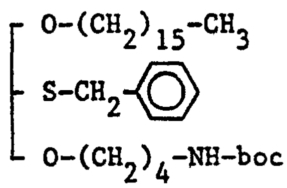

By the same procedure as described in Example 8, the title compound having the following physical data were prepared.

Starting Material: the mesyl compound prepared in Reference Example 16(c);

TLC (AcOEt:n-hexane=1:8): Rf=0.60;

NMR: δ=7.15 (5H, broad s), 3.77 (2H, s), 3.7-3.0 (8H, m), 2.70-2.50 (2H, m), 2.29 (9H, s), 0.88 (3H, broad t);

IR: ν=3300, 1695, 1520 $cm^{-1}$;

Mass: m/e=593 ($M^+$), 536, 520, 502, 492, 470.

Example 9

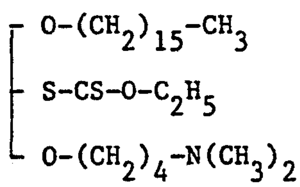

Under an atmosphere of argon, 100 mg of potassium O-ethyl dithiocarbonate (i.e. [$C_2H_5$O-CS-S]K) were added to a

solution of 110 mg of the mesyl compound (prepared in Reference Example 16) in one ml of DMF, and the solution was stirred overnight at 70°C. To the reaction mixture was added water and the mixture was extracted with AcOEt. The extract was washed, dried and concentrated, and the residue was purified by column chromatography on silica gel (a mixture of $CHCl_3$ and MeOH) to give 19 mg of the title compound having the following physical data:

TLC (AcOEt:AcOH:water=3:1:1): Rf=0.69;

NMR: δ=4.67-3.23 (8H, m), 3.23-2.93 (1H, m), 2.87-2.45 (2H, q), 2.45-2.10 (2H, m), 2.33 (6H, s), 1.87-0.63 (38H, m);

IR: ν=2940, 2870, 1460, 1370, 1115 $cm^{-1}$;

Mass: m/e=490, 430, 398, 385, 116, 100.

By the same procedure as described in Example 9, the compound having the following physical data was prepared.

(a) 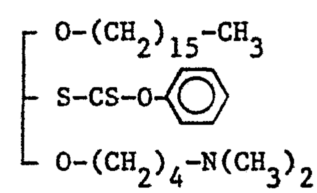

Starting Material: the mesyl compound prepared in Reference Example 16;

TLC (AcOEt:AcOH:water=3:1:1): Rf=0.72;

NMR: δ=7.6-6.8 (5H, m), 4.67-3.23 (8H, m), 3.23-2.93 (1H, m),

2.45-2.10 (2H, m), 2.33 (6H, s), 1.87-0.63 (35H, m);

IR: ν=2940, 2870, 1460, 1370, 1115 $cm^{-1}$;

Mass: m/e=490, 430, 398.

Example 10

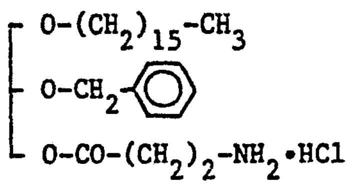

To a solution of 329 mg of the boc compound [prepared in Reference Example 11(a)] in 5 ml of $CHCl_3$ were added 0.3 ml of a 30% solution of hydrogen bromide in AcOH with cooling under ice, and the solution was stirred for 30 minutes. To the reaction mixture were added 5 ml of water and the mixture was extracted with AcOEt and the extract was concentrated. The residue was purified by column chromatography on silica gel (a mixture of $CHCl_3$, MeOH and triethylamine) and concentrated after the addition of a 1N hydrochloric acid to give 30 mg of the title compound having the following physical data:

TLC ($CHCl_3$:MeOH:AcOH=10:1:1): Rf=0.41;

NMR: δ=7.35 (5H, broad s), 4.66 (2H, s), 4.5-4.1 (2H, m), 3.9-3.7 (1H, m), 3.7-3.1 (6H, m), 3.1-2.75 (2H, m), 1.8-1.1 (28H, m), 1.0-0.8 (3H, m);

IR: ν=3700-3300, 3300-2200, 1730, 1600, 1460 $cm^{-1}$;

Mass: m/e=477 ($M^+$), 406, 282, 253.

By the same procedure as described in Example 10, the compound having the following physical data was prepared.

(a) 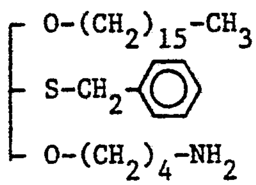

Starting Material: the boc compound prepared in Reference Example 17;

TLC (AcOEt:AcOH:water=3:1:1): Rf=0.60;

NMR: δ=7.16 (5H, broad s), 3.80 (2H, s), 3.75-3.15 (8H, m), 2.35-2.05 (2H, m), 0.87 (3H, broad t);

IR: ν=3300, 1120 $cm^{-1}$;

Mass: m/e=493 ($M^+$), 402, 370.

Example 11

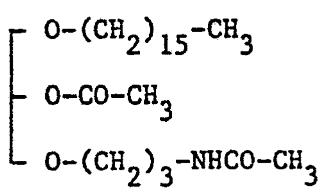

A mixture of 37 mg of the amino compound (prepared in Example 3), one ml of pyridine and 0.1 ml of acetic anhydride was stirred for 2 hours at room temperature. The reaction mixture was concentrated and the residue was purified by column chromatography on silica gel (a mixture of $CHCl_3$ and MeOH) to give 21 mg of the title compound having the following physical data:

TLC ($CHCl_3$:MeOH=10:1): Rf=0.55;

NMR: δ=6.1-6.0 (1H, m), 5.18-5.0 (1H, m), 3.6-3.18 (10H, m), 2.05- (3H, s), 1.94 (3H, s), 1.8-1.65 (2H, m), 1.6-1.44 (2H, m), 1.4-1.14 (26H, m), 0.94-0.8 (3H, m);

IR: ν=3600-3100, 2910, 2850, 1740, 1650 $cm^{-1}$.

By the same procedure as described in Example 11, the compound having the following physical data was prepared.

(a)

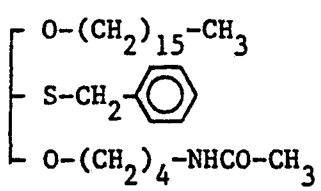

Starting Material: the amino compound prepared in Example 10(a);

TLC (AcOEt:n-hexane=1:8): Rf=0.20;

NMR: δ=7.15 (5H, broad s), 3.75 (2H, s), 3.70-3.20 (8H, m), 2.65-2.45 (2H, m), 2.00 (3H, s), 0.88 (3H, broad t);

IR: ν=3350, 1680, 1520 $cm^{-1}$;

Mass: m/e=535 ($M^+$), 444.

Example 12

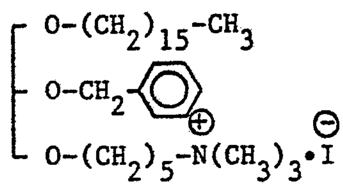

To a solution of 249 mg of the dimethylamino compound [prepared in Example 1(h)] in 5 ml of MeOH were added 0.45 ml of methyl iodide, and the mixture was stirred for 2 hours at room temperature. The reaction mixture was concentrated and the residue was purified by column chromatography on silica gel (a mixture of $CHCl_3$ and MeOH) to give 260 mg of the title compound as pale yellow crystal having the following physical data:

Melting point: 78-81°C;

NMR: δ=7.45-7.22 (5H, m), 4.69 (2H, q), 3.80-3.65 (1H, m), 3.65-3.38 (10H, m), 3.33 (9H, s), 1.90-1.40 (8H, m), 1.40-1.10 (26H, m), 0.88 (3H, t);

IR (KBr method): ν=3440, 2920, 2850, 1620, 1470, 1120, 1055, 945 $cm^{-1}$.

By the same procedure as described in Example 12, the compounds having the following physical data in Table 10 were prepared.

Table 10(1)

| Example No. | Example No. of Starting Material | Structure of Product | Physical Data: TLC (developing solvent) | NMR | IR | MS |
|---|---|---|---|---|---|---|
| (a) | 1(p) | 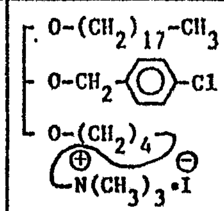 | Rf=0.45 (AcOEt:AcOH: water=3:1:1) | 7.44-7.28(4H,m), 4.68 (2H,t), 3.80-3.65(1H,m), 3.65-3.38(10H,m), 3.32 (9H,s), 1.93-1.46(6H,m), 1.45-1.13(30H,m), 0.88 (3H,m) | 2920, 2850, 1480, 1460, 1450, 1110 | 553, 428, 412 |
| (b) | 1(q) | 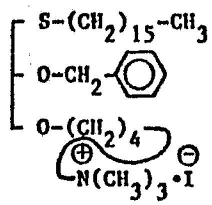 | Rf=0.38 (AcOEt:AcOH: water=3:1:1)<br>Melting point: 48-55°C | 7.40-7.28(5H,m), 4.63 (2H,q), 3.76-3.40(7H,m), 3.27(9H,s), 2.71(2H,d), 2.52(2H,t), 1.95-1.45 (6H,m), 1.26(26H,m), 0.88(3H,m) | 3450, 2930, 2850, 1470,1120 | 521, 414 |
| (c) | 1(a) | 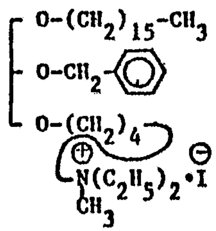 | Rf=0.63 (AcOEt:AcOH: water=3:1:1)<br>Melting point: 41-45°C | 7.40-7.25(5H,m), 4.66 (2H,q), 3.77-3.65(1H,m), 3.63-3.30(14H,m), 3.09 (3H,s), 1.90-1.46(6H,m), 1.16(32H,m), 0.88(3H,m) | 3450, 2920, 2850, 1460, 1110<br>(KBr method) | 588, 533, 519, 504, 490 |
| (d) | 1(a) | 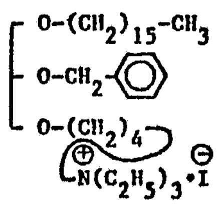 | Rf=0.49 (AcOEt:AcOH: water=3:1:1)<br>Melting point: 52-54°C | 7.4-7.26(5H,m), 4.66(2H, q), 3.8-3.64(1H,m), 3.64-3.38(8H,m), 3.3(6H,q), 3.38-3.22(2H,m), 1.92-1.46 (6H,m), 1.26(35H,m), 0.88 (3H,m) | 2920, 2840, 1450 | 585, 533, 518 504, 442, 426 |

Table 10(2)

| Example No. | Example No. of Starting Material | Structure of Product | Physical Data: TLC (developing solvent) | Physical Data: NMR | Physical Data: IR | Physical Data: MS |
|---|---|---|---|---|---|---|
| (e) | 1(b) | 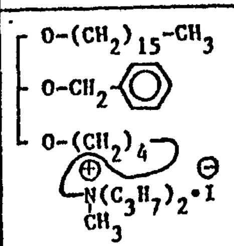 | Rf=0.66 (AcOEt:AcOH: water=3:1:1) | 7.39-7.25(5H,m), 4.67 (2H,q), 3.77-3.65(1H,m), 3.60-3.22(14H,m), 3.16 (3H,s), 1.94-1.40(10H,m) 1.26(26H,m), 2.00(6H,t), 0.88(3H,m) | 3500, 2920, 2860, 1460, 1110 | 588, 561, 533, 504, 454 |
| (f) | 1(c) | 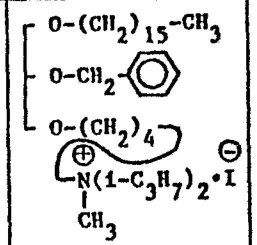 | Rf=0.68 (AcOEt:AcOH: water=3:1:1) | 7.38-7.24(5H,m), 4.66 (2H,q), 4.04-3.86(2H,m), 3.78-3.64(1H,m), 3.64-3.24(10H,m), 2.99(3H,s), 2.00-1.68(2H,m), 1.68-1.50(4H,m), 1.43(12H,t), 1.26(26H,m), 0.88(3H,m) | 3400, 2930, 2850 1460, 1110 | 588, 561, 546, 533 |
| (g) | 1(d) | 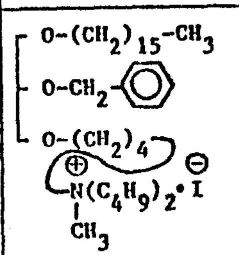 | Rf=0.75 (AcOEt:AcOH: water=3:1:1) | 7.39-7.14(5H,m), 4.65 (2H,q), 3.78-3.63(1H,m), 3.62-3.25(14H,m), 3.14 (3H,s), 1.92-1.18(40H, m), 0.98(6H,t), 0.87 (3H,m) | 3400, 2940, 2860, 1460, 1120 | 589, 546, 504, 482, 440 |
| (h) | 1(e) | 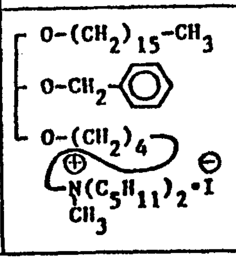 | Rf=0.81 (AcOEt:AcOH: water=6:2:1) | 7.40-7.25(5H,m), 4.66 (2H,q), 3.78-3.66(1H,m), 3.64-3.22(14H,m), 3.14 (3H,s), 1.92-1.46(10H,m), 1.46-1.20(34H,m), 1.06-0.80(9H,m) | 3450, 2930, 2860, 1660, 1460, 1110 | 617, 560, 504 |

Table 10(3)

| Example No. | Example No. of Starting Material | Structure of Product | Physical Data: TLC (developing solvent) | Physical Data: NMR | Physical Data: IR | Physical Data: MS |
|---|---|---|---|---|---|---|
| (i) | 1(f) | 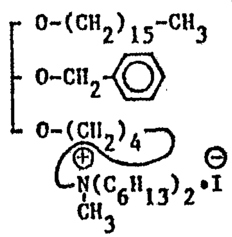 | Rf=0.85 (AcOEt:AcOH: water=6:2:1) | 7.40-7.24(5H,m), 4.67 (2H,q), 3.78-3.65(1H,m), 3.65-3.20(14H,m), 3.14 (3H,s), 1.90-1.45(10H,m) 1.45-1.20(38H,m), 2.00-0.80(9H,m) | 3450, 2940, 2860, 1660, 1460, 1110 | 645, 574, 504, 468 |
| (j) | 1(r) | 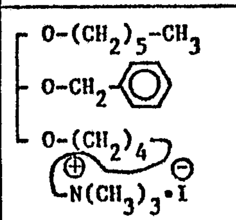 | Rf=0.47 (AcOEt:AcOH: water=3:1:1)<br>Melting point: 30-40°C | 7.42-7.28(5H,m), 4.66 (2H,q), 3.80-3.66(1H,m), 3.66-3.49(8H,m), 3.44 (2H,t), 3.27(9H,s), 1.96-1.49(6H,m), 1.45-1.24 (6H,m), 0.89(3H,m) | 2920, 2850, 1480, 1450, 1110 | 365, 350, 280, 274, 258, 250 |
| (k) | 1(s) | 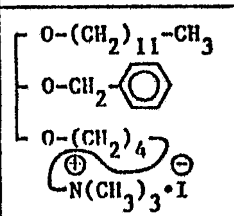 | Rf=0.47 (AcOEt:AcOH: water=3:1:1)<br>Melting Point: 40-45°C | 7.41-7.27(5H,m), 4.67 (2H,q), 3.80-3.67(1H,m), 3.67-3.48(8H,m), 3.44 (2H,t), 3.25(9H,s), 1.96-1.45(6H,m), 1.25 (18H,m), 0.87(3H,m) | 2920, 2850, 1480, 1450, 1110 | 449, 434, 358, 342, 280, 250 |
| (l) | 6(b) | 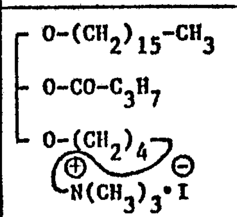 | Rf=0.58 (AcOEt:AcOH: water=3:1:1)<br>Melting point: 58-60°C | 5.26-5.1(1H,m), 3.42 (9H,s), 3.74-3.34(10H,m), 2.31(2H,t), 2.0-1.4(8H, m), 1.24(26H,m), 1.94 (3H,t), 0.88(3H,m) | 2920, 2840, 1730, 1480, 1460, 1260, 1110 | 485, 414, 398, 369 |

Table 10(4)

| Example No. | Example No. of Starting Material | Structure of Product | Physical Data: TLC (developing solvent) | Physical Data: NMR | Physical Data: IR | Physical Data: MS |
|---|---|---|---|---|---|---|
| (m) | 6(c) | 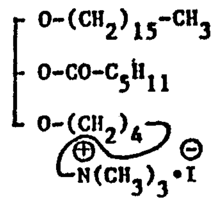 | Rf=0.53 (AcOEt:AcOH: water=3:1:1)<br>Melting point: 67-70°C | 5.38-5.12(1H,m), 3.42 (9H,s), 3.74-3.30(10H, m), 2.64-2.40(2H,m), 2.00-1.40(8H,m), 1.26 (30H,m), 1.00-0.80 (6H,m) | 2920, 2840, 1740, 1460 | 612, 513, 470 414, 398 |
| (n) | 6(d) | 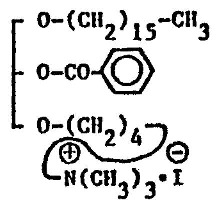 | Rf=0.36 (AcOEt:AcOH: water=3:1:1)<br>Melting point: 45-49°C | 8.12-8.00(2H,m), 7.66-7.40(3H,m), 5.52-5.37 (1H,m), 3.78-3.36(10H, m), 3.29 (9H,s), 1.92-1.40(6H,m), 1.40-1.10 (26H,m), 0.87(3H,t) | 2920, 2845, 1720, 1465, 1445, 1275, 1115, 715<br>(KBr method) | |
| (o) | 7 | 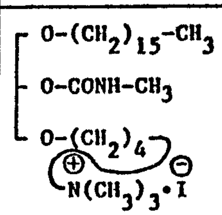 | Rf=0.42 (AcOEt:AcOH: water=3:1:1)<br>Melting point: 62-64°C | 5.10-4.94(2H,m), 3.4 (9H,s), 3.74-2.30(10H, m), 3.78(3H,d), 2.0-1.45 (6H,m), 1.25(26H,m), 0.88(3H,t) | 3400, 2920, 2840, 1690, 1530, 1450, 1260 | 472, 414, 398 |
| (p) | 7(a) | 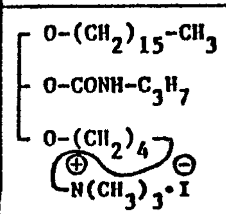 | Rf=0.64 (AcOEt:AcOH: water=3:1:1)<br>Melting point: 64-66°C | 5.08-4.9(2H,m), 3.41 (9H,s), 3.72-3.36(10H,m), 3.22-3.02(2H,m), 2.0-1.2 (8H,m), 1.25(26H,m), 0.92(3H,t), 0.88(3H,m) | 3400, 2920, 2840, 1690, 1450, 1260, 1110 | 500, 471, 415, 414 |

Table 10(5)

| Example No. | Example No. of Starting Material | Structure of Product | Physical Data: TLC (developing solvent) | NMR | IR | MS |
|---|---|---|---|---|---|---|
| (q) | 7(b) | 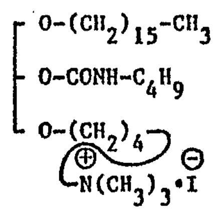 | Rf=0.68 (AcOEt:AcOH: water=3:1:1) Melting point: 56-59°C | 5.08-4.84(2H,m), 3.41 (9H,s), 3.72-3.32(10H,m), 3.24-3.02(2H,m), 2.0-1.35(8H,m), 1.25(28H,m), 0.92(3H,t), 0.88(3H,m) | 3300, 2920, 2840, 1680, 1460, 1270 | 514, 414, 398 |
| (r) | 7(c) | 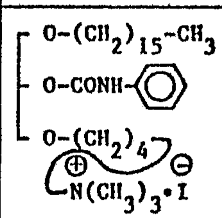 | Rf=0.45 (AcOEt:AcOH: water=3:1:1) | 7.52-7.4(2H,m), 7.38-7.18(2H,m), 7.12-7.0 (1H,m), 5.26-5.1(1H,m), 3.74-3.3(1H,m), 3.22 (9H,s), 1.96-1.42(6H, m), 1.25(26H,m), 0.88 (3H,m) | 3450, 3250, 2910, 2840, 1720, 1590, 1520, 1440 | 534, 519, 414, 398 |
| (s) | 8 | 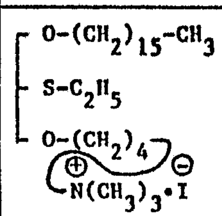 | Rf=0.48 (AcOEt:AcOH: water=3:1:1) Melting Point: 56-60°C | 3.9-3.3(19H,m), 3.05-2.88(1H,m), 2.63(2H,q), 2.04-1.83(2H,m), 1.83-1.65(2H,m), 1.65-1.45 (2H,m), 1.26(26H,m), 0.88(3H,m) | 3600-3100, 3010, 2910, 2840, 1480 1460, 1120 | 459, 430, 398 |
| (t) | 8(a) | 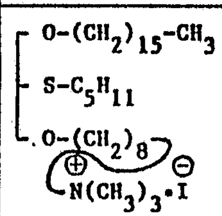 | Rf=0.39 (AcOEt:AcOH: water=3:1:1) | 3.80-3.43(10H,m), 3.32 (9H,s), 3.05-2.94(1H,m), 2.58-2.46(2H,t), 1.85-1.45(8H,m), 1.26(38H,m), 0.88(6H,m) | 2920, 2850, 1480, 1120 | 557 |

Table 10(6)

| Example No. | Example No. of Starting Material | Structure of Product | Physical Data: TLC (developing solvent) | Physical Data: NMR | Physical Data: IR | Physical Data: MS |
|---|---|---|---|---|---|---|
| (u) | 8(b) | 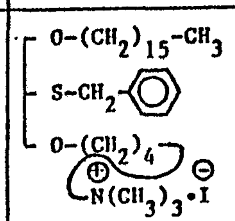 | Rf=0.57 (AcOEt:AcOH: water=3:1:1)<br>Melting point: 58-62°C | 7.3(5H,m), 3.82(2H,s), 3.75-3.4(10H,m), 3.36 (9H,s), 3.0-2.8(1H,m), 2.0-1.79(2H,m), 1.79-1.63(2H,m), 1.62-1.45 (2H,m), 1.25(26H,m), 0.88(3H,t) | 3600-3100, 2920, 2850, 1470, 1110<br>(KBr method) | 521, 430, 398 |
| (v) | 8(c) | 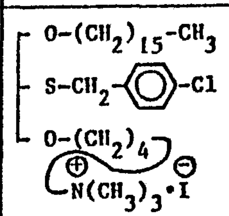 | Rf=0.57 (AcOEt:AcOH: water=3:1:1) | 7.3(4H,m), 3.82(2H,s), 3.75-3.4(10H,m), 3.36 (9H,s), 3.0-2.8(1H,m), 2.0-1.79(2H,m), 1.79-1.63(2H,m), 1.62-1.45 (2H,m), 1.25(26H,m), 0.88(3H,m) | 3450, 2920, 2850, 1470, 1110 | 555, 464, 432 |
| (w) | 8(d) | 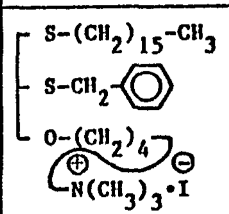 | Rf=0.40 (AcOEt:AcOH: water=3:1:1) | 3.76(2H,s), 3.65-3.0 (4H,m), 3.20-3.10(2H,m), 3.10(9H,s) | 2950, 1125 | 537, 446 |
| (x) | 9 | 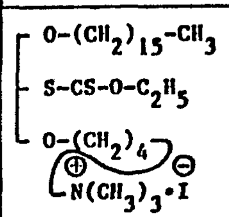 | Rf=0.77 (AcOEt:AcOH: water=3:1:1)<br>Melting point: 69-72°C | 3.66-3.36(10H,m), 3.44 (9H,s), 3.18-3.00(1H,m), 2.71(2H,q), 2.04-1.45 (6H,m), 1.45-1.16(26H, m), 1.32(3H,t), 0.88 (3H,t) | 2925, 2855, 1620 1480, 1460, 1110<br>(KBr method) | 490, 430, 398, 385, 142, 116, 100 |

Table 10(7)

| Example No. | Example No. of Starting Material | Structure of Product | Physical Data | | | |
|---|---|---|---|---|---|---|
| | | | TLC (developing solvent) | NMR | IR | MS |
| (y) | 9(a) | 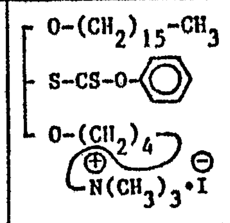 | Rf=0.69 (AcOEt:AcOH: water=3:1:1) | 7.6-6.8(5H,m), 3.66-3.36(10H,m), 3.44(9H,s), 3.18-3.00(1H,m), 2.04-1.45(6H,m), 1.45-1.16 (26H,m), 0.88(3H,m) | 2925, 2855, 1620 1480, 1460, 1110 | 490, 430 |

Reference Example 18

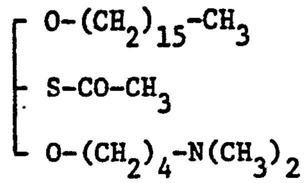

Under an atmosphere of argon, a mixture of 115 mg of thiolacetic acid, 60 mg of potassium hydride (content: 35%) and 5 ml of dry DMF was stirred for 10 minutes at room temperature. Then, to the solution was added a solution of 500 mg of the mesyl compound (prepared in Reference Example 16) in 10 ml of dry DMF and the solution was stirred overnight at 50°C. The reaction mixture was concentrated to distill off DMF. To the residue was added 50 ml of $CHCl_3$ and the solution was washed, dried and concentrated. The residue was purified by column chromatography on silica gel (a mixture of $CHCl_3$ and MeOH) to give 455 mg of the title compound having the following physical data:

TLC (AcOEt:AcOH:water=3:1:1): Rf=0.60;

NMR: $\delta$=3.8-3.1 (9H, m), 2.4-2.2 (11H, m), 1.8-0.7 (35H, m);

IR: $\nu$=2920, 2850, 1690, 1460, 1110 $cm^{-1}$;

Mass: m/e=473 ($M^+$), 430.

Example 13

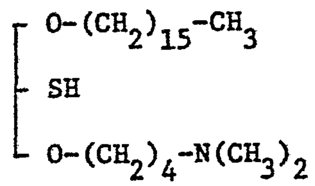

Under an atmosphere of argon, a mixture of 455 mg of the acetyl compound (prepared in Reference Example 18), 5 ml of triethylamine and 15 ml of methanol was refluxed for one hour and the reaction mixture was concentrated. The residue was purified by column chromatography on silica gel (a mixture of $CHCl_3$ and MeOH) to give 400 mg of the title compound having the following physical data:

TLC (AcOEt:AcOH:water=3:1:1): Rf=0.59;

NMR: $\delta$=3.8-3.0 (9H, m), 2.4-2.0 (8H, m), 1.8-0.7 (36H, m);

IR: $\nu$=2920, 2850, 1460, 1110 $cm^{-1}$;

Mass: m/e=431 ($M^+$), 416, 398.

Example 14

5 g of (2RS)-1-O-hexadecyl-2-O-benzyl-3-O-[4-(N,N,N-trimethylammonio)butyl]glycerol chloride, 200 mg of cellulose calcium gluconate (disintegrator), 100 mg of magnesium stearate (lubricant) and 9.7 g of crystalline cellulose were mixed and punched out in a usual manner to obtain tablets each containing 50 mg of the active ingredient.

CLAIMS

1. A glycerol derivative of the general formula:

$$\begin{array}{ll} 1 & \ulcorner A\text{-}R^1 \\ 2 & \vdash B\text{-}R^2 \\ 3 & \llcorner D\text{-}R^3\text{-}R^4 \end{array} \qquad \text{I}$$

[wherein A represents an oxygen atom or a sulfur atom, B represents an oxygen atom or a sulfur atom, D represents an oxygen atom or a carbonyloxy group (i.e. $-O\underset{\underset{O}{\|}}{C}-$ , in which the carbonyl group should be attached to a group $R^3$ and the oxa group should be attached to the carbon atom at the third position of the glycerol skeleton), $R^1$ represents an alkyl group of 6 to 22 carbon atoms, or an alkyl group of 2 to 5 carbon atoms being substituted by a phenyl group, $R^2$ represents, when B is an oxygen atom, a hydrogen atom, or a group of the general formula: $-CH_2$-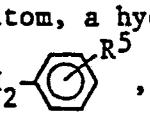 , $-COR^6$ or $-CONHR^7$, in which $R^5$ represents a hydrogen atom, a halogen atom, an alkyl group of 1 to 4 carbon atoms or an alkoxy group of 1 to 4 carbon atoms, $R^6$ represents an alkyl group of 1 to 6 carbon atoms or a phenyl group and $R^7$ represents an alkyl group of 1 to 6 carbon atoms or a phenyl group, and $R^2$ represents, when B is a sulfur atom, a hydrogen atom, an alkyl group of 1 to 6 carbon atoms, or a group

of the general formula: $-CH_2-$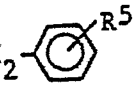 or $-CS-OR^8$, in which $R^8$ represents an alkyl group of 1 to 6 carbon atoms or a phenyl group and $R^5$ is as hereinbefore defined, $R^3$ represents an alkylene group of 1 to 12 carbon atoms and $R^4$ represents an amino group, or a group of the general formula: $-NHCOR^9$, $-NHR^{10}$, $-N(R^{10})_2$, $-\overset{\oplus}{N}(R^{10})_3\cdot\overset{\ominus}{Y}$, in which $R^9$ represents an alkyl group of 1 to 6 carbon atoms or a phenyl group, $R^{10}$ represents an alkyl group of 1 to 8 carbon atoms, with the proviso that, when $R^4$ represents a group of the formula $-N(R^{10})_2$ or $-\overset{\oplus}{N}(R^{10})_3\cdot\overset{\ominus}{Y}$, two or more of $R^{10}$ may be same or different to each other, and $\overset{\ominus}{Y}$ represents an anion of an acid], or a non-toxic acid addition salt thereof.

2. A glycerol derivative according to claim 1 wherein A represents an oxygen atom.

3. A glycerol derivative according to claim 1 wherein A represents a sulfur atom.

4. A glycerol derivative according to claim 1 wherein D represents an oxygen atom.

5. A glycerol derivative according to claim 1 wherein D represents a carbonyloxy group.

6. A glycerol derivative according to claim 1 wherein B represents an oxygen atom and $R^2$ represents a hydrogen atom.

7. A glycerol derivative according to claim 6 which is:

(2RS)-1-O-hexadecyl-3-O-(4-aminobutyl)glycerol,

(2RS)-1-O-hexadecyl-3-O-[4-(N,N-dimethylamino)butyl]glycerol,

(2RS)-1-O-hexadecyl-3-O-[4-(N,N,N-trimethylammonio)butyl]glycerol halide,

(2RS)-1-O-hexadecyl-3-O-[8-(N,N-dimethylamino)octyl]glycerol or

(2RS)-1-hexadecylthio-3-O-[4-(N,N-dimethylamino)butyl]-2,3-propanediol.

8. A glycerol derivative according to claim 1 wherein B represents an oxygen atom and $R^2$ represents a group of the general formula: $-CH_2-$ 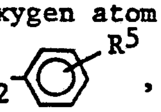 , in which $R^5$ is as defined in claim 1.

9. A glycerol derivative according to claim 8 which is:

(2RS)-1-O-hexyl-2-O-benzyl-3-O-[4-(N,N-dimethylamino)butyl]-glycerol,

(2RS)-1-O-hexyl-2-O-benzyl-3-O-[4-(N,N,N-trimethylammonio)butyl]-glycerol halide,

(2RS)-1-O-dodecyl-2-O-benzyl-3-O-[4-(N,N-dimethylamino)butyl]-glycerol,

(2RS)-1-O-dodecyl-2-O-benzyl-3-O-[4-(N,N,N-trimethylammonio)butyl]-glycerol halide,

(2RS)-1-O-hexadecyl-2-O-benzyl-3-O-(4-aminobutyl)glycerol,

(2RS)-1-O-hexadecyl-2-O-benzyl-3-O-[4-(N,N-dimethylamino)butyl]-glycerol,

(2RS)-1-O-hexadecyl-2-O-benzyl-3-O-[4-(N,N,N-trimethylammonio)-butyl]glycerol halide,

(2RS)-1-O-hexadecyl-2-O-benzyl-3-O-[4-(N,N-diethylamino)butyl]-glycerol,

(2RS)-1-O-hexadecyl-2-O-benzyl-3-O-[4-(N,N-diethyl-N-methyl-ammonio)butyl]glycerol halide,

(2RS)-1-O-hexadecyl-2-O-benzyl-3-O-[4-(N,N,N-triethylammonio)-butyl]glycerol halide,

(2RS)-1-O-hexadecyl-2-O-benzyl-3-O-[4-(N,N-dipropylamino)butyl]-glycerol,

(2RS)-1-O-hexadecyl-2-O-benzyl-3-O-[4-(N,N-dipropyl-N-methyl-ammonio)butyl]glycerol halide,

(2RS)-1-O-hexadecyl-2-O-benzyl-3-O-[4-(N,N-di-isopropylamino)-butyl]glycerol,

(2RS)-1-O-hexadecyl-2-O-benzyl-3-O-[4-(N,N-di-isopropyl-N-methylammonio)butyl]glycerol halide,

(2RS)-1-O-hexadecyl-2-O-benzyl-3-O-[4-(N,N-dibutylamino)butyl]-glycerol,

(2RS)-1-O-hexadecyl-2-O-benzyl-3-O-[4-(N,N-dibutyl-N-methyl-ammonio)butyl]glycerol halide,

(2RS)-1-O-hexadecyl-2-O-benzyl-3-O-[4-(N,N-dipentylamino)butyl]-glycerol,

(2RS)-1-O-hexadecyl-2-O-benzyl-3-O-[4-(N,N-dipentyl-N-methyl-ammonio)butyl]glycerol halide,

(2RS)-1-O-hexadecyl-2-O-benzyl-3-O-[4-(N,N-dihexylamino)butyl]-

glycerol,

(2RS)-1-O-hexadecyl-2-O-benzyl-3-O-[4-(N,N-dihexyl-N-methyl-ammonio)butyl]glycerol halide,

(2RS)-1-O-hexadecyl-2-O-benzyl-3-O-[5-(N,N-dimethylamino)pentyl]-glycerol,

(2RS)-1-O-hexadecyl-2-O-benzyl-3-O-[5-(N,N,N-trimethylammonio)-pentyl]glycerol halide,

(2RS)-1-O-hexadecyl-2-O-benzyl-3-O-[6-(N,N,N-trimethylammonio)-hexyl]glycerol halide,

(2RS)-1-O-hexadecyl-2-O-benzyl-3-O-[7-(N,N,N-trimethylammonio)-heptyl]glycerol halide,

(2RS)-1-O-hexadecyl-2-O-benzyl-3-O-[8-(N,N-dimethylamino)octyl]-glycerol,

(2RS)-1-O-hexadecyl-2-O-benzyl-3-O-[8-(N,N,N-trimethylammonio)-octyl]glycerol halide,

(2RS)-1-O-hexadecyl-2-O-benzyl-3-O-[9-(N,N,N-trimethylammonio)-nonyl]glycerol halide,

(2RS)-1-O-hexadecyl-2-O-benzyl-3-O-[10-(N,N,N-trimethylammonio)-decyl]glycerol halide,

(2RS)-1-O-hexadecyl-2-O-benzyl-3-O-(3-aminopropionyl)glycerol,

(2RS)-1-O-hexadecyl-2-O-benzyl-3-O-[3-(N,N,N-trimethylammonio)-propionyl]glycerol halide,

(2RS)-1-O-hexadecyl-2-O-benzyl-3-O-[4-(N,N,N-trimethylammonio)-butyryl]glycerol halide,

(2RS)-1-O-octadecyl-2-O-(4-chlorophenyl)methyl-3-O-[4-(N,N-dimethylamino)butyl]glycerol,

(2RS)-1-O-octadecyl-2-O-(4-chlorophenyl)methyl-3-O-[4-(N,N,N-trimethylammonio)butyl]glycerol halide,

(2RS)-1-O-eicosyl-2-O-benzyl-3-O-[4-(N-methylamino)butyl]glycerol,

(2RS)-1-hexadecylthio-2-O-benzyl-3-O-[4-(N,N-dimethylamino)butyl]-2,3-propanediol or

(2RS)-1-hexadecylthio-2-O-benzyl-3-O-[4-(N,N,N-trimethylammonio)-butyl]-2,3-propanediol halide.

10. A glycerol derivative according to claim 1 wherein B represents an oxygen atom and $R^2$ represents a group of the general formula: $-COR^6$, in which $R^6$ is as defined in claim 1.

11. A glycerol derivative according to claim 10 which is:

(2RS)-1-O-hexadecyl-2-O-acetyl-3-O-(3-aminopropyl)glycerol,

(2RS)-1-O-hexadecyl-2-O-acetyl-3-O-[3-(N-acetylamino)propyl]-glycerol,

(2RS)-1-O-hexadecyl-2-O-acetyl-3-O-[4-(N-acetylamino)butyl]-glycerol,

(2RS)-1-O-hexadecyl-2-O-acetyl-3-O-[4-(N,N-dimethylamino)butyl]-glycerol,

(2RS)-1-O-hexadecyl-2-O-acetyl-3-O-[4-(N,N,N-trimethylammonio)-butyl]glycerol halide,

(2RS)-1-O-hexadecyl-2-O-butyryl-3-O-[4-(N,N-dimethylamino)butyl]-glycerol,

(2RS)-1-O-hexadecyl-2-O-butyryl-3-O-[4-(N,N,N-trimethylammonio)-butyl]glycerol halide,

(2RS)-1-O-hexadecyl-2-O-hexanoyl-3-O-[4-(N,N-dimethylamino)butyl]-glycerol,

(2RS)-1-O-hexadecyl-2-O-hexanoyl-3-O-[4-(N,N,N-trimethylammonio)-butyl]glycerol halide,

(2RS)-1-O-hexadecyl-2-O-benzoyl-3-O-[4-(N,N-dimethylamino)butyl]-glycerol,

(2RS)-1-O-hexadecyl-2-O-benzoyl-3-O-[4-(N,N,N-trimethylammonio)-butyl]glycerol halide,

(2RS)-1-O-octadecyl-2-O-acetyl-3-O-(3-aminopropionyl)glycerol,

12. A glycerol derivative according to claim 1 wherein B represents an oxygen atom and $R^2$ represents a group of the general formula: $-CONHR^7$, in which $R^7$ is as defined in claim 1.

13. A glycerol derivative according to claim 12 which is:

(2RS)-1-O-hexadecyl-2-O-(N-methylcarbamoyl)-3-O-[4-(N,N-dimethyl-amino)butyl]glycerol,

(2RS)-1-O-hexadecyl-2-O-(N-methylcarbamoyl)-3-O-[4-(N,N,N-trimethylammonio)butyl]glycerol halide,

(2RS)-1-O-hexadecyl-2-O-(N-propylcarbamoyl)-3-O-[4-(N,N-dimethyl-amino)butyl]glycerol,

(2RS)-1-O-hexadecyl-2-O-(N-propylcarbamoyl)-3-O-[4-(N,N,N-trimethylammonio)butyl]glycerol halide,

(2RS)-1-O-hexadecyl-2-O-(N-butylcarbamoyl)-3-O-[4-(N,N-dimethylamino)butyl]glycerol,

(2RS)-1-O-hexadecyl-2-O-(N-butylcarbamoyl)-3-O-[4-(N,N,N-trimethylammonio)butyl]glycerol halide,

(2RS)-1-O-hexadecyl-2-O-(N-phenylcarbamoyl)-3-O-[4-(N,N-dimethylamino)butyl]glycerol or

(2RS)-1-O-hexadecyl-2-O-(N-phenylcarbamoyl)-3-O-[4-(N,N,N-trimethylammonio)butyl]glycerol halide.

14. A glycerol derivative according to claim 1 wherein B represents a sulfur atom and $R^2$ represents a hydrogen atom.

15. A glycerol derivative according to claim 14 which is (2RS)-1-O-hexadecyl-2-mercapt-3-O-[4-(N,N-dimethylamino)butyl]-1,3-propanediol.

16. A glycerol derivative according to claim 1 wherein B represents a sulfur atom and $R^2$ represents an alkyl group of 1 to 6 carbon atoms.

17. A glycerol derivative according to claim 16 which is:

(2RS)-1-O-hexadecyl-2-ethylthio-3-O-[4-(N,N-dimethylamino)butyl]-1,3-propanediol,

(2RS)-1-O-hexadecyl-2-ethylthio-3-O-[4-(N,N,N-trimethylammonio)-butyl]-1,3-propanediol halide,

(2RS)-1-O-hexadecyl-2-pentylthio-3-O-[8-(N,N-dimethylamino)octyl]-1,3-propanediol or

(2RS)-1-O-hexadecyl-2-pentylthio-3-O-[8-(N,N,N-trimethylammonio)-octyl]-1,3-propanediol halide.

18. A glycerol derivative according to claim 1 wherein B represents a sulfur atom and $R^2$ represents a group of the general formula: $-CH_2-$ 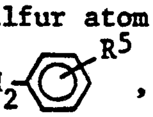 , in which $R^5$ is as defined in claim 1.

19. A glycerol derivative according to claim 18 which is:

(2RS)-1-O-hexadecyl-2-benzylthio-3-O-(4-aminobutyl)-1,3-propanediol,

(2RS)-1-O-hexadecyl-2-benzylthio-3-O-[4-(N-acetylamino)butyl]-1,3-propanediol,

(2RS)-1-O-hexadecyl-2-benzylthio-3-O-[4-(N,N-dimethylamino)-butyl]-1,3-propanediol,

(2RS)-1-O-hexadecyl-2-benzylthio-3-O-[4-(N,N,N-trimethylammonio)-butyl]-1,3-propanediol halide,

(2RS)-1-O-hexadecyl-2-(4-chlorophenyl)methylthio-3-O-[4-(N,N-dimethylamino)butyl]-1,3-propanediol,

(2RS)-1-O-hexadecyl-2-(4-chlorophenyl)methylthio-3-O-[4-(N,N,N-trimethylammonio)butyl]-1,3-propanediol halide,

(2RS)-1-hexadecylthio-2-benzylthio-3-O-[4-(N,N-dimethylamino)-butoxy]propane or

(2RS)-1-hexadecylthio-2-benzylthio-3-O-[4-(N,N,N-trimethylammonio)-butoxy]propane halide.

20. A glycerol derivative according to claim 1 wherein B represents a sulfur atom and $R^2$ represents a group of the general formula: $-CS-OR^8$, in which $R^8$ is as defined in claim 1.

21. A glycerol derivative according to claim 20 which is:
(2RS)-1-O-hexadecyl-2-[ethoxy(thiocarbonyl)thio]-3-O-[4-(N,N-dimethylamino)butyl]-1,3-propanediol,
(2RS)-1-O-hexadecyl-2-[ethoxy(thiocarbonyl)thio]-3-O-[4-(N,N,N-trimethylammonio)butyl]-1,3-propanediol halide,
(2RS)-1-O-hexadecyl-2-[phenoxy(thiocarbonyl)thio]-3-O-[4-(N,N-dimethylamino)butyl]-1,3-propanediol or
(2RS)-1-O-hexadecyl-2-[phenoxy(thiocarbonyl)thio]-3-O-[4-(N,N,N-trimethylammonio)butyl]-1,3-propanediol halide.

22. A process for the preparation of glycerol derivatives as claimed in claim 1 and conforming to the general formula:

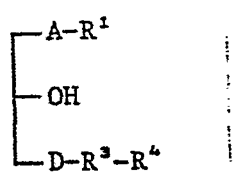

IA

(wherein the various symbols are as defined in claim 1) which comprises reductive elimination of the benzyl group attached to the 2nd position of a compound of the general formula:

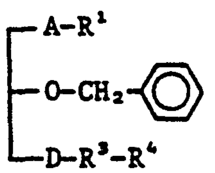

IB'

(wherein the various symbols are as hereinbefore defined).

23. A process for the preparation of glycerol derivatives as claimed in claim 1 and conforming to the general formula:

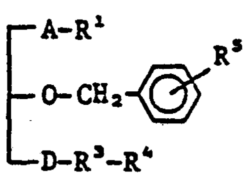

IB

(wherein the various symbols are as defined in claim 1 ) which comprises

(i) elimination of the trityl group of a compound of the general formula:

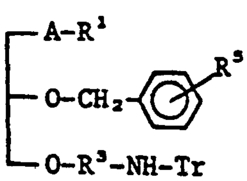

VIII

(wherein Tr represents a trityl group and the other symbols are as hereinbefore defined),

(ii) elimination of the group Q of a compound of the general formula:

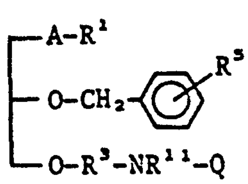

V

or

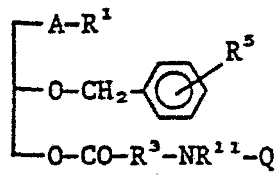

IX

(wherein $R^{11}$ represents a hydrogen atom or an alkyl group of 1 to 8 carbon atoms, Q represents a tert-butoxycarbonyl group or a benzyloxycarbonyl group and the other symbols are as hereinbefore defined),

(iii) reaction of a compound of the general formula:

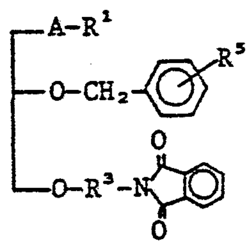

VI

(wherein the various symbols are as hereinbefore defined) with hydrazine,

(iv) reaction of a compound of the general formula:

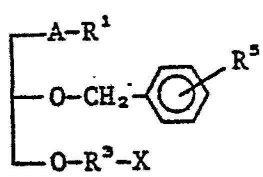

VII

(wherein X represents a chlorine, bromine or iodine atom and the other symbols are as hereinbefore defined) with a compound of the general formula:

$H_2N-R^{10}$ , $HN(R^{10})_2$ or $N(R^{10})_3$

XV XVI XVII

(wherein $R^{10}$ is as hereinbefore defined),

(v) acylation of a compound of the general formula:

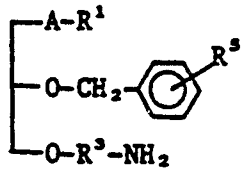

IB-11

(wherein the various symbols are as hereinbefore defined),

(vi) reaction of a compound of the general formula:

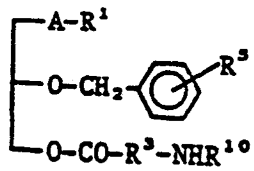

IB-22

(wherein the various symbols are as hereinbefore defined) with an equimolecular amount of alkyl halide,

(vii) reaction of a compound of the general formula:

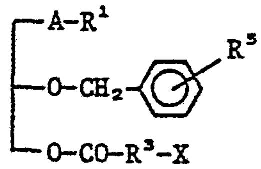

X

(wherein the various symbols are as hereinbefore defined) with a compound of the general formula XVII,

(viii) reaction of a compound of the general formula:

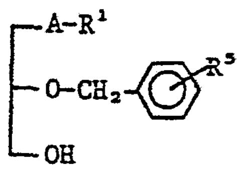

II

(wherein the various symbols are as hereinbefore defined) with a compound of the general formula:

$$HOOC-R^3-NHCOR^9 \qquad XX$$

(wherein $R^3$ and $R^9$ are as hereinbefore defined), or

(ix) subjecting a method for salt-exchange, if desired.

24. A process for the preparation of glycerol derivatives as claimed in claim 1 and conforming to the general formula:

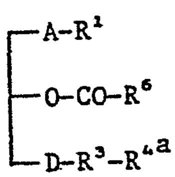

IC-1

and

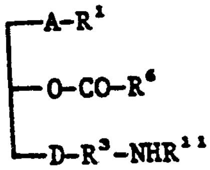

IC-2

(wherein $R^{4a}$ represents a group of the general formula: $-NHCOR^9$, $-N(R^{10})_2$ or $-\overset{\oplus}{N}(R^{10})_3 \cdot \overset{\ominus}{Y}$, in which the various symbols are as hereinbefore defined, $R^{11}$ represents a hydrogen atom or an alkyl group of 1 to 8 carbon atoms and the other symbols are as defined in claim 1) which comprises

(i) acylation of a compound of the general formula:

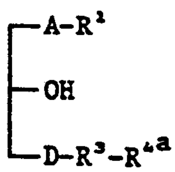

IA-1

(wherein the various symbols are as hereinbefore defined) or

(ii) elimination of the group Q of a compound of the general formula:

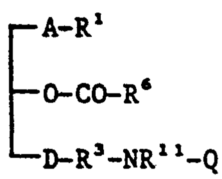

XXIV

(wherein Q represents a tert-butoxycarbonyl gorup or a benzyloxycarbonyl group and the other symbols are as hereinbefore defined).

25. A process for the preparation of glycerol derivatives as claimed in claim 1 and conforming to the general formula:

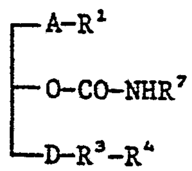

ID

(wherein the various symbols are as defined in claim 1 ) which comprises

(i) reaction of a compound of the general formula:

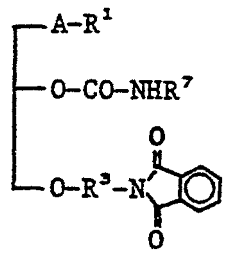

XXVIII

(wherein the various symbols are as hereinbefore defined) with hydrazine,

(ii) elimination of the group Q of a compound of the general formula:

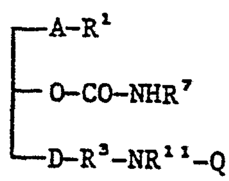

XXIX

(wherein Q represents a tert-butoxycarbonyl group or a benzyloxycarbonyl group, $R^{11}$ represents a hydrogen atom or an alkyl group of 1 to 8 carbon atoms and the other symbols are as hereinbefore defined),

(iii) reaction of a compound of the general formula:

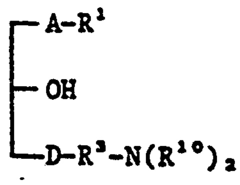

IA-3

or

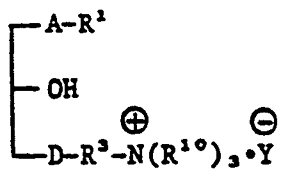

IA-4

(wherein the various symbols are as hereinbefore defined) with a compound of the general formula:

$R^7=N=C=O$ XXX

(wherein $R^7$ is as hereinbefore defined), or

(iv) acylation of a compound of the general formula:

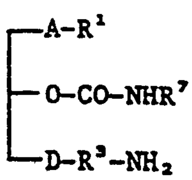

ID-1

(wherein the various symbols are as hereinbefore defined).

26. A process for the preparation of glycerol derivatives as claimed in claim 1 and conforming to the general formula:

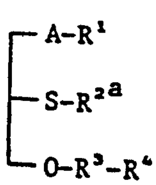

IE

0094586

(wherein $R^{2a}$ represents a hydrogen atom, an alkyl group of 1 to 6 carbon atoms or a group of the general formula: 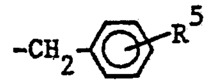 or $-CS-OR^8$, in which the various symbols are as defined in claim 1 and the other symbols are as defined in claim 1) which comprises

(i) reaction of a compound of the general formula:

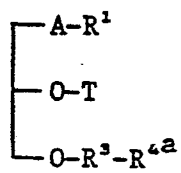

XXXI

(wherein T represents an alkylsulfonyl group or a substituted or unsubstituted arylsulfonyl group, $R^{4a}$ is as defined in claim 24 and the other symbols are as hereinbefore defined) with a compound of the general formula:

$HS-R^{12}$, 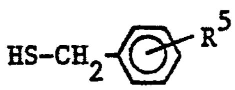 or 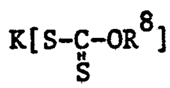

XXXIX XL XLI

(wherein $R^{12}$ represents an alkyl group of 1 to 6 carbon atoms and the other symbols are as hereinbefore defined),

(ii) elimination of the acetyl group or both acetyl group and trifluoroacetyl group of a compound of the general formula:

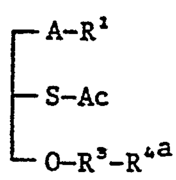

XXXII

or

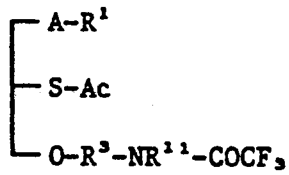

XXXVIII

(wherein Ac is acetyl group, $R^{11}$ represents a hydrogen atom or an alkyl group of 1 to 8 carbon atoms and the other symbols are as hereinbefore defined) or

(iii) elimination of the group Q of a compound of the general formula:

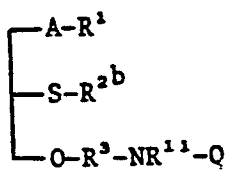

XXXV

(wherein Q represents a tert-butoxycarbonyl group or a benzyloxycarbonyl group, $R^{2b}$ represents an alkyl group of 1 to 6 carbon atoms or a group of the general formula: 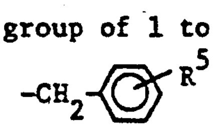 or $-CS-OR^8$, in which the various symbols are as defined in claim 1 and the other symbols are as hereinbefore defined).

27. A process for the preparation of glycerol derivatives as claimed in claim 1 and conforming to the general formula:

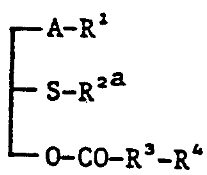

IF

(wherein $R^{2a}$ is as defined in claim 26 and the other symbols are defined in claim 1) which comprises

(i) elimination of the group Q of a compound of the general formula:

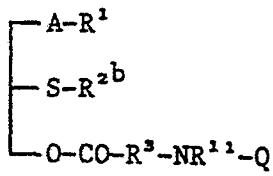

XLV

or

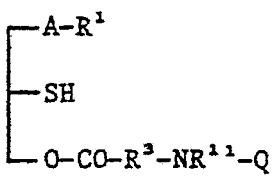

L

(wherein Q represents a tert-butoxycarbonyl group or a benzyloxycarbonyl group, $R^{11}$ represents a hydrogen atom or an alkyl group of 1 to 8 carbon atoms, $R^{2b}$ is as defined in claim 26 and the other symbols are as hereinbefore defined),

(ii) reaction of a compound of the general formula:

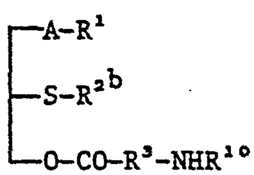

IF-2

(wherein the various symbols are as hereinbefore defined) with an equimolecular amount of alkyl halide,

(iii) reaction of a compound of the general formula:

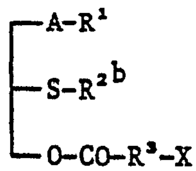

XLVI

(wherein X represents a chlorine, bromine or iodine atom and the other symbols are as hereinbefore defined) with a compound of the general formula XVII depicted in claim 23,

(iv) reaction of a compound of the general formula:

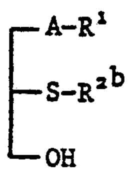

XLIV

(wherein the various symbols are as hereinbefore defined) with a compound of the general formula XX depicted in claim 23,

(v) elimination of the acetyl group of a compound of the general formula:

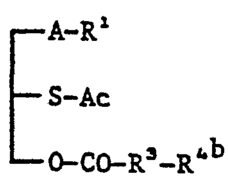

LII

(wherein Ac is acetyl group, $R^{4b}$ represents a group of the general formula: $-NHCOR^9$ or $-N(R^{10})_2$, in which the various symbols are as hereinbefore defined and the other symbols are as hereinbefore

defined),

(vi) reaction of a compound of the general formula:

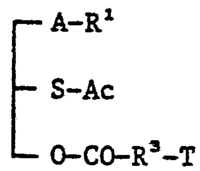

LVI

(wherein T represents an alkylsulfonyl group or a substituted or unsubstituted arylsulfonyl group and the other symbols are as hereinbefore defined) with a compound of the general formula XVII depicted in claim 23, or

(vii) subjecting a method for salt-exchange, if desired.

28. A process for the preparation of glycerol derivatives as claimed in claim 1 and conforming to the general formula:

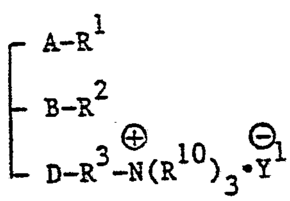

IG

(wherein $Y^1$ represents a halogen atom and the other symbols are as defined in claim 1) which comprises reaction of a compound of the general formula:

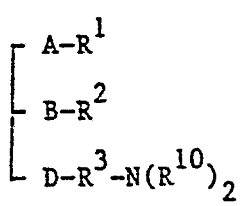

IH

(wherein the various symbols are as hereinbefore defined) with an alkyl halide.

29. A process for the preparation of glycerol derivatives as claimed in claim 1 and conforming to the general formula:

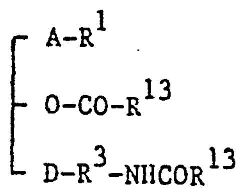

IJ

(wherein two $R^{13}$ being the same, represent alkyl groups of 1 to 6 carbon atoms or phenyl groups, and the other symbols are as defined in claim 1) which comprises acylation of a compound of the general formula:

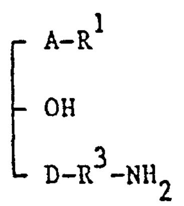

IA-6

(wherein the various symbols are as hereinbefore defined).

30. A process for the preparation of glycerol derivatives as claimed in claim 1 and conforming to the general formula:

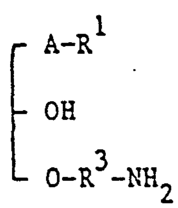

IA-7

(wherein the various symbols are as defined in claim 1) which comprises reaction of a compound of the general formula:

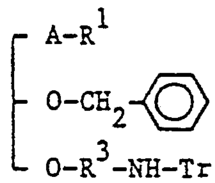

LIX

(wherein Tr is trityl group and the other symbols are as hereinbefore defined) under acidic conditions.

31. A platelet aggregation inhibiting, anti-asthmatic, anti-allergic, anti-inflammatory, hypotensive or anti-tumour pharmaceutical composition which comprises, as active ingredient, an effective amount of at least one compound of the general formula I depicted in claim 1, wherein the various symbols are as defined in claim 1, or a non-toxic acid addition salt thereof, together with a pharmaceutical carrier or coating.